# EUROPEAN PATENT APPLICATION

(11) **EP 4 806 466 A1**
(43) Date of publication of application: **16.09.2026**
(21) Application number: 26164300.1
(22) Date of filing: 12.03.2026
(51) Int. Cl.: A61K 47/68, A61P 35/00, C07K 16/28, C07K 16/46

(54) **FOLATE RECEPTOR ALPHA ANTIBODY DRUG CONJUGATES**

(30) Priority: 14.03.2025 US 202563772218 P
(71) Applicant: AbbVie Inc., North Chicago, IL 60064 (US)
(72) Inventor: MORGAN-LAPPE, Susan E., North Chicago, Illinois, 60064 (US); PHILLIPS, Andrew C., North Chicago, Illinois, 60064 (US); REILLY, Regina M., North Chicago, Illinois, 60064 (US)
(74) Representative: Hoffmann Eitle

(57) **Abstract**

The present disclosure provides anti-folate receptor alpha (FRα) antibody drug conjugates (ADCs), including compositions and methods of using the same. More particularly, the invention provides ADCs conjugated to topoisomerase inhibiting compounds.

## Description

### CROSS-REFERENCE TO RELATED PATENT APPLICATIONS

This patent application claims the benefit of U.S. Provisional Patent Application No. 63/772,218, filed March 14, 2025. Each of the aforementioned patent applications is hereby incorporated by reference in its entirety.

### REFERENCE TO SEQUENCE LISTING SUBMITTED ELECTRONICALLY

The content of the electronically submitted sequence listing (Name: ABV21790US01_ST26.xml; Size: 401,773 bytes; and Date of Creation: October 15, 2025) filed with the application is incorporated herein by reference in its entirety.

### TECHNICAL FIELD

The invention generally relates to antibodies linked (also referred to as coupled or conjugated) with therapeutically useful chemicals. Antibodies coupled with chemicals may be referred to as immunoconjugates or antibody drug conjugates (ADCs). More particularly, the invention relates to anti-Folate Receptor-alpha antibodies coupled with topoisomerase inhibiting chemicals and uses thereof in the treatment of ovarian, peritoneal (primary peritoneal), fallopian tube, endometrial, and cervical cancers.

### BACKGROUND

Folate Receptor-alpha (FRα), also known as Folate Receptor 1 (FOLR1), or Folate Binding Protein, is an N-glycosylated glycosylphosphatidylinositol-anchored cell surface protein (encoded by the folate receptor 1 (*FOLR1*) gene) expressed on plasma membrane of cells. FRα has a high affinity for folic acid and for several reduced folic acid derivatives. FRα mediates delivery of the physiological folate (5-methyltetrahydrofolate), which is an essential co-factor for one-carbon transfer reactions that are required for DNA and RNA synthesis, cell growth and proliferation, to the interior of cells. Marked upregulation of FRα occurs during neonatal development and in cancer, suggesting that the receptor functions primarily under conditions of high folate demand. In contrast, normal adult tissues generally lack FRα expression and employ alternative transporters such as folate receptor B, reduced folate carrier, and proton-coupled folate transporter for folate uptake (Weitman et al., Cancer Res. 1992;52(12):3396-401; Mantovani et al., Eur J Cancer. 1994;30A(3):363-9; Elnakat et al., Adv Drug Deliv Rev. 2004;56(8):1067-84, Kelemen et al., Int J Cancer. 2006;119(2):243-50).

FRα is overexpressed in vast majority of ovarian cancers, as well as in many uterine, endometrial, pancreatic, renal, lung, and breast cancers, while the expression of FRα on normal tissues is restricted to the apical membrane of epithelial cells in the kidney proximal tubules, alveolar pneumocytes of the lung, bladder, testes, choroid plexus, and thyroid (Weitman SD, et al., Cancer Res 52: 3396-3401 (1992); Antony AC, Annu Rev Nutr 16: 501-521 (1996); Kalli KR, et al. Gynecol Oncol 108: 619-626 (2008)).

High levels of FRα have been found in serous and endometrioid epithelial ovarian cancer, endometrial adenocarcinoma, and non-small cell lung cancer of the adenocarcinoma subtype. Importantly, FRα expression is maintained in metastatic foci and recurrent carcinomas in ovarian cancer patients, and after chemotherapy in epithelial ovarian and endometrial cancers. These properties, together with the highly restricted expression of FRα on normal tissues, make FRα a highly promising target for targeted therapies such as that provided by antibody drug conjugates (ADCs).

Because ovarian cancers, are typically asymptomatic until advanced stage, they are often diagnosed at a late stage and have poor prognosis when treated with currently available procedures, typically chemotherapeutic drugs after surgical de-bulking (von Gruenigen V et al., Cancer 112: 2221-2227 (2008); Ayhan A et al., Am J Obstet Gynecol 196: 81 e81-86 (2007); Harry VN et al., Obstet Gynecol Surv 64: 548-560 (2009)).

Mirvetuximab soravtansine (ELAHERE^{®}), a FRα targeting antibody drug conjugate (ADC) that comprises a FRα targeting antibody, mirvetuximab, conjugated to a potent tubulin-acting maytansinoid, DM4, is approved for the treatment of adult patients with FRα positive, platinum-resistant epithelial ovarian, fallopian tube, or primary peritoneal cancer, who have received one to three prior systemic treatment regimens. Patients whose tumors express a high level of FRα as measured by immunohistochemistry (IHC) staining are selected for therapy.

However, there remains a need in the art for improved anti-FRα ADCs that have reduced toxicity, improved efficacy, and broader activity for use in treatment of cancer, for example, ovarian, peritoneal (primary peritoneal), fallopian tube, endometrial, and cervical cancer.

### SUMMARY OF THE INVENTION

Provided herein are antibody drug conjugates ("ADCs"), also known as immunoconjugates, wherein the target antigen is folate receptor alpha (FRα) and the conjugated cytotoxin (drug) is a topoisomerase-1 inhibitor (TOP1i). In some embodiments, the antibody drug conjugates described herein are for use in the treatment of cancer. Also provided herein are methods of using the anti-FRα-TOP1i immunoconjugates described herein in the treatment of cancer. In some embodiments of the methods described herein, the targeted tumor or cancer cells over-express folate receptor alpha (FRα) compared to noncancerous cells.

Provided herein are ADCs useful in treating ovarian, peritoneal (primary peritoneal), fallopian tube, endometrial, and cervical cancers.

In some embodiments, provided herein is an ADC-1 anti-FRα antibody drug conjugate of formula (IV): 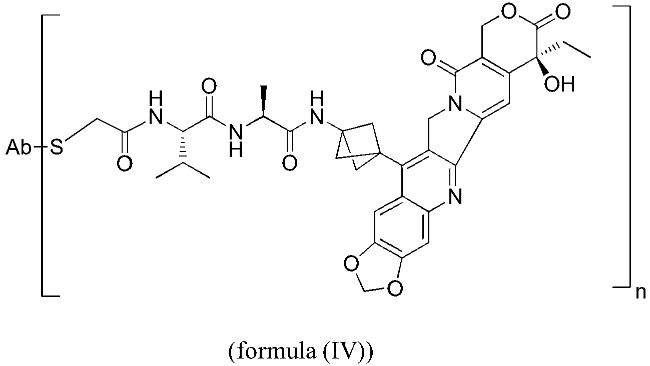 wherein n is an integer from 1 to 10, and wherein "Ab" is an anti-FRα antibody comprising a heavy chain variable region comprising a CDR-H1, a CDR-H2, and a CDR-H3; and a light chain variable region comprising a CDR-L1, a CDR-L2, and a CDR-L3, wherein
CDR-H1 has the amino acid sequence shown as SEQ ID NO: 1 or SEQ ID NO: 23,
CDR-H2 has the amino acid sequence shown as SEQ ID NO: 2 or SEQ ID NO:24,
CDR-H3 has the amino acid sequence shown as SEQ ID NO: 3,
CDR-L1 has the amino acid sequence shown as SEQ ID NO: 7,
CDR-L2 has the amino acid sequence shown as SEQ ID NO: 8, and
CDR-L3 has the amino acid sequence shown as SEQ ID NO: 9.

In some embodiments, the anti-FRα antibody of ADC-1 comprises (a) a heavy chain variable region (VH) having the amino acid sequence shown as SEQ ID NO: 4 and (b) a light chain variable region (VL) having the amino acid sequence shown as SEQ ID NO: 10 or SEQ ID NO: 22.

In some embodiments, the anti-FRα antibody of ADC-1 comprises (a) a heavy chain (HC) having the amino acid shown as SEQ ID NO: 5 or SEQ ID NO: 6 and (b) a light chain (LC) having the amino acid sequence shown as SEQ ID NO: 11.

In some embodiments, the anti-FRα antibody of ADC-1 is an IgG1 antibody.

In some embodiments, the anti-FRα antibody drug conjugate ADC-1 has the structure of formula (IV): 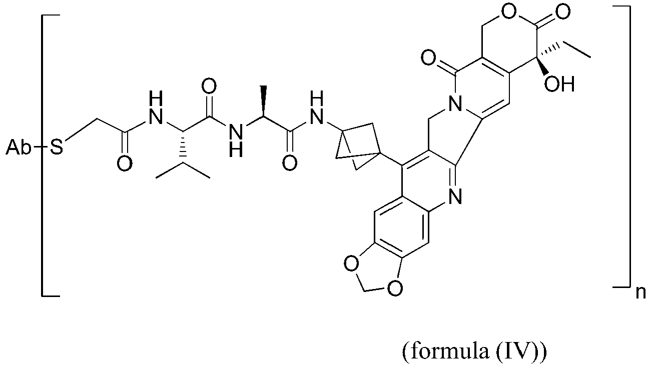 wherein n is an integer from 1 to 10, and wherein "Ab" is an anti-FRα antibody comprising: two heavy chains each having an amino acid sequence shown as SEQ ID NO: 5 or SEQ ID NO: 6 and two light chains each having the amino acid sequence shown as SEQ ID NO: 11.

In another embodiment, provided herein is a bivalent biparatopic anti-FRα antibody drug conjugate (ADC-2) of formula (IV): 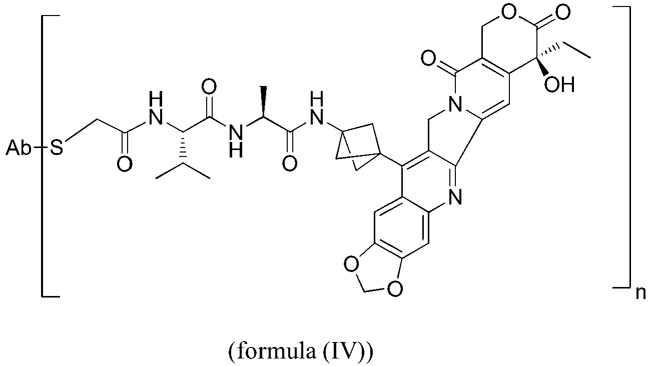
wherein n is an integer from 1 to 10, and wherein "Ab" is a bivalent biparatopic anti-FRα antibody comprising (a) a first antigen-antigen binding moiety comprising (i) a first heavy chain variable region comprising a first CDR-H1, a first CDR-H2, and a first CDR-H3; and (ii) a first light chain variable region comprising a first CDR-L1, a first CDR-L2, and a first CDR-L3, wherein
the first CDR-H1 has the amino acid sequence shown as SEQ ID NO: 19 or SEQ ID NO:25,
the first CDR-H2 has the amino acid sequence shown as SEQ ID NO: 20 or SEQ ID NO:26,
the first CDR-H3 has the amino acid sequence shown as SEQ ID NO: 21,
the first CDR-L1 has the amino acid sequence shown as SEQ ID NO: 16,
the first CDR-L2 has the amino acid sequence shown as SEQ ID NO: 17, and
the first CDR-L3 has the amino acid sequence shown as SEQ ID NO: 18,
wherein the bivalent biparatopic anti-FRα antibody further comprises (b) a second antigen-antigen binding moiety comprising (i) a second heavy chain variable region comprising a second CDR-H1, a second CDR-H2, and a second CDR-H3; and (ii) a second light chain variable region comprising a second CDR-L1, a second CDR-L2, and a second CDR-L3, wherein
the second CDR-H1 has the amino acid sequence shown as SEQ ID NO: 1 or SEQ ID NO: 23,
the second CDR-H2 has the amino acid sequence shown as SEQ ID NO: 2 or SEQ ID NO:24,
the second CDR-H3 has the amino acid sequence shown as SEQ ID NO: 3,
the second CDR-L1 has the amino acid sequence shown as SEQ ID NO: 7,
the second CDR-L2 has the amino acid sequence shown as SEQ ID NO: 8, and
the second CDR-L3 has the amino acid sequence shown as SEQ ID NO: 9.

In some embodiments, the anti-FRα antibody of ADC-2 comprises: (a) a first antigen binding moiety comprising a first heavy chain variable region having the amino acid sequence shown as SEQ ID NO: 14; and a first light chain variable region having the amino acid sequence shown as SEQ ID NO: 15; and (b) a second antigen binding moiety comprising a second heavy chain variable region having the amino acid sequence shown as SEQ ID NO: 4; and a second light chain variable region having the amino acid sequence shown as SEQ ID NO: 10 or SEQ ID NO: 22.

In some embodiments, the anti-FRα antibody of ADC-2 comprises (a) a first polypeptide (Heavy Chain 1 or HC 1) having the amino acid shown as SEQ ID NO: 12, (b) a second polypeptide (Heavy Chain 2 or HC 2) having the amino acid shown as SEQ ID NO: 13 and (c) a third polypeptide (Light Chain) having the amino acid sequence shown as SEQ ID NO: 11, wherein the first polypeptide comprises an scFv-IgG Fc fusion polypeptide, the second polypeptide comprises a heavy chain and the third polypeptide comprises a light chain.

In some embodiments, the anti-FRα antibody of ADC-2 is derived from an IgG1 antibody.

In some embodiments, the anti-FRα antibody drug conjugate ADC-2 has the structure of formula (IV): 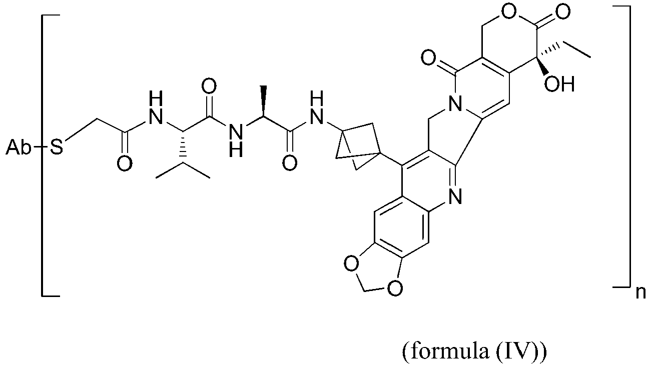 wherein n is an integer from 1 to 10, and wherein "Ab" is a bivalent biparatopic anti-FRα antibody comprising: (a) a first polypeptide (Heavy Chain 1 or HC 1) having the amino acid shown as SEQ ID NO: 12, (b) a second polypeptide (Heavy Chain 2 or HC 2) having the amino acid shown as SEQ ID NO: 13 and (c) a third polypeptide (Light Chain or LC) having the amino acid sequence shown as SEQ ID NO: 11.

In some embodiments, an anti-FRα antibody drug conjugate described herein has n of 4.

In some embodiments, an anti-FRα antibody drug conjugate described herein has n of about 4.

Also provided herein is a composition comprising a plurality of an anti-FRα antibody drug conjugate molecules described herein, wherein the plurality of antibody drug conjugate molecules in the composition has an average n of 4 or an average n of about 4.

Also provided herein is a composition comprising a plurality of an anti-FRα antibody drug conjugate molecules described herein, wherein the predominant species of the antibody drug conjugate molecules in the composition has an n of 4.

Also provided herein is a pharmaceutical composition comprising a plurality of an anti-FRα antibody drug conjugate molecules described herein and a pharmaceutically acceptable excipient, wherein the pharmaceutical composition has a DAR of 4 or about 4.

Also provided herein is a method of treating cancer (e.g., FRα-expressing cancers) such as, gynecological cancers, not limited to, ovarian, peritoneal (primary peritoneal), fallopian tube, endometrial, and cervical cancers, wherein the method comprises administering a therapeutically effective amount of an anti-FRα antibody drug conjugate described herein to a patient in need thereof.

Also provided herein a method of treating cancer (*e.g*., FRα-expressing cancers) such as, gynecological cancers, not limited to, ovarian, peritoneal (primary peritoneal), fallopian tube, endometrial, and cervical cancers, wherein the method comprises administering a therapeutically effective amount of a pharmaceutical composition described herein comprising a therapeutically effective amount of an anti-FRα antibody drug conjugate described herein to a patient in need thereof.

### DESCRIPTION OF THE DRAWINGS

FIG. 1 demonstrates *in vitro* cytotoxicity of immunoconjugates (ADCs) on different cell types expressing FRα. *In vitro* cytotoxicity was monitored for a variety of cells (COV318, IGROV-1, KB, OV-90 and OVSAHO cells) incubated across a titration of treatments: Top1 inhibitor payload only (open square), ADC-1 (open circle), ADC-2 (closed circle), or non-targeting ADC-3 (X) as evaluated by the CELLTITER-GLO^{®} cell viability assay.

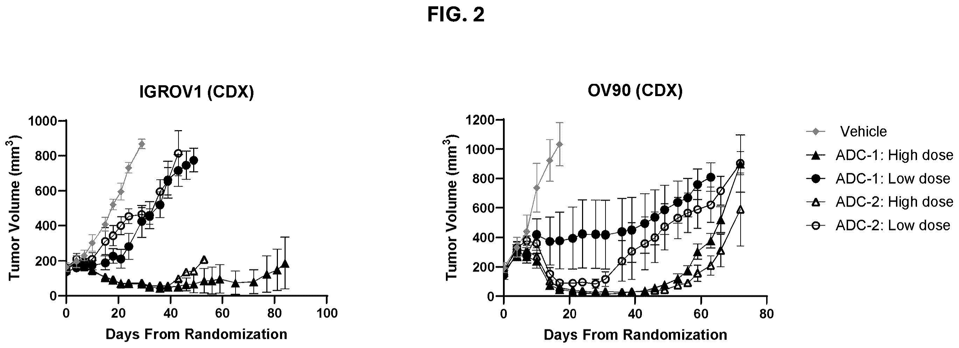

FIG. 2 shows growth of cancer cell line xenografts (CDX) differing in FOLR1 expression (IGROV1, high; OV90, low) were inhibited by a single dose of ADC treatments in CB17/SCID mice.

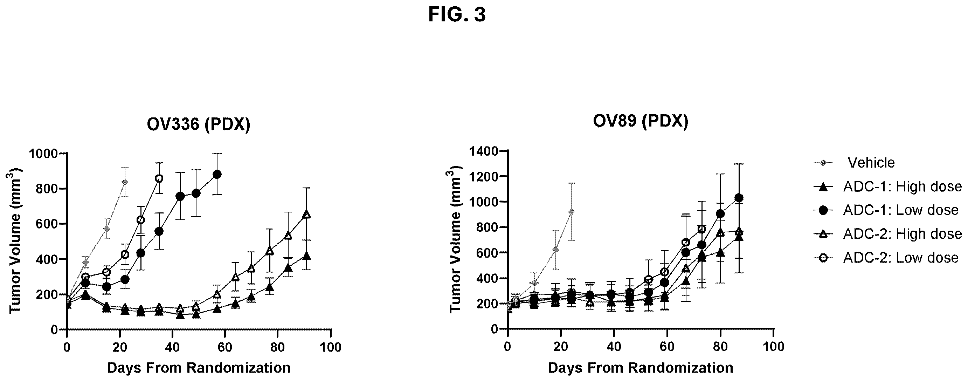

FIG. 3 shows growth inhibition of patient-derived xenografts (PDX) with high FOLR1 expression were inhibited by a single dose of ADC treatments in NSG mice..

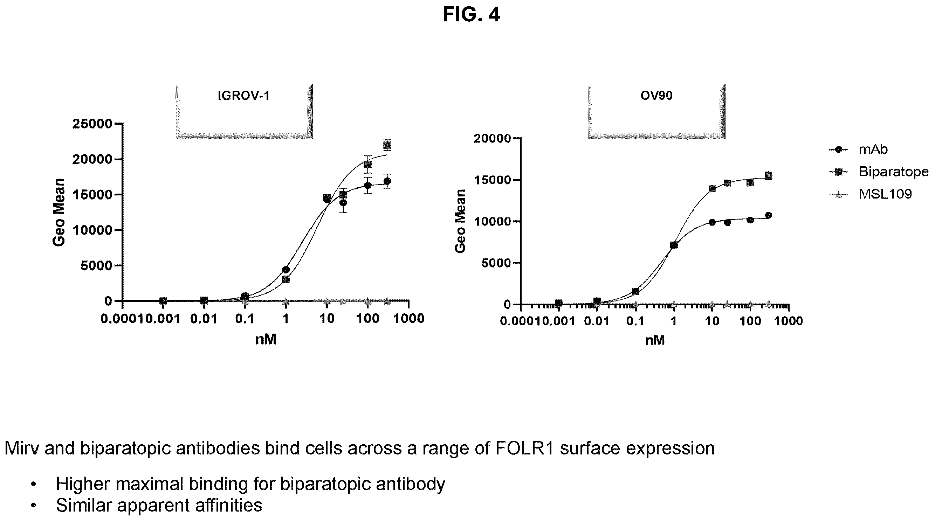

FIG. 4 shows that in preclinical models both ADC-1 and ADC-2 bound cells with varying FOLR1 cell surface expression levels (*e.g*., low, medium and high FOLR1 cell surface expression) with similar affinities.
FIG. 5A & FIG. 5B show results of anti-tumor efficacy experiments performed using IGROV-1 cells (high FRα expression levels) in a mouse model cancer system. *See,* Example *7. (Symbols:* Solid squares (5A) or solid circles (5B) = mirvetuximab soravtansine (ELAHERE^{®}); solid triangles, dotted line = ADC-1; solid triangles, solid line = ADC-2; solid diamonds = vehicle; open squares = Isotype Top1i.D6 control).
FIG. 6 shows results of anti-tumor efficacy experiments performed using OV336 and OV89 cells (high FRα expression levels) in an ovarian patient-derived xenograft model system ("ovarian PDX model"). *See,* Example 7.
FIG. 7 shows results of anti-tumor efficacy experiments performed using OV-90 cells (low FRα expression levels) in a mouse model cancer system. *See,* Example 7.

### DETAILED DESCRIPTION

As used in this disclosure, the singular forms "a", "an" and "the" include plural referents unless the context clearly dictates otherwise. The terms "a" (or "an"), as well as the terms "one or more," and "at least one" can be used interchangeably herein unless the context clearly dictates otherwise.

As used in this disclosure and unless otherwise specified, the terms "about" and "approximately" generally refer to a range of numbers that one of skill in the art would consider equivalent to the recited value (*i.e.,* having the same function or result). In many instances, the terms "about" and "approximately" may include numbers that are rounded to the nearest significant figure. In specific embodiments, the terms "about" and "approximately" shall be construed so as to allow normal variation as judged by a person of ordinary skill in the art, such as, for example, a variation within 20% or 10% or 5%. In specific embodiments, the terms "about" and "approximately" encompass the exact value recited. Unless the context clearly dictates otherwise, all numerical values provided herein are modified by the term about.

In some embodiments, the anti-FRα antibody of ADC-1 is the huMov19 (M9346A) antibody is encoded by the plasmids deposited with the American Type Culture Collection (ATCC), located at 10801 University Boulevard, Manassas, Va. 20110 on Apr. 7, 2010 under the terms of the Budapest Treaty and having ATCC deposit nos. PTA-10772 and PTA-10774. DM4 refers to N2'-deacetyl-N2'-(4-mercapto-4-methyl-1-oxopentyl) maytansinoid.

In some embodiments, the bivalent biparatopic anti-FRα antibody of ADC-2 is encoded by the plasmids deposited with the American Type Culture Collection (ATCC), located at 10801 University Boulevard, Manassas, Va. 20110 under the terms of the Budapest Treaty and having ATCC deposit nos. PTA-10774 (deposited in Apr. 7, 2010), PTA-125915 ("Mov19-Fc-hole"; deposited to the ATCC on Apr. 29, 2019 and received by the ATCC on Apr. 30, 2019), and PTA-125916 ("FR57scFv2-Fc-knob"; deposited to the ATCC on Apr. 29, 2019 and received by the ATCC on Apr. 30, 2019).

It is understood that wherever embodiments are described herein with the language "comprising," otherwise analogous embodiments described in terms of "consisting of" and/or "consisting essentially of" are also provided. In this disclosure, "comprises," "comprising," "containing" and "having" and the like can mean "includes," "including," and the like; "consisting essentially of" or "consists essentially" are open-ended, allowing for the presence of more than that which is recited so long as basic or novel characteristics of that which is recited is not changed by the presence of more than that which is recited, but excludes prior art embodiments.

Unless specifically stated or obvious from context, as used herein, the term "or" is understood to be inclusive. The term "and/or" as used in a phrase such as "A and/or B" herein is intended to include both "A and B," "A or B," "A," and "B." Likewise, the term "and/or" as used in a phrase such as "A, B, and/or C" is intended to encompass each of the following: A, B, and C; A, B, or C; A or C; A or B; B or C; A and C; A and B; B and C; A (alone); B (alone); and C (alone).

As described herein, anti-FRα antibodies can be used therapeutically (*e.g*., to treat cancer) and/or to detect FRα (*e.g*., membrane FRα). Exemplary anti-FRα antibodies are known in the art and have been disclosed, for example, in WO 2011/106528, WO 2014/036495, WO 2015/031815, and U.S. Published Application No. 2020/0362029, each of which is herein incorporated by reference in its entirety. Additional anti- FRα antibodies are known in the art and have been disclosed, for example, in WO 2012/061759, U.S. Published Application No. 2019/0233512, U.S. Published Application No. 2020/0147229, U.S. Published Application No. 2020/0297860 U.S. Published Application No. 2020/0353076, U.S. Patent No. 10,822,410 and U.S. Patent No. 10,101,343, each of which is herein incorporated by reference in its entirety.

Amino acid sequences of the anti-FRα antibody of ADC-1 and ADC-2 are provided in the Tables below.

**Table 1: Variable heavy chain CDR amino acid sequences**

| Antibody | VH-CDR1 | VH-CDR2 | VH-CDR3 |
|---|---|---|---|
| ADC-1 | Kabat Defined: | Kabat Defined: | Kabat Defined: |
| | GYFMN (SEQ ID NO: 1) | RIHPYDGDTFYNQKFQG (SEQ ID NO: 2) | YDGSRAMDY (SEQ ID NO: 3) |
| ADC-2 | | | |
| ADC-1 | AbM Defined: | AbM Defined: | AbM Defined: |
| | GYTFTGYFMN (SEQ ID NO: 23) | RIHPYDGDTF (SEQ ID NO: 24) | YDGSRAMDY (SEQ ID NO:3) |
| ADC-2 | | | |
| ADC-2 | Kabat Defined: | Kabat Defined: | Kabat Defined: |
| | SFGMH (SEQ ID NO: 19) | YISSGSSTISYADSVKG (SEQ ID NO: 20) | EAYGSSMEY (SEQ ID NO: 21) |
| ADC-2 | AbM Defined: | AbM Defined: | AbM Defined: |
| | GFTFSSFGMH (SEQ ID NO: 25) | YISSGSSTIS (SEQ ID NO: 26) | EAYGSSMEY (SEQ ID NO: 21) |

**Table 2: Variable light chain CDR amino acid sequences**

| Antibody | VL-CDR1 | VL-CDR2 | VL-CDR3 |
|---|---|---|---|
| ADC-1 | Kabat Defined: | Kabat Defined: | Kabat Defined: |
| | KASQSVSFAGTSLMH (SEQ ID NO: 7) | RASNLEA (SEQ ID NO: 8) | QQSREYPYT (SEQ ID NO: 9) |
| ADC-2 | | | |
| ADC-2 | Kabat Defined: | Kabat Defined: | Kabat Defined: |
| | RASQNINNNLH (SEQ ID NO: 16) | YVSQSVS (SEQ ID NO: 17) | QQSNSWPHYT (SEQ ID NO: 18) |

**Table 3: Heavy chain variable region (VH) amino acid sequences**

| Antibody | Amino Acid Sequences |
|---|---|
| ADC-1 | QVQLVQSGAEVVKPGASVKISCKASGYTFTGYFMNWVKQSPGQSLE WIGRIHPYDGDTFYNQKFQGKATLTVDKSSNTAHMELLSLTSEDFAV YYCTRYDGSRAMDYWGQGTTVTVSS (SEQ ID NO: 4) |
| ADC-2 | |
| ADC-2 | EVQLVQSGGGLVQPGGSRRLSCAASGFTFSSFGMHWVRQAPGKCLE WVAYISSGSSTISYADSVKGRFTISRDNSKKTLLLQMTSLRAEDTAMY YCAREAYGSSMEYWGQGTLVTVSS (SEQ ID NO: 14) |

**Table 4: Light chain variable region (VL) amino acid sequences**

| Antibody | Amino Acid Sequences |
|---|---|
| ADC-1 | DIVLTQSPLSLAVSLGQPAIISCKASQSVSFAGTSLMHWYHQKPGQQP RLLIYRASNLEAGVPDRFSGSGSKTDFTLTISPVEAEDAATYYCQQSR EYPYTFGGGTKLEIKR (SEQ ID NO: 10) |
| ADC-2 | |
| ADC-1 | DIVLTQSPLSLAVSLGQPAIISCKASQSVSFAGTSLMHWYHQKPGQQP RLLIYRASNLEAGVPDRFSGSGSKTDFTLTISPVEAEDAATYYCQQSR EYPYTFGGGTKLEIK (SEQ ID NO: 22) |
| ADC-2 | |
| ADC-2 | EIVLTQSPATLSVTPGDRVSLSCRASQNINNNLHWYQQKPGQSPRLLI KYVSQSVSGIPDRFSGSGSGTDFTLSISSVEPEDEGMYFCQQSNSWPH YTFGCGTKLEIK (SEQ ID NO: 15) |

**Table 5: Full-length heavy chain (HC) amino acid sequences**

| Antibody | Full-Length Amino Acid Sequences |
|---|---|
| ADC-1 | QVQLVQSGAEVVKPGASVKISCKASGYTFTGYFMNWVKQSPGQSLE WIGRIHPYDGDTFYNQKFQGKATLTVDKSSNTAHMELLSLTSEDFAV YYCTRYDGSRAMDYWGQGTTVTVSSASTKGPSVFPLAPSSKSTSGGT AALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGN VFSCSVMHEALHNHYTQKSLSLSPGK (SEQ ID NO: 5) |
| ADC-1 | QVQLVQSGAEVVKPGASVKISCKASGYTFTGYFMNWVKQSPGQSLE WIGRIHPYDGDTFYNQKFQGKATLTVDKSSNTAHMELLSLTSEDFAV YYCTRYDGSRAMDYWGQGTTVTVSSASTKGPSVFPLAPSSKSTSGGT AALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGN VFSCSVMHEALHNHYTQKSLSLSPG (SEQ ID NO: 6 (SEQ ID NO:5 minus C-terminal K)) |
| ADC-2 | EIVLTQSPATLSVTPGDRVSLSCRASQNINNNLHWYQQKPGQSPRLLI KYVSQSVSGIPDRFSGSGSGTDFTLSISSVEPEDEGMYFCQQSNSWPH YTFGCGTKLEIKGGGGSGGGGSGGGGSGGGGSEVQLVQSGGGLVQP GGSRRLSCAASGFTFSSFGMHWVRQAPGKCLEWVAYISSGSSTISYA DSVKGRFTISRDNSKKTLLLQMTSLRAEDTAMYYCAREAYGSSMEY WGQGTLVTVSSGSEPKSSDKTHTCPPCPAPELLGGPSVFLFPPKPKDT LMISRTPEVTCVVVDVSHEDPEVKFNWYVDGVEVHNAKTKPREEQY NSTYRVVSVLTVLHQDWLNGKEYKCKVSNKALPAPIEKTISKAKGQP REPQVYTLPPSREEMTKNQVSLWCLVKGFYPSDIAVEWESNGQPENN YKTTPPVLDSDGSFFLYSKLTVDKSRWQQGNVFSCSVMHEALHNHY TQKSLSLSPG (SEQ ID NO: 12 (first polypeptide or Heavy Chain 1: scFv-Fc-knob (T366W)) |
| ADC-2 | QVQLVQSGAEVVKPGASVKISCKASGYTFTGYFMNWVKQSPGQSLE WIGRIHPYDGDTFYNQKFQGKATLTVDKSSNTAHMELLSLTSEDFAV YYCTRYDGSRAMDYWGQGTTVTVSSASTKGPSVFPLAPSSKSTSGGT AALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDG |
| | VEVHNAKTKPREEQYNSTYRVVSVLTVLHQDWLNGKEYKCKVSNK ALPAPIEKTISKAKGQPREPQVYTLPPSREEMTKNQVSLSCAVKGFYP SDIAVEWESNGQPENNYKTTPPVLDSDGSFFLVSKLTVDKSRWQQGN VFSCSVMHEALHNHYTQKSLSLSPG (SEQ ID NO: 13 (second polypeptide or Heavy Chain 2: Fc-hole (T366S, L368A, Y407V)) |

**Table 6: Full-length light chain amino acid sequences**

| Antibody | Full-Length Amino Acid Sequence |
|---|---|
| ADC-1 | DIVLTQSPLSLAVSLGQPAIISCKASQSVSFAGTSLMHWYHQKPGQQP RLLIYRASNLEAGVPDRFSGSGSKTDFTLTISPVEAEDAATYYCQQSR EYPYTFGGGTKLEIKRTVAAPSVFIFPPSDEQLKSGTASVVCLLNNFYP REAKVQWKVDNALQSGNSQESVTEQDSKDSTYSLSSTLTLSKADYE KHKVYACEVTHQGLSSPVTKSFNRGEC (SEQ ID NO: 11) |
| ADC-2 | |

In some embodiments, the anti-FRα antibody of an antibody drug conjugate described herein comprises the sequences of SEQ ID Nos: 1, 2, and 3. In some embodiments, the anti-FRα antibody comprises the sequences of SEQ ID Nos: 23 and 24. In some embodiments, the anti-FRα antibody comprises the sequences of SEQ ID Nos: 23, 24 and 3. In some embodiments, the anti-FRα antibody comprises the sequences of SEQ ID Nos: 19, 20, and 21. In some embodiments, the anti-FRα antibody comprises the sequences of SEQ ID Nos: 25 and 26. In some embodiments, the anti-FRα antibody comprises the sequences of SEQ ID Nos: 25, 26 and 21. In some embodiments, the anti-FRα antibody comprises the sequences of SEQ ID Nos: 7, 8 and 9. In some embodiments, the anti-FRα antibody comprises the sequences of SEQ ID Nos: 16, 17 and 18.

In some embodiments, the anti-FRα antibody of an antibody drug conjugate described herein comprises the sequences of SEQ ID Nos: 1, 2, 3, 7, 8 and 9. In some embodiments, the anti-FRα antibody comprises the sequences of SEQ ID Nos: 23, 24, 3, 7, 8 and 9. In some embodiments, the anti-FRα antibody comprises the sequences of SEQ ID Nos: 19, 20, 21, 16, 17 and 18. In some embodiments, the anti-FRα antibody comprises the sequences of SEQ ID Nos: 25, 26, 21, 16, 17 and 18.

In some embodiments, the anti-FRα antibody of ADC-2 comprises the sequences of SEQ ID Nos: 1, 2, 3, 19, 20, 21, 7, 8, 9, 16, 17 and 18. In some embodiments, the anti-FRα antibody of ADC-2 comprises the sequences of SEQ ID Nos: 1, 2, 3, 25, 26, 21, 7, 8, 9, 16, 17 and 18. In some embodiments, the anti-FRα antibody of ADC-2 comprises the sequences of SEQ ID Nos: 23, 24, 3, 19, 20, 21, 7, 8, 9, 16, 17 and 18. In some embodiments, the anti-FRα antibody of ADC-2 comprises the sequences of SEQ ID Nos: 23, 24, 3, 25, 26, 21, 7, 8, 9, 16, 17 and 18.

In some embodiments, the anti-FRα antibody of an antibody drug conjugate described herein comprises the sequences of SEQ ID Nos: 4 and 10. In some embodiments, the anti-FRα antibody comprises the sequences of SEQ ID Nos: 4 and 22. In some embodiments, the anti-FRα antibody comprises the sequences of SEQ ID Nos: 14 and 15. In some embodiments, the anti-FRα antibody comprises the sequences of SEQ ID Nos: 4, 10, 14 and 15. In some embodiments, the anti-FRα antibody comprises the sequences of SEQ ID Nos: 4, 22, 14 and 15.

In some embodiments, the anti-FRα antibody of ADC-1 comprises the sequences of SEQ ID Nos: 5 and 11. In some embodiments, the anti-FRα antibody of ADC-1 comprises the sequences of SEQ ID Nos: 6 and 11. In some embodiments, the anti-FRα antibody of ADC-2 comprises the sequences of SEQ ID Nos: 12 and 13. In some embodiments, the anti-FRα antibody of ADC-2 comprises the sequences of SEQ ID Nos: 11, 12 and 13.

In some embodiments, the anti-FRα antibody of an antibody drug conjugate described herein comprises any combination of the sequences of SEQ ID NOs: 1-3, 7-9, 16-21 and 23-26.

In some embodiments, the anti-FRα antibody of an antibody drug conjugate described herein that specifically binds a human FRα can comprise any of the Kabat-defined variable heavy chain CDR amino acid sequences, the Abm-defined variable heavy chain CDR amino acid sequences and/or any of the Kabat-defined variable light chain CDR amino acid sequences, or any of the Abm-defined variable light chain CDR amino acid sequences described herein, in any combination.

In some embodiments, the anti-FRα antibody of an antibody drug conjugate described herein can comprise one of the individual light chains or heavy chains described herein. In some embodiments, the anti-FRα antibody of an antibody drug conjugate described herein comprises both a light chain and a heavy chain. In some embodiments, the anti-FRα antibody of an antibody drug conjugate described herein comprises a light chain, a heavy chain and an scFv linked to an Fc region polypeptide.

In some embodiments, the bivalent biparatopic antibody of ADC-2 has a knob-in-hole (KIH) structure. In some embodiments, the bivalent biparatopic antibody comprises a FRα binding domain, wherein the FRα binding domain comprises SEQ ID NOs: 1-3 and 7-9. In some embodiments, the bivalent biparatopic antibody comprises a FRα binding domain, wherein the FRα binding domain comprises SEQ ID NOs: 16-21. In some embodiments, the bivalent biparatopic antibody comprises two FRα binding domains, wherein the FRα binding domains comprise SEQ ID NOs: 1-3, 7-9 and 16-21.

In some embodiments, the anti-FRα antibody of an antibody drug conjugate described herein specifically binds a human FRα and comprises a VH comprising the sequence of SEQ ID NO: 4 and a VL comprising the sequence of SEQ ID NO: 10 or SEQ ID NO: 22. In some embodiments, the anti-FRα antibody specifically binds a human FRα and comprises a VH comprising the sequence of SEQ ID NO: 14 and a VL comprising the sequence of SEQ ID NO:15. In some embodiments, the anti-FRα antibody specifically binds a human FRα and comprises VH sequences of SEQ ID NO: 4 and SEQ ID NO: 14 and VL sequences of SEQ ID NO: 15 and SEQ ID NO:10 or SEQ ID NO:22.

In some embodiments, the anti-FRα antibody of an antibody drug conjugate described herein specifically binds a human FRα and comprises the sequences of: SEQ ID Nos: 5 and 11; SEQ ID Nos: 6 and 11; SEQ ID Nos: 12 and 13; or SEQ ID Nos: 11, 12 and 13.

In some embodiments, the anti-FRα antibody of an antibody drug conjugate described herein comprises a heavy chain constant region, such as an IgG1 constant region. In some embodiments, the heavy chain constant region is an IgG1 heavy chain constant region. Furthermore, in some embodiments, the anti-FRα-antibody can comprise a light chain constant region, either a kappa light chain constant region or a lambda light chain constant region. In some embodiments, the light chain constant region is a kappa light chain constant region.

In some embodiments, the anti-FRα-antibody of ADC-1 may be in a format, such as an IgG1 or IgG4 format, that has been modified to confer desired properties, such as having the Fc mutated to reduce or "silence" effector function or extend half-life. In some embodiments where half-life extending and/or Fc silencing mutations are introduced, the anti-FRα-antibody of ADC-1 may comprise the following heavy chain (HC) constant region sequences in the antibody combinations shown in **Table 7:**

**Table 7: Exemplary Fc Mutated and non-Mutated ADC-1 anti-FRα-antibody Heavy Chain (HC) Constant Regions**

| SEQ ID NO: | Description | Sequence |
|---|---|---|
| 27 | ADC-1 IgG1_HC_Wild-type constant region with terminal lysine | |
| 28 | ADC-1 IgG1_HC_Wild-type constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY |
| | | RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHNHYTQKSLSLSP G |
| 29 | ADC-1 IgG1_HC_STR constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG K |
| 30 | ADC-1 IgG1_HC_STR constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG |
| 31 | ADC-1 IgG1_HC_LALA constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHNHYTQKSLSLSP GK |
| 32 | ADC-1 IgG1_HC_LALA constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHNHYTQKSLSLSP G |
| 33 | ADC-1 IgG1_HC_YTE constant region | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLYITREPEVTCVVVDV |
| | with terminal lysine | SHEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG K |
| 34 | ADC-1 IgG1_HC_YTE constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLYITREPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG |
| 35 | ADC-1 IgG1_HC_STR+ YTE constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLYITREPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG K |
| 36 | ADC-1 IgG1_HC_STR+ YTE constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLYITREPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG |
| 37 | ADC-1 IgG1_HC_LALA +YTE constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLYITREPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHNHYTQKSLSLSP GK |
| 38 | ADC-1 IgG1_HC_LALA +YTE constant | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLYITREPEVTCVVVD |
| | region without terminal lysine | VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHNHYTQKSLSLSP G |
| 39 | ADC-1 IgG1_HC_REW constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHRDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVEHEALHWHYTQKSLSLSPG K |
| 40 | ADC-1 IgG1_HC_REW constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHRDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVEHEALHWHYTQKSLSLSPG |
| 41 | ADC-1 IgG1_HC_STR+ REW constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHRDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVEHEALHWHYTQKSLSLSPG K |
| 42 | ADC-1 IgG1_HC_STR+ REW constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHRDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVEHEALHWHYTQKSLSLSPG |
| 43 | ADC-1 IgG1_HC_LALA +REW constant | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD |
| | region with terminal lysine | VSHEDPEVKFNWYVDGVEVHNAKTKPREEQYNSTY RVVSVLTVLHRDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVEHEALHWHYTQKSLSLSPG K |
| 44 | ADC-1 IgG1_HC_LALA +REW constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHRDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVEHEALHWHYTQKSLSLSPG |
| 45 | ADC-1 IgG1_HC_LS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHSHYTQKSLSLSPG K |
| 46 | ADC-1 IgG1_HC_LS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHSHYTQKSLSLSPG |
| 47 | ADC-1 IgG1_HC_STR+ LS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVLHEALHSHYTQKSLSLSPGK |
| 48 | ADC-1 IgG1_HC_STR+ LS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR |
| | | VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVLHEALHSHYTQKSLSLSPG |
| 49 | ADC-1 IgG1_HC_LALA +LS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHSHYTQKSLSLSPG K |
| 50 | ADC-1 IgG1_HC_LALA +LS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHSHYTQKSLSLSPG |
| 51 | ADC-1 IgG1_HC_QL constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHNHYTQKSLSLSPG K |
| 52 | ADC-1 IgG1_HC_QL constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHNHYTQKSLSLSPG |
| 53 | ADC-1 IgG1_HC_STR+ QL constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI |
| | | SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHNHYTQKSLSLSPG K |
| 54 | ADC-1 IgG1_HC_STR+ QL constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHNHYTQKSLSLSPG |
| 55 | ADC-1 IgG1_HC_LALA +QL constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHNHYTQKSLSLSPG K |
| 56 | ADC-1 IgG1_HC_LALA +QL constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVLHEALHNHYTQKSLSLSPG |
| 57 | ADC-1 IgG1_HC_DHS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVDHHDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHSHYTQKSLSLSPG K |
| 58 | ADC-1 IgG1_HC_DHS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVDHHDWLNGKEYKCKVSNKALPAPIEKTI |
| | | SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHSHYTQKSLSLSPG |
| 59 | ADC-1 IgG1_HC_STR+ DHS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVDHHDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHSHYTQKSLSLSPG K |
| 60 | ADC-1 IgG1_HC_STR+ DHS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVDHHDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHSHYTQKSLSLSPG |
| 61 | ADC-1 IgG1_HC_LALA +DHS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVDHHDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHSHYTQKSLSLSPG K |
| 62 | ADC-1 IgG1_HC_LALA +DHS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVDHHDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHSHYTQKSLSLSPG |
| 63 | ADC-1 IgG4_S228P_HC_ Wild-type constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI |
| | | AVEWESNGQPENNYKTTPPVLDSDGSFFLYSRLTVD KSRWQEGNVFSCSVMHEALHNHYTQKSLSLSLGK |
| 64 | ADC-1 IgG4_S228P_HC_ Wild-type constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 65 | ADC-1 IgG4_S228P_HC-STR constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 66 | ADC-1 IgG4_S228P_HC-STR constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 67 | ADC-1 IgG4_S228P_HC-FALA constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 68 | ADC-1 IgG4_S228P_HC-FALA constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 69 | ADC-1 IgG4_S228P_HC_ YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 70 | ADC-1 IgG4_S228P_HC-YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 71 | ADC-1 IgG4_S228P_HC_ STR+YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 72 | ADC-1 IgG4_S228P_HC_ STR+YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 73 | ADC-1 IgG4_S228P_HC-FALA+YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLYITREPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 74 | ADC-1 IgG4_S228P_HC_ FALA+YTE | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA |
| | constant region without terminal lysine | PEAAGGPSVFLFPPKPKDTLYITREPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 75 | ADC-1 IgG4_S228P_HC_ REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLGK |
| 76 | ADC-1 IgG4_S228P_HC-REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLG |
| 77 | ADC-1 IgG4_S228P_HC_ STR+REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLGK |
| 78 | ADC-1 IgG4_S228P_HC_ STR+REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLG |
| 79 | ADC-1 IgG4_S228P_HC-FALA+REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHRDWLNGKEYKCKVSNKGLPSSIEKTISKAK |
| | | GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLGK |
| 80 | ADC-1 IgG4_S228P_HC_ FALA+REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLG |
| 81 | ADC-1 IgG4_S228P_HC_ LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLGK |
| 82 | ADC-1 IgG4_S228P_HC_ LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLG |
| 83 | ADC-1 IgG4_S228P_HC_ LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLGK |
| 84 | ADC-1 IgG4_S228P_HC_ LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI |
| | | AVEWESNGQPENNYKTTPPVLDSDGSFFLYSRLTVD KSRWQEGNVFSCSVLHEALHSHYTQKSLSLSLG |
| 85 | ADC-1 IgG4_S228P_HC_ FALA+LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLGK |
| 86 | ADC-1 IgG4_S228P_HC_ FALA+LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLG |
| 87 | ADC-1 IgG4_S228P_HC-QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLGK |
| 88 | ADC-1 IgG4_S228P_HC-QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLG |
| 89 | ADC-1 IgG4_S228P_HC_ STR+QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDQLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLGK |
| 90 | ADC-1 IgG4_S228P_HC_ STR+QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDQLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLG |
| 91 | ADC-1 IgG4_S228P_HC-FALA+QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLGK |
| 92 | ADC-1 IgG4_S228P_HC_ FALA+QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLG |
| 93 | ADC-1 IgG4_S228P_HC-DHS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHSHYTQKSLSLSLGK |
| 94 | ADC-1 IgG4_S228P_HC-DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHSHYTQKSLSLSLG |
| 95 | ADC-1 IgG4_S228P_HC_ STR+DHS | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA |
| | constant region with terminal lysine | PESTRGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHSHYTQKSLSLSLGK |
| 96 | ADC-1 IgG4_S228P_HC_ STR+DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHSHYTQKSLSLSLG |
| 97 | ADC-1 IgG4_S228P_HC-FALA+DHS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVDHHDWLNGKEYKCKVSNKGLPSSIEKTISKA KGQPREPQVYTLPPSQEEMTKNQVSLTCLVKGFYPS DIAVEWESNGQPENNYKTTPPVLDSDGSFFLYSRLTV DKSRWQEGNVFSCSVMHEALHSHYTQKSLSLSLGK |
| 98 | ADC-1 IgG4_S228P_HC_ FALA+DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVDHHDWLNGKEYKCKVSNKGLPSSIEKTISKA KGQPREPQVYTLPPSQEEMTKNQVSLTCLVKGFYPS DIAVEWESNGQPENNYKTTPPVLDSDGSFFLYSRLTV DKSRWQEGNVFSCSVMHEALHSHYTQKSLSLSLG |
| 99 | ADC-1 IgG4_HC_Wild-type constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 100 | ADC-1 IgG4_HC_Wild-type constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK |
| | | GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 101 | ADC-1 IgG4_HC_STR constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 102 | ADC-1 IgG4_HC_STR constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 103 | ADC-1 IgG4_HC_FALA constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 104 | ADC-1 IgG4_HC_FALA constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 105 | ADC-1 IgG4_HC_YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 106 | ADC-1 IgG4_HC_YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 107 | ADC-1 IgG4_HC_STR+ YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 108 | ADC-1 IgG4_HC_STR+ YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 109 | ADC-1 IgG4_HC_FALA +YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLYITREPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLGK |
| 110 | ADC-1 IgG4_HC_FALA +YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLYITREPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHNHYTQKSLSLSLG |
| 111 | ADC-1 IgG4_HC_REW constant region | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA |
| | with terminal lysine | PEFLGGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLGK |
| 112 | ADC-1 IgG4_HC_REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLG |
| 113 | ADC-1 IgG4_HC_STR+ REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLGK |
| 114 | ADC-1 IgG4_HC_STR+ REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLG |
| 115 | ADC-1 IgG4_HC_FALA +REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHRDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLGK |
| 116 | ADC-1 IgG4_HC_FALA +REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHRDWLNGKEYKCKVSNKGLPSSIEKTISKAK |
| | | GQPREPQVYTLPPSQEEMTKNQVSLTCLVKGFYPSDI AVEWESNGQPENNYKTTPPVLDSDGSFFLYSRLTVD KSRWQEGNVFSCSVEHEALHWHYTQKSLSLSLG |
| 117 | ADC-1 IgG4_HC_LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLGK |
| 118 | ADC-1 IgG4_HC_LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLG |
| 119 | ADC-1 IgG4_HC_STR+ LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLGK |
| 120 | ADC-1 IgG4_HC_STR+ LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLG |
| 121 | ADC-1 IgG4_HC_FALA +LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLGK |
| 122 | ADC-1 IgG4_HC_FALA +LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHSHYTQKSLSLSLG |
| 123 | ADC-1 IgG4_HC_QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLGK |
| 124 | ADC-1 IgG4_HC_QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLG |
| 125 | ADC-1 IgG4_HC_STR+ QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDQLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLGK |
| 126 | ADC-1 IgG4_HC_STR+ QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDQLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLG |
| 127 | ADC-1 IgG4_HC_FALA +QL constant | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA |
| | region with terminal lysine | PEAAGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLGK |
| 128 | ADC-1 IgG4_HC_FALA +QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVLHEALHNHYTQKSLSLSLG |
| 129 | ADC-1 IgG4_HC_DHS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHSHYTQKSLSLSLGK |
| 130 | ADC-1 IgG4_HC_DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHSHYTQKSLSLSLG |
| 131 | ADC-1 IgG4_HC_STR+ DHS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVD KSRWQEGNVFSCSVMHEALHSHYTQKSLSLSLGK |
| 132 | ADC-1 IgG4_HC_STR+ DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK |
| | | GQPREPQVYTLPPSQEEMTKNQVSLTCLVKGFYPSDI AVEWESNGQPENNYKTTPPVLDSDGSFFLYSRLTVD KSRWQEGNVFSCSVMHEALHSHYTQKSLSLSLG |
| 133 | ADC-1 IgG4_HC_FALA +DHS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVDHHDWLNGKEYKCKVSNKGLPSSIEKTISKA KGQPREPQVYTLPPSQEEMTKNQVSLTCLVKGFYPS DIAVEWESNGQPENNYKTTPPVLDSDGSFFLYSRLTV DKSRWQEGNVFSCSVMHEALHSHYTQKSLSLSLGK |
| 134 | ADC-1 IgG4_HC_FALA +DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVDHHDWLNGKEYKCKVSNKGLPSSIEKTISKA KGQPREPQVYTLPPSQEEMTKNQVSLTCLVKGFYPS DIAVEWESNGQPENNYKTTPPVLDSDGSFFLYSRLTV DKSRWQEGNVFSCSVMHEALHSHYTQKSLSLSLG |

In some embodiments, the anti-FRα-antibody of ADC-1 comprises an IgG1 heavy chain comprising (i) a VH comprising the amino acid sequence shown as SEQ ID NO: 4 and (ii) a heavy chain constant region comprising an amino acid sequence shown as any one of SEQ ID NOs: 27 - 62. In some embodiments, the anti-FRα-antibody of ADC-1 may comprise a wild type IgG1 heavy chain constant region comprising amino acid sequences according to SEQ ID NO: 27 (with terminal lysine) and SEQ ID NO: 28 (without terminal lysine). In some embodiments, the IgG1heavy chain constant region comprises Fc silencing mutations selected from L234A, L235A (LALA) or L234S, L235T, G236R (STR). In some embodiments, the anti-FRα-antibody of ADC-1 comprises an IgG1heavy chain constant region comprising the mutations L234A, L235A (LALA) with or without terminal lysine. In some embodiments, the anti-FRα-antibody of ADC-1 comprises an IgG1heavy chain constant region comprising the mutations L234S, L235T, G236R (STR) with or without terminal lysine. In some embodiments, the anti-FRα-antibody of ADC-1 may comprise a heavy chain constant region IgG1comprising half-life extension mutations selected from M252Y, S254T, T256E (YTE); Q311R, M428E, N434W (REW); M428L, N434S (LS); L309D, Q311H, N434S (DHS); or T250Q, M428L (QL). In some embodiments, the anti-FRα-antibody of ADC-1 comprises an IgG1heavy chain constant region comprising M252Y, S254T, T256E (YTE) with or without terminal lysine. In some embodiments, the anti-FRα-antibody of ADC-1 comprises an IgG1 heavy chain constant region comprising Q311R, M428E, N434W (REW) with or without terminal lysine. In some embodiments, the anti-FRα-antibody of ADC-1 comprises an IgG1 heavy chain constant region comprising M428L, N434S (LS) with or without terminal lysine. In some embodiments, the anti-FRα-antibody of ADC-1 comprises an IgG1heavy chain constant region comprising L309D, Q311H, N434S (DHS) with or without terminal lysine. In some embodiments, the anti-FRα-antibody of ADC-1 comprises an IgG1 heavy chain constant region comprising T250Q, M428L (QL) with or without terminal lysine. In some embodiments, the anti-FRα-antibody of ADC-1 constant region comprises both Fc silencing mutations and half-life extension mutations. In some embodiments, the anti-FRα-antibody of ADC-1 comprises an IgG1heavy chain constant region comprising the mutations L234A, L235A (LALA) and M252Y, S254T, T256E (YTE) with or without terminal lysine. In some embodiments, the anti-FRα-antibody of ADC-1 comprises an IgG1heavy chain constant region comprising the mutations L234S, L235T, G236R (STR) and M252Y, S254T, T256E (YTE) with or without terminal lysine. In some embodiments, the anti-FRα-antibody of ADC-1 comprises an IgG1 heavy chain constant region comprising the mutations L234A, L235A (LALA) and Q311R, M428E, N434W (REW) with or without terminal lysine. In some embodiments, the anti-FRα-antibody of ADC-1 comprises an IgG1 heavy chain constant region comprising the mutations L234S, L235T, G236R (STR) and Q311R, M428E, N434W (REW) with or without terminal lysine. In some embodiments, the anti-FRα-antibody of ADC-1 comprises an IgG1 heavy chain constant region comprising the mutations L234A, L235A (LALA) and M428L, N434S (LS) with or without terminal lysine. In some embodiments, the anti-FRα-antibody of ADC-1 comprises an IgG1 heavy chain constant region comprising the mutations L234S, L235T, G236R (STR) and M428L, N434S (LS) with or without terminal lysine. In some embodiments, the anti-FRα-antibody of ADC-1 comprises an IgG1 heavy chain constant region comprising the mutations L234A, L235A (LALA) and L309D, Q311H, N434S (DHS) with or without terminal lysine. In some embodiments, the anti-FRα-antibody of ADC-1 comprises an IgG1 heavy chain constant region comprising the mutations L234S, L235T, G236R (STR) and L309D, Q311H, N434S (DHS) with or without terminal lysine. In some embodiments, the anti-FRα-antibody of ADC-1 comprises an IgG1 heavy chain constant region comprising the mutations L234A, L235A (LALA) and T250Q, M428L (QL) with or without terminal lysine. In some embodiments, the anti-FRα-antibody of ADC-1 comprises an IgG1 heavy chain constant region comprising the mutations L234S, L235T, G236R (STR) and T250Q, M428L (QL) with or without terminal lysine. In some embodiments, the position of the mutations is according to EU numbering.

In some embodiments, the anti-FRα-antibody of ADC-1 comprises an IgG4 heavy chain comprising (i) a VH comprising the amino acid sequence shown as SEQ ID NO: 4 and (ii) a heavy chain constant region comprising an amino acid sequence shown as any one of SEQ ID NOs: 63 - 134. In some embodiments, the anti-FRα-antibody of ADC-1 comprises a wild type IgG4 heavy chain constant region comprising amino acid sequences according to SEQ ID NO: 99 (with terminal lysine) and SEQ ID NO: 100 (without terminal lysine). In some embodiments, the anti-FRα-antibody of ADC-1 may comprise the IgG4 heavy chain constant region further comprising an S228P mutation according to SEQ ID NO: 63 (with terminal lysine) and SEQ ID NO: 64 (without terminal lysine). In some embodiments, the IgG4 heavy chain constant region comprises Fc silencing mutations selected from F234A, L235A (FALA) or L234S, L235T, G236R (STR). In some embodiments, the anti-FRα-antibody of ADC-1 comprises an IgG4 heavy chain constant region comprising the mutations F234A, L235A (FALA) with or without terminal lysine and with or without an S228P mutation. In some embodiments, the anti-FRα-antibody of ADC-1 comprises an IgG4 heavy chain constant region comprising the mutations L234S, L235T, G236R (STR) with or without terminal lysine and with or without an S228P mutation. In some embodiments, the anti-FRα-antibody of ADC-1 may comprise an IgG4 heavy chain constant region comprising half-life extension mutations selected from M252Y, S254T, T256E (YTE); Q311R, M428E, N434W (REW); M428L, N434S (LS); L309D, Q311H, N434S (DHS); or T250Q, M428L (QL). In some embodiments, the anti-FRα-antibody of ADC-1 comprises an IgG4 heavy chain constant region comprising M252Y, S254T, T256E (YTE) with or without terminal lysine and with or without an S228P mutation. In some embodiments, the anti-FRα-antibody of ADC-1 comprises an IgG4 heavy chain constant region comprising Q311R, M428E, N434W (REW) with or without terminal lysine and with or without an S228P mutation. In some embodiments, the anti-FRα-antibody of ADC-1 comprises an IgG4 heavy chain constant region comprising M428L, N434S (LS) with or without terminal lysine and with or without an S228P mutation. In some embodiments, the anti-FRα-antibody of ADC-1 comprises an IgG4 heavy chain constant region comprising L309D, Q311H, N434S (DHS) with or without terminal lysine and with or without an S228P mutation. In some embodiments, the anti-FRα-antibody of ADC-1 comprises an IgG4 heavy chain constant region comprising T250Q, M428L (QL) with or without terminal lysine and with or without an S228P mutation. In some embodiments, the anti-FRα-antibody of ADC-1 constant region may comprise both Fc silencing mutations and half-life extension mutations. In some embodiments, the anti-FRα-antibody of ADC-1 comprises an IgG4 heavy chain constant region comprising the mutations F234A, L235A (FALA) and M252Y, S254T, T256E (YTE) with or without terminal lysine and with or without an S228P mutation. In some embodiments, the anti-FRα-antibody of ADC-1 comprises an IgG4 heavy chain constant region comprising the mutations L234S, L235T, G236R (STR) and M252Y, S254T, T256E (YTE) with or without terminal lysine and with or without an S228P mutation. In some embodiments, the anti-FRα-antibody of ADC-1 comprises an IgG4 heavy chain constant region comprising the mutations F234A, L235A (FALA) and Q311R, M428E, N434W (REW) with or without terminal lysine and with or without an S228P mutation. In some embodiments, the anti-FRα-antibody of ADC-1 comprises an IgG4 heavy chain constant region comprising the mutations L234S, L235T, G236R (STR) and Q311R, M428E, N434W (REW) with or without terminal lysine and with or without an S228P mutation. In some embodiments, the anti-FRα-antibody of ADC-1 comprises an IgG4 heavy chain constant region comprising the mutations F234A, L235A (FALA) and M428L, N434S (LS) with or without terminal lysine and with or without an S228P mutation. In some embodiments, the anti-FRα-antibody of ADC-1 comprises an IgG4 heavy chain constant region comprising the mutations L234S, L235T, G236R (STR) and M428L, N434S (LS) with or without terminal lysine and with or without an S228P mutation. In some embodiments, the anti-FRα-antibody of ADC-1 comprises an IgG4 heavy chain constant region comprising the mutations F234A, L235A (FALA) and L309D, Q311H, N434S (DHS) with or without terminal lysine and with or without an S228P mutation. In some embodiments, the anti-FRα-antibody of ADC-1 comprises an IgG4 heavy chain constant region comprising the mutations L234S, L235T, G236R (STR) and L309D, Q311H, N434S (DHS) with or without terminal lysine and with or without an S228P mutation. In some embodiments, the anti-FRα-antibody of ADC-1 comprises an IgG4 heavy chain constant region comprising the mutations F234A, L235A (FALA) and T250Q, M428L (QL) with or without terminal lysine and with or without an S228P mutation. In some embodiments, the anti-FRα-antibody of ADC-1 comprises an IgG4 heavy chain constant region comprising the mutations L234S, L235T, G236R (STR) and T250Q, M428L (QL) with or without terminal lysine and with or without an S228P mutation. In some embodiments, the position of the mutations is according to EU numbering.

In some embodiments, the anti-FRα bivalent biparatopic antibody of ADC-2 may be in a format, such as an IgGl or IgG4 format, that has been modified to confer desired properties, such as having the Fc region mutated to reduce or "silence" effector function or extend half-life. In some embodiments, where half-life extending and/or Fc silencing mutations are introduced, the Heavy Chain 1 (HC 1) and Heavy Chain 2 (HC 2) of the anti-FRα bivalent biparatopic antibody of ADC-2 may comprise the following Fc region and heavy chain constant region sequences shown in **Tables 8 and 9,** respectively.

**Table 8: Exemplary Mutated and non-Mutated anti-FRα Bivalent Biparatopic Antibody of ADC-2 Heavy Chain 1 (HC 1) Fc Regions**

| SEQ ID NO: | Description | Sequence |
|---|---|---|
| 135 | ADC-2 IgG1 HC 1 Fc region with terminal lysine | DKTHTCPPCPAPELLGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPGK |
| 136 | ADC-2 IgG1 HC 1 Fc region without terminal lysine | DKTHTCPPCPAPELLGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPG |
| 137 | ADC-2 IgG1 HC 1 STR Fc region with terminal lysine | DKTHTCPPCPAPESTRGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPGK |
| 138 | ADC-2 IgG1 HC 1 STR Fc region without terminal lysine | DKTHTCPPCPAPESTRGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPG |
| 139 | ADC-2 IgG1 HC 1 LALA Fc region with terminal lysine | DKTHTCPPCPAPEAAGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPGK |
| 140 | ADC-2 IgG1 HC 1 LALA Fc region without terminal lysine | DKTHTCPPCPAPEAAGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPG |
| 141 | ADC-2 IgG1 HC 1 YTE Fc region with terminal lysine | DKTHTCPPCPAPELLGGPSVFLFPPKPKDTLYITREPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPGK |
| 142 | ADC-2 IgG1 HC 1 YTE Fc region without terminal lysine | DKTHTCPPCPAPELLGGPSVFLFPPKPKDTLYITREPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD |
| | | GSFFLYSKLTVDKSRWQQGNVFSCSVMHEALHNHY TQKSLSLSPG |
| 143 | ADC-2 IgG1 HC 1 STR+YTE Fc region with terminal lysine | DKTHTCPPCPAPESTRGPSVFLFPPKPKDTLYITREPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPGK |
| 144 | ADC-2 IgG1 HC 1 STR+YTE Fc region without terminal lysine | DKTHTCPPCPAPESTRGPSVFLFPPKPKDTLYITREPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPG |
| 145 | ADC-2 IgG1 HC 1 LALA+YTE Fc region with terminal lysine | DKTHTCPPCPAPEAAGGPSVFLFPPKPKDTLYITREPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPGK |
| 146 | ADC-2 IgG1 HC 1 LALA+YTE Fc region without terminal lysine | DKTHTCPPCPAPEAAGGPSVFLFPPKPKDTLYITREPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHNHY TQKSLSLSPG |
| 147 | ADC-2 IgG1 HC 1 REW Fc region with terminal lysine | DKTHTCPPCPAPELLGGPSVFLFPPKPKDTLMISRTPE VTCVVVDVSHEDPEVKFNWYVDGVEVHNAKTKPRE EQYNSTYRVVSVLTVLHRDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVEHEALHWHYT QKSLSLSPGK |
| 148 | ADC-2 IgG1 HC 1 REW Fc region without terminal lysine | DKTHTCPPCPAPELLGGPSVFLFPPKPKDTLMISRTPE VTCVVVDVSHEDPEVKFNWYVDGVEVHNAKTKPRE EQYNSTYRVVSVLTVLHRDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVEHEALHWHYT QKSLSLSPG |
| 149 | ADC-2 IgG1 HC 1 STR+REW Fc region with terminal lysine | DKTHTCPPCPAPESTRGPSVFLFPPKPKDTLMISRTPE VTCVVVDVSHEDPEVKFNWYVDGVEVHNAKTKPRE EQYNSTYRVVSVLTVLHRDWLNGKEYKCKVSNKAL PAPIEKTISKAKGQPREPQVYTLPPSREEMTKNQVSL |
| | | WCLVKGFYPSDIAVEWESNGQPENNYKTTPPVLDSD GSFFLYSKLTVDKSRWQQGNVFSCSVEHEALHWHYT QKSLSLSPGK |
| 150 | ADC-2 IgG1 HC 1 STR+REW Fc region without terminal lysine | DKTHTCPPCPAPESTRGPSVFLFPPKPKDTLMISRTPE VTCVVVDVSHEDPEVKFNWYVDGVEVHNAKTKPRE EQYNSTYRVVSVLTVLHRDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVEHEALHWHYT QKSLSLSPG |
| 151 | ADC-2 IgG1 HC 1 LALA+REW Fc region with terminal lysine | DKTHTCPPCPAPEAAGGPSVFLFPPKPKDTLMISRTPE VTCVVVDVSHEDPEVKFNWYVDGVEVHNAKTKPRE EQYNSTYRVVSVLTVLHRDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVEHEALHWHYT QKSLSLSPGK |
| 152 | ADC-2 IgG1 HC 1 LALA+REW Fc region without terminal lysine | DKTHTCPPCPAPEAAGGPSVFLFPPKPKDTLMISRTPE VTCVVVDVSHEDPEVKFNWYVDGVEVHNAKTKPRE EQYNSTYRVVSVLTVLHRDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVEHEALHWHYT QKSLSLSPG |
| 153 | ADC-2 IgG1 HC 1 LS Fc region with terminal lysine | DKTHTCPPCPAPELLGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHSHYT QKSLSLSPGK |
| 154 | ADC-2 IgG1 HC 1 LS Fc region without terminal lysine | DKTHTCPPCPAPELLGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHSHYT QKSLSLSPG |
| 155 | ADC-2 IgG1 HC 1 STR+LS Fc region with terminal lysine | DKTHTCPPCPAPESTRGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHSHYT QKSLSLSPGK |
| 156 | ADC-2 IgG1 HC 1 STR+LS Fc | DKTHTCPPCPAPESTRGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL |
| | region without terminal lysine | PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHSHYT QKSLSLSPG |
| 157 | ADC-2 IgG1 HC 1 LALA+LS Fc region with terminal lysine | DKTHTCPPCPAPEAAGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHSHYT QKSLSLSPGK |
| 158 | ADC-2 IgG1 HC 1 LALA+LS Fc region without terminal lysine | DKTHTCPPCPAPEAAGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHSHYT QKSLSLSPG |
| 159 | ADC-2 IgG1 HC 1 QL Fc region with terminal lysine | DKTHTCPPCPAPELLGGPSVFLFPPKPKDQLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHNHYT QKSLSLSPGK |
| 160 | ADC-2 IgG1 HC 1 QL Fc region without terminal lysine | DKTHTCPPCPAPELLGGPSVFLFPPKPKDQLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHNHYT QKSLSLSPG |
| 161 | ADC-2 IgG1 HC 1 STR+QL Fc region with terminal lysine | DKTHTCPPCPAPESTRGPSVFLFPPKPKDQLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHNHYT QKSLSLSPGK |
| 162 | ADC-2 IgG1 HC 1 STR+QL Fc region without terminal lysine | DKTHTCPPCPAPESTRGPSVFLFPPKPKDQLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHNHYT QKSLSLSPG |
| 163 | ADC-2 IgG1 HC 1 LALA+QL Fc | DKTHTCPPCPAPEAAGGPSVFLFPPKPKDQLMISRTPE VTCVVVDVSHEDPEVKFNWYVDGVEVHNAKTKPRE |
| | region with terminal lysine | EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHNHYT QKSLSLSPGK |
| 164 | ADC-2 IgG1 HC 1 LALA+QL Fc region without terminal lysine | DKTHTCPPCPAPEAAGGPSVFLFPPKPKDQLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVLHEALHNHYT QKSLSLSPG |
| 165 | ADC-2 IgG1 HC 1 DHS Fc region with terminal lysine | DKTHTCPPCPAPELLGGPSVFLFPPKPKDTLMISRTPE VTCVVVDVSHEDPEVKFNWYVDGVEVHNAKTKPRE EQYNSTYRVVSVLTVDHHDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHSHYT QKSLSLSPGK |
| 166 | ADC-2 IgG1 HC 1 DHS Fc region without terminal lysine | DKTHTCPPCPAPELLGGPSVFLFPPKPKDTLMISRTPE VTCVVVDVSHEDPEVKFNWYVDGVEVHNAKTKPRE EQYNSTYRVVSVLTVDHHDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHSHYT QKSLSLSPG |
| 167 | ADC-2 IgG1 HC 1 STR+DHS Fc region with terminal lysine | DKTHTCPPCPAPESTRGPSVFLFPPKPKDTLMISRTPE VTCVVVDVSHEDPEVKFNWYVDGVEVHNAKTKPRE EQYNSTYRVVSVLTVDHHDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHSHYT QKSLSLSPGK |
| 168 | ADC-2 IgG1 HC 1 STR+DHS Fc region without terminal lysine | DKTHTCPPCPAPESTRGPSVFLFPPKPKDTLMISRTPE VTCVVVDVSHEDPEVKFNWYVDGVEVHNAKTKPRE EQYNSTYRVVSVLTVDHHDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHSHYT QKSLSLSPG |
| 169 | ADC-2 IgG1 HC 1 LALA+DHS Fc region with terminal lysine | DKTHTCPPCPAPEAAGGPSVFLFPPKPKDTLMISRTPE VTCVVVDVSHEDPEVKFNWYVDGVEVHNAKTKPRE EQYNSTYRVVSVLTVDHHDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHSHYT QKSLSLSPGK |
| 170 | ADC-2 IgG1 HC 1 LALA+DHS Fc region without terminal lysine | DKTHTCPPCPAPEAAGGPSVFLFPPKPKDTLMISRTPE VTCVVVDVSHEDPEVKFNWYVDGVEVHNAKTKPRE EQYNSTYRVVSVLTVDHHDWLNGKEYKCKVSNKAL PAPIEKTISKAKGQPREPQVYTLPPSREEMTKNQVSL WCLVKGFYPSDIAVEWESNGQPENNYKTTPPVLDSD GSFFLYSKLTVDKSRWQQGNVFSCSVMHEALHSHYT QKSLSLSPG |
| 171 | ADC-2 IgG4_S228P_HC 1_Wild-type Fc region with terminal lysine | KYGPPCPPCPAPEFLGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLGK |
| 172 | ADC-2 IgG4_S228P_HC 1_Wild-type Fc region without terminal lysine | KYGPPCPPCPAPEFLGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLG |
| 173 | ADC-2 IgG4_S228P HC 1 STR Fc region with terminal lysine | KYGPPCPPCPAPESTRGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLGK |
| 174 | ADC-2 IgG4_S228P HC 1 STR Fc region without terminal lysine | KYGPPCPPCPAPESTRGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLG |
| 175 | ADC-2 IgG4_S228P HC 1 FALA Fc region with terminal lysine | KYGPPCPPCPAPEAAGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLGK |
| 176 | ADC-2 IgG4_S228P HC 1 FALA Fc region without terminal lysine | KYGPPCPPCPAPEAAGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD |
| | | GSFFLYSRLTVDKSRWQEGNVFSCSVMHEALHNHYT QKSLSLSLG |
| 177 | ADC-2 IgG4_S228P HC 1 YTE Fc region with terminal lysine | KYGPPCPPCPAPEFLGGPSVFLFPPKPKDTLYITREPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLGK |
| 178 | ADC-2 IgG4_S228P HC 1 YTE Fc region without terminal lysine | KYGPPCPPCPAPEFLGGPSVFLFPPKPKDTLYITREPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLG |
| 179 | ADC-2 IgG4_S228P HC 1 STR+YTE Fc region with terminal lysine | KYGPPCPPCPAPESTRGPSVFLFPPKPKDTLYITREPEV TCVVVDVSQEDPEVQFNWYVDGVEVHNAKTKPREE QFNSTYRVVSVLTVLHQDWLNGKEYKCKVSNKGLP SSIEKTISKAKGQPREPQVYTLPPSQEEMTKNQVSLW CLVKGFYPSDIAVEWESNGQPENNYKTTPPVLDSDGS FFLYSRLTVDKSRWQEGNVFSCSVMHEALHNHYTQ KSLSLSLGK |
| 180 | ADC-2 IgG4_S228P HC 1 STR+YTE Fc region without terminal lysine | KYGPPCPPCPAPESTRGPSVFLFPPKPKDTLYITREPEV TCVVVDVSQEDPEVQFNWYVDGVEVHNAKTKPREE QFNSTYRVVSVLTVLHQDWLNGKEYKCKVSNKGLP SSIEKTISKAKGQPREPQVYTLPPSQEEMTKNQVSLW CLVKGFYPSDIAVEWESNGQPENNYKTTPPVLDSDGS FFLYSRLTVDKSRWQEGNVFSCSVMHEALHNHYTQ KSLSLSLG |
| 181 | ADC-2 IgG4_S228P HC 1 FALA+YTE Fc region with terminal lysine | KYGPPCPPCPAPEAAGGPSVFLFPPKPKDTLYITREPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLGK |
| 182 | ADC-2 IgG4_S228P HC 1 FALA+YTE Fc region without terminal lysine | KYGPPCPPCPAPEAAGGPSVFLFPPKPKDTLYITREPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLG |
| 183 | ADC-2 IgG4_S228P HC 1 REW Fc region | KYGPPCPPCPAPEFLGGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL |
| | with terminal lysine | WCLVKGFYPSDIAVEWESNGQPENNYKTTPPVLDSD GSFFLYSRLTVDKSRWQEGNVFSCSVEHEALHWHYT QKSLSLSLGK |
| 184 | ADC-2 IgG4_S228P HC 1 REW Fc region without terminal lysine | KYGPPCPPCPAPEFLGGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLG |
| 185 | ADC-2 IgG4_S228P HC 1 STR+REW Fc region with terminal lysine | KYGPPCPPCPAPESTRGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLGK |
| 186 | ADC-2 IgG4_S228P HC 1 STR+REW Fc region without terminal lysine | KYGPPCPPCPAPESTRGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLG |
| 187 | ADC-2 IgG4_S228P HC 1 FALA+REW Fc region with terminal lysine | KYGPPCPPCPAPEAAGGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLGK |
| 188 | ADC-2 IgG4_S228P HC 1 FALA+REW Fc region without terminal lysine | KYGPPCPPCPAPEAAGGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLG |
| 189 | ADC-2 IgG4_S228P HC 1 LS Fc region with terminal lysine | KYGPPCPPCPAPEFLGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLGK |
| 190 | ADC-2 IgG4_S228P HC 1 LS Fc region | KYGPPCPPCPAPEFLGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL |
| | without terminal lysine | PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLG |
| 191 | ADC-2 IgG4_S228P HC 1 STR+LS Fc region with terminal lysine | KYGPPCPPCPAPESTRGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLGK |
| 192 | ADC-2 IgG4_S228P HC 1 STR+LS Fc region without terminal lysine | KYGPPCPPCPAPESTRGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLG |
| 193 | ADC-2 IgG4_S228P HC 1 FALA+LS Fc region with terminal lysine | KYGPPCPPCPAPEAAGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLGK |
| 194 | ADC-2 IgG4_S228P HC 1 FALA+LS Fc region without terminal lysine | KYGPPCPPCPAPEAAGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLG |
| 195 | ADC-2 IgG4_S228P HC 1 QL Fc region with terminal lysine | KYGPPCPPCPAPEFLGGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLGK |
| 196 | ADC-2 IgG4_S228P HC 1 QL Fc region without terminal lysine | KYGPPCPPCPAPEFLGGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLG |
| 197 | ADC-2 IgG4_S228P HC 1 | KYGPPCPPCPAPESTRGPSVFLFPPKPKDQLMISRTPE VTCVVVDVSQEDPEVQFNWYVDGVEVHNAKTKPRE |
| | STR+QL Fc region with terminal lysine | EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLGK |
| 198 | ADC-2 IgG4_S228P HC 1 STR+QL Fc region without terminal lysine | KYGPPCPPCPAPESTRGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLG |
| 199 | ADC-2 IgG4_S228P HC 1 FALA+QL Fc region with terminal lysine | KYGPPCPPCPAPEAAGGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLGK |
| 200 | ADC-2 IgG4_S228P HC 1 FALA+QL Fc region without terminal lysine | KYGPPCPPCPAPEAAGGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLG |
| 201 | ADC-2 IgG4_S228P HC 1 DHS Fc region with terminal lysine | KYGPPCPPCPAPEFLGGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLGK |
| 202 | ADC-2 IgG4_S228P HC 1 DHS Fc region without terminal lysine | KYGPPCPPCPAPEFLGGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLG |
| 203 | ADC-2 IgG4_S228P HC 1 STR+DHS Fc region with terminal lysine | KYGPPCPPCPAPESTRGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLGK |
| 204 | ADC-2 IgG4_S228P HC 1 STR+DHS Fc region without terminal lysine | KYGPPCPPCPAPESTRGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLG |
| 205 | ADC-2 IgG4_S228P HC 1 FALA+DHS Fc region with terminal lysine | KYGPPCPPCPAPEAAGGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLGK |
| 206 | ADC-2 IgG4_S228P HC 1 FALA+DHS Fc region without terminal lysine | KYGPPCPPCPAPEAAGGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLG |
| 207 | ADC-2 IgG4_HC 1_Wild-type Fc region with terminal lysine | KYGPPCPSCPAPEFLGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLGK |
| 208 | ADC-2 IgG4_HC 1_Wild-type Fc region without terminal lysine | KYGPPCPSCPAPEFLGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLG |
| 209 | ADC-2 IgG4HC 1_STR Fc region with terminal lysine | KYGPPCPSCPAPESTRGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLGK |
| 210 | ADC-2 IgG4_HC 1 STR Fc region without terminal lysine | KYGPPCPSCPAPESTRGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD |
| | | GSFFLYSRLTVDKSRWQEGNVFSCSVMHEALHNHYT QKSLSLSLG |
| 211 | ADC-2 IgG4_HC 1 FALA Fc region with terminal lysine | KYGPPCPSCPAPEAAGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLGK |
| 212 | ADC-2 IgG4_HC 1 FALA Fc region without terminal lysine | KYGPPCPSCPAPEAAGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLG |
| 213 | ADC-2 IgG4_HC 1 YTE Fc region with terminal lysine | KYGPPCPSCPAPEFLGGPSVFLFPPKPKDTLYITREPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLGK |
| 214 | ADC-2 IgG4_HC 1 YTE Fc region without terminal lysine | KYGPPCPSCPAPEFLGGPSVFLFPPKPKDTLYITREPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLG |
| 215 | ADC-2 IgG4_HC 1 STR+YTE Fc region with terminal lysine | KYGPPCPSCPAPESTRGPSVFLFPPKPKDTLYITREPEV TCVVVDVSQEDPEVQFNWYVDGVEVHNAKTKPREE QFNSTYRVVSVLTVLHQDWLNGKEYKCKVSNKGLP SSIEKTISKAKGQPREPQVYTLPPSQEEMTKNQVSLW CLVKGFYPSDIAVEWESNGQPENNYKTTPPVLDSDGS FFLYSRLTVDKSRWQEGNVFSCSVMHEALHNHYTQ KSLSLSLGK |
| 216 | ADC-2 IgG4_HC 1 STR+YTE Fc region without terminal lysine | KYGPPCPSCPAPESTRGPSVFLFPPKPKDTLYITREPEV TCVVVDVSQEDPEVQFNWYVDGVEVHNAKTKPREE QFNSTYRVVSVLTVLHQDWLNGKEYKCKVSNKGLP SSIEKTISKAKGQPREPQVYTLPPSQEEMTKNQVSLW CLVKGFYPSDIAVEWESNGQPENNYKTTPPVLDSDGS FFLYSRLTVDKSRWQEGNVFSCSVMHEALHNHYTQ KSLSLSLG |
| 217 | ADC-2 IgG4_HC 1 FALA+YTE Fe region with terminal lysine | KYGPPCPSCPAPEAAGGPSVFLFPPKPKDTLYITREPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL |
| | | WCLVKGFYPSDIAVEWESNGQPENNYKTTPPVLDSD GSFFLYSRLTVDKSRWQEGNVFSCSVMHEALHNHYT QKSLSLSLGK |
| 218 | ADC-2 IgG4_HC 1 FALA+YTE Fe region without terminal lysine | KYGPPCPSCPAPEAAGGPSVFLFPPKPKDTLYITREPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHNHYT QKSLSLSLG |
| 219 | ADC-2 IgG4_HC 1 REW Fc region with terminal lysine | KYGPPCPSCPAPEFLGGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLGK |
| 220 | ADC-2 IgG4_HC 1 REW Fc region without terminal lysine | KYGPPCPSCPAPEFLGGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLG |
| 221 | ADC-2 IgG4_HC 1 STR+REW Fc region with terminal lysine | KYGPPCPSCPAPESTRGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLGK |
| 222 | ADC-2 IgG4_HC 1 STR+REW Fc region without terminal lysine | KYGPPCPSCPAPESTRGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLG |
| 223 | ADC-2 IgG4_HC 1 FALA+REW Fc region with terminal lysine | KYGPPCPSCPAPEAAGGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLGK |
| 224 | ADC-2 IgG4_HC 1 FALA+REW Fc | KYGPPCPSCPAPEAAGGPSVFLFPPKPKDTLMISRTPE VTCVVVDVSQEDPEVQFNWYVDGVEVHNAKTKPRE EQFNSTYRVVSVLTVLHRDWLNGKEYKCKVSNKGL |
| | region without terminal lysine | PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVEHEALHWHYT QKSLSLSLG |
| 225 | ADC-2 IgG4_HC 1 LS Fc region with terminal lysine | KYGPPCPSCPAPEFLGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLGK |
| 226 | ADC-2 IgG4_HC 1 LS Fc region without terminal lysine | KYGPPCPSCPAPEFLGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLG |
| 227 | ADC-2 IgG4_HC 1 STR+LS Fc region with terminal lysine | KYGPPCPSCPAPESTRGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLGK |
| 228 | ADC-2 IgG4_HC 1 STR+LS Fc region without terminal lysine | KYGPPCPSCPAPESTRGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLG |
| 229 | ADC-2 IgG4_HC 1 FALA+LS Fc region with terminal lysine | KYGPPCPSCPAPEAAGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLGK |
| 230 | ADC-2 IgG4_HC 1 FALA+LS Fc region without terminal lysine | KYGPPCPSCPAPEAAGGPSVFLFPPKPKDTLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHSHYT QKSLSLSLG |
| 231 | ADC-2 IgG4_HC 1 QL Fc region | KYGPPCPSCPAPEFLGGPSVFLFPPKPKDQLMISRTPE VTCVVVDVSQEDPEVQFNWYVDGVEVHNAKTKPRE |
| | with terminal lysine | EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLGK |
| 232 | ADC-2 IgG4_HC 1 QL Fc region without terminal lysine | KYGPPCPSCPAPEFLGGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLG |
| 233 | ADC-2 IgG4_HC 1 STR+QL Fc region with terminal lysine | KYGPPCPSCPAPESTRGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLGK |
| 234 | ADC-2 IgG4_HC 1 STR+QL Fc region without terminal lysine | KYGPPCPSCPAPESTRGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLG |
| 235 | ADC-2 IgG4_HC 1 FALA+QL Fc region with terminal lysine | KYGPPCPSCPAPEAAGGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLGK |
| 236 | ADC-2 IgG4_HC 1 FALA+QL Fc region without terminal lysine | KYGPPCPSCPAPEAAGGPSVFLFPPKPKDQLMISRTPE VTCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVLHEALHNHYT QKSLSLSLG |
| 237 | ADC-2 IgG4_HC 1 DHS Fc region with terminal lysine | KYGPPCPSCPAPEFLGGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLGK |
| 238 | ADC-2 IgG4_HC 1 DHS Fc region without terminal lysine | KYGPPCPSCPAPEFLGGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLG |
| 239 | ADC-2 IgG4_HC 1 STR+DHS Fc region with terminal lysine | KYGPPCPSCPAPESTRGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLGK |
| 240 | ADC-2 IgG4_HC 1 STR+DHS Fc region without terminal lysine | KYGPPCPSCPAPESTRGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLG |
| 241 | ADC-2 IgG4_HC 1 FALA+DHS Fc region with terminal lysine | KYGPPCPSCPAPEAAGGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLGK |
| 242 | ADC-2 IgG4_HC 1 FALA+DHS Fc region without terminal lysine | KYGPPCPSCPAPEAAGGPSVFLFPPKPKDTLMISRTPE VTCVVVDVSQEDPEVQFNWYVDGVEVHNAKTKPRE EQFNSTYRVVSVLTVDHHDWLNGKEYKCKVSNKGL PSSIEKTISKAKGQPREPQVYTLPPSQEEMTKNQVSL WCLVKGFYPSDIAVEWESNGQPENNYKTTPPVLDSD GSFFLYSRLTVDKSRWQEGNVFSCSVMHEALHSHYT QKSLSLSLG |

In some embodiments, the anti-FRα bivalent biparatopic antibody of ADC-2 comprises a heavy chain 1 (HC 1 or first polypeptide) comprising (i) the scFv-hinge polypeptide of SEQ ID NO: 351 and (ii) an IgG1 Fc region comprising an amino acid sequence shown as any one of SEQ ID NOs: 135 - 170. In some embodiments, the anti-FRα bivalent biparatopic antibody heavy chain 1 (HC 1) may comprise a wild type IgG1 Fc region comprising amino acid sequences according to SEQ ID NO: 135 (with terminal lysine) and SEQ ID NO: 136 (without terminal lysine). In some embodiments, the IgG1 Fc region comprises Fc silencing mutations selected from L234A, L235A (LALA) or L234S, L235T, G236R (STR). In some embodiments, the anti-FRα bivalent biparatopic antibody heavy chain 1 (HC 1) comprises an IgG1 Fc region comprising the mutations L234A, L235A (LALA) with or without terminal lysine. In some embodiments, the anti-FRα bivalent biparatopic antibody heavy chain 1 (HC 1) comprises an IgG1 Fc region comprising the mutations L234S, L235T, G236R (STR) with or without terminal lysine. In some embodiments, the anti-FRα bivalent biparatopic antibody heavy chain 1 (HC 1) may comprise an IgG Fc region comprising half-life extension mutations selected from M252Y, S254T, T256E (YTE); Q311R, M428E, N434W (REW); M428L, N434S (LS); L309D, Q311H, N434S (DHS); or T250Q, M428L (QL). In some embodiments, the anti-FRα bivalent biparatopic antibody heavy chain 1 (HC 1) comprises an IgG1 Fc region comprising M252Y, S254T, T256E (YTE) with or without terminal lysine. In some embodiments, the anti-FRα bivalent biparatopic antibody heavy chain 1 (HC 1) comprises an IgG1 Fc region comprising Q311R, M428E, N434W (REW) with or without terminal lysine. In some embodiments, the anti-FRα bivalent biparatopic antibody heavy chain 1 (HC 1) comprises an IgG1 Fc region comprising M428L, N434S (LS) with or without terminal lysine. In some embodiments, the anti-FRα bivalent biparatopic antibody heavy chain 1 (HC 1) comprises an IgG1 Fc region comprising L309D, Q311H, N434S (DHS) with or without terminal lysine. In some embodiments, the anti-FRα bivalent biparatopic antibody heavy chain 1 (HC 1) comprises an IgG1 Fc region comprising T250Q, M428L (QL) with or without terminal lysine. In some embodiments, the anti-FRα bivalent biparatopic antibody heavy chain 1 (HC 1) Fc region may comprise both Fc silencing mutations and half-life extension mutations. In some embodiments, the anti-FRα bivalent biparatopic antibody heavy chain 1 (HC 1) comprises an IgG1 Fc region comprising the mutations L234A, L235A (LALA) and M252Y, S254T, T256E (YTE) with or without terminal lysine. In some embodiments, anti-FRα bivalent biparatopic antibody heavy chain 1 (HC 1) comprises an IgG1 Fc region comprising the mutations L234S, L235T, G236R (STR) and M252Y, S254T, T256E (YTE) with or without terminal lysine. In some embodiments, the anti-FRα bivalent biparatopic antibody heavy chain 1 (HC 1) comprises an IgG1 Fc region comprising the mutations L234A, L235A (LALA) and Q311R, M428E, N434W (REW) with or without terminal lysine. In some embodiments, the anti-FRα bivalent biparatopic antibody heavy chain 1 (HC 1) comprises an IgG1 Fc region comprising the mutations L234S, L235T, G236R (STR) and Q311R, M428E, N434W (REW) with or without terminal lysine. In some embodiments, the anti-FRα bivalent biparatopic antibody heavy chain 1 (HC 1) comprises an IgG1 Fc region comprising the mutations L234A, L235A (LALA) and M428L, N434S (LS) with or without terminal lysine. In some embodiments, the anti-FRα bivalent biparatopic antibody heavy chain 1 (HC 1) comprises an IgG1 Fc region comprising the mutations L234S, L235T, G236R (STR) and M428L, N434S (LS) with or without terminal lysine. In some embodiments, the anti-FRα bivalent biparatopic antibody heavy chain 1 (HC 1) comprises an IgG1 Fc region comprising the mutations L234A, L235A (LALA) and L309D, Q311H, N434S (DHS) with or without terminal lysine. In some embodiments, the anti-FRα bivalent biparatopic antibody heavy chain 1 (HC 1) comprises an IgG1 Fc region comprising the mutations L234S, L235T, G236R (STR) and L309D, Q311H, N434S (DHS) with or without terminal lysine. In some embodiments, the anti-FRα bivalent biparatopic antibody heavy chain 1 (HC 1) comprises an IgG1 Fc region comprising the mutations L234A, L235A (LALA) and T250Q, M428L (QL) with or without terminal lysine. In some embodiments, the anti-FRα bivalent biparatopic antibody heavy chain 1 (HC 1) comprises an IgG1 Fc region comprising the mutations L234S, L235T, G236R (STR) and T250Q, M428L (QL) with or without terminal lysine. In some embodiments, the position of the mutations is according to EU numbering.

In some embodiments, the anti-FRα bivalent biparatopic antibody of ADC-2 comprises a heavy chain 1 (HC 1 or first polypeptide) comprising (i) the scFv-hinge polypeptide of SEQ ID NO: 351 and (ii) an IgG4 Fc region comprising an amino acid sequence shown as any one of SEQ ID NOs: 171 - 242. In some embodiments, the anti-FRα bivalent biparatopic antibody heavy chain 1 (HC 1) may comprise a wild type IgG4 Fc region comprising amino acid sequences according to SEQ ID NO: 207 (with terminal lysine) and SEQ ID NO: 208 (without terminal lysine). In some embodiments, the anti-FRα bivalent biparatopic antibody heavy chain 1 (HC 1) may comprise the IgG4 Fc region comprising an S228P mutation according to SEQ ID NO: 171 (with terminal lysine) and SEQ ID NO: 172 (without terminal lysine). In some embodiments, the IgG4 Fc region comprises Fc silencing mutations selected from F234A, L235A (FALA) or L234S, L235T, G236R (STR). In some embodiments, the anti-FRα bivalent biparatopic antibody heavy chain 1 (HC 1) comprises an IgG4 Fc region comprising the mutations F234A, L235A (FALA) with or without terminal lysine and with or without an S228P mutation. In some embodiments, the anti-FRα bivalent biparatopic antibody heavy chain 1 (HC 1) comprises an IgG4 Fc region comprising the mutations L234S, L235T, G236R (STR) with or without terminal lysine and with or without an S228P mutation. In some embodiments, the anti-FRα bivalent biparatopic antibody heavy chain 1 (HC 1) may comprise an IgG4 Fc region comprising half-life extension mutations selected from M252Y, S254T, T256E (YTE); Q311R, M428E, N434W (REW); M428L, N434S (LS); L309D, Q311H, N434S (DHS); or T250Q, M428L (QL). In some embodiments, anti-FRα bivalent biparatopic antibody heavy chain 1 (HC 1) comprises an IgG4 Fc region comprising M252Y, S254T, T256E (YTE) with or without terminal lysine and with or without an S228P mutation. In some embodiments, the anti-FRα bivalent biparatopic antibody heavy chain 1 (HC 1) comprises an IgG4 Fc region comprising Q311R, M428E, N434W (REW) with or without terminal lysine and with or without an S228P mutation. In some embodiments, the anti-FRα bivalent biparatopic antibody heavy chain 1 (HC 1) comprises an IgG4 Fc region comprising M428L, N434S (LS) with or without terminal lysine and with or without an S228P mutation. In some embodiments, the anti-FRα bivalent biparatopic antibody heavy chain 1 (HC 1) comprises an IgG4 Fc region comprising L309D, Q311H, N434S (DHS) with or without terminal lysine and with or without an S228P mutation. In some embodiments, the anti-FRα bivalent biparatopic antibody heavy chain 1 (HC 1) comprises an IgG4 Fc region comprising T250Q, M428L (QL) with or without terminal lysine and with or without an S228P mutation. In some embodiments, the anti-FRα bivalent biparatopic antibody heavy chain 1 (HC 1) constant region may comprise both Fc silencing mutations and half-life extension mutations. In some embodiments, the anti-FRα bivalent biparatopic antibody heavy chain 1 (HC 1) comprises an IgG4 Fc region comprising the mutations F234A, L235A (FALA) and M252Y, S254T, T256E (YTE) with or without terminal lysine and with or without an S228P mutation. In some embodiments, the anti-FRα bivalent biparatopic antibody heavy chain 1 (HC 1) comprises an IgG4 Fc region comprising the mutations L234S, L235T, G236R (STR) and M252Y, S254T, T256E (YTE) with or without terminal lysine and with or without an S228P mutation. In some embodiments, the anti-FRα bivalent biparatopic antibody heavy chain 1 (HC 1) comprises an IgG4 Fc region comprising the mutations F234A, L235A (FALA) and Q311R, M428E, N434W (REW) with or without terminal lysine and with or without an S228P mutation. In some embodiments, the anti-FRα bivalent biparatopic antibody heavy chain 1 (HC 1) comprises an IgG4 Fc region comprising the mutations L234S, L235T, G236R (STR) and Q311R, M428E, N434W (REW) with or without terminal lysine and with or without an S228P mutation. In some embodiments, the anti-FRα bivalent biparatopic antibody heavy chain 1 (HC 1) comprises an IgG4 Fc region comprising the mutations F234A, L235A (FALA) and M428L, N434S (LS) with or without terminal lysine and with or without an S228P mutation. In some embodiments, the anti-FRα bivalent biparatopic antibody heavy chain 1 (HC 1) comprises an IgG4 Fc region comprising the mutations L234S, L235T, G236R (STR) and M428L, N434S (LS) with or without terminal lysine and with or without an S228P mutation. In some embodiments, the anti-FRα bivalent biparatopic antibody heavy chain 1 (HC 1) comprises an IgG4 Fc region comprising the mutations F234A, L235A (FALA) and L309D, Q311H, N434S (DHS) with or without terminal lysine and with or without an S228P mutation. In some embodiments, the anti-FRα bivalent biparatopic antibody heavy chain 1 (HC 1) comprises an IgG4 Fc region comprising the mutations L234S, L235T, G236R (STR) and L309D, Q311H, N434S (DHS) with or without terminal lysine and with or without an S228P mutation. In some embodiments, the anti-FRα bivalent biparatopic antibody heavy chain 1 (HC 1) comprises an IgG4 Fc region comprising the mutations F234A, L235A (FALA) and T250Q, M428L (QL) with or without terminal lysine and with or without an S228P mutation. In some embodiments, the anti-FRα bivalent biparatopic antibody heavy chain 1 (HC 1) comprises an IgG4 Fc region comprising the mutations L234S, L235T, G236R (STR) and T250Q, M428L (QL) with or without terminal lysine and with or without an S228P mutation. In some embodiments, the position of the mutations is according to EU numbering.

**Table 9: Exemplary Fc Mutated and non-Mutated ADC-2 anti-FRα Antibody Heavy Chain 2 (HC 2) Constant Regions**

| SEQ ID NO: | Description | Sequence |
|---|---|---|
| 243 | ADC-2 IgG1 HC 2 constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVMHEALHNHYTQKSLSLSP GK |
| 244 | ADC-2 IgG1 HC 2 constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVMHEALHNHYTQKSLSLSP G |
| 245 | ADC-2 IgG1 HC 2 STR constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVMHEALHNHYTQKSLSLSP GK |
| 246 | ADC-2 IgG1 HC 2 STR constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVMHEALHNHYTQKSLSLSP G |
| 247 | ADC-2 IgG1 HC 2 LALA constant | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP |
| | region with terminal lysine | CPAPEAAGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVMHEALHNHYTQKSLSLSP GK |
| 248 | ADC-2 IgG1 HC 2 LALA constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVMHEALHNHYTQKSLSLSP G |
| 249 | ADC-2 IgG1 HC 2 YTE constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLYITREPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVMHEALHNHYTQKSLSLSP GK |
| 250 | ADC-2 IgG1 HC 2 YTE constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLYITREPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVMHEALHNHYTQKSLSLSP G |
| 251 | ADC-2 IgG1 HC 2 STR+YTE constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLYITREPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVMHEALHNHYTQKSLSLSP GK |
| 252 | ADC-2 IgG1 HC 2 STR+YTE constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLYITREPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVMHEALHNHYTQKSLSLSP G |
| 253 | ADC-2 IgG1 HC 2 LALA+YTE constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLYITREPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVMHEALHNHYTQKSLSLSP GK |
| 254 | ADC-2 IgG1 HC 2 LALA+YTE constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLYITREPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVMHEALHNHYTQKSLSLSP G |
| 255 | ADC-2 IgG1 HC 2 REW constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHRDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVEHEALHWHYTQKSLSLSP GK |
| 256 | ADC-2 IgG1 HC 2 REW constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHRDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK |
| | | LTVDKSRWQQGNVFSCSVEHEALHWHYTQKSLSLSP G |
| 257 | ADC-2 IgG1 HC 2 STR+REW constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHRDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVEHEALHWHYTQKSLSLSPG K |
| 258 | ADC-2 IgG1 HC 2 STR+REW constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHRDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVEHEALHWHYTQKSLSLSPG |
| 259 | ADC-2 IgG1 HC 2 LALA+REW constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHRDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVEHEALHWHYTQKSLSLSP GK |
| 260 | ADC-2 IgG1 HC 2 LALA+REW constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHRDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVEHEALHWHYTQKSLSLSP G |
| 261 | ADC-2 IgG1 HC 2 LS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF |
| | | YPSDIAVEWESNGQPENNYKTTPPVLDSDGSFFLVSK LTVDKSRWQQGNVFSCSVLHEALHSHYTQKSLSLSP GK |
| 262 | ADC-2 IgG1 HC 2 LS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVLHEALHSHYTQKSLSLSP G |
| 263 | ADC-2 IgG1 HC 2 STR+LS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVLHEALHSHYTQKSLSLSPG K |
| 264 | ADC-2 IgG1 HC 2 STR+LS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVLHEALHSHYTQKSLSLSPG |
| 265 | ADC-2 IgG1 HC 2 LALA+LS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVLHEALHSHYTQKSLSLSP GK |
| 266 | ADC-2 IgG1 HC 2 LALA+LS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI |
| | | SKAKGQPREPQVYTLPPSREEMTKNQVSLSCAVKGF YPSDIAVEWESNGQPENNYKTTPPVLDSDGSFFLVSK LTVDKSRWQQGNVFSCSVLHEALHSHYTQKSLSLSP G |
| 267 | ADC-2 IgG1 HC 2 QL constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVLHEALHNHYTQKSLSLSP GK |
| 268 | ADC-2 IgG1 HC 2 QL constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVLHEALHNHYTQKSLSLSP G |
| 269 | ADC-2 IgG1 HC 2 STR+QL constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVLHEALHNHYTQKSLSLSP GK |
| 270 | ADC-2 IgG1 HC 2 STR+QL constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVLHEALHNHYTQKSLSLSP G |
| 271 | ADC-2 IgG1 HC 2 LALA+QL constant region | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDOLMISRTPEVTCVVVD |
| | with terminal lysine | VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVLHEALHNHYTQKSLSLSP GK |
| 272 | ADC-2 IgG1 HC 2 LALA+QL constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDQLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVLHEALHNHYTQKSLSLSP G |
| 273 | ADC-2 IgG1 HC 2 DHS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVDHHDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVMHEALHSHYTQKSLSLSP GK |
| 274 | ADC-2 IgG1 HC 2 DHS constant region without terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVD VSHEDPEVKFNWYVDGVEVHNAKTKPREEQYNSTY RVVSVLTVDHHDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVMHEALHSHYTQKSLSLSP G |
| 275 | ADC-2 IgG1 HC 2 STR+DHS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVDHHDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVMHEALHSHYTQKSLSLSPG K |
| 276 | ADC-2 IgG1 HC 2 STR+DHS | ASTKGPSVFPLAPSSKSTSGGTAALGCLVKDYFPEPV TVSWNSGALTSGVHTFPAVLOSSGLYSLSSVVTVPSS |
| | constant region without terminal lysine | SLGTQTYICNVNHKPSNTKVDKKVEPKSCDKTHTCPP CPAPESTRGPSVFLFPPKPKDTLMISRTPEVTCVVVDV SHEDPEVKFNWYVDGVEVHNAKTKPREEQYNSTYR VVSVLTVDHHDWLNGKEYKCKVSNKALPAPIEKTIS KAKGQPREPQVYTLPPSREEMTKNQVSLSCAVKGFY PSDIAVEWESNGQPENNYKTTPPVLDSDGSFFLVSKL TVDKSRWQQGNVFSCSVMHEALHSHYTQKSLSLSPG |
| 277 | ADC-2 IgG1 HC 2 LALA+DHS constant region with terminal lysine | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVD VSHEDPEVKFNWYVDGVEVHNAKTKPREEQYNSTY RVVSVLTVDHHDWLNGKEYKCKVSNKALPAPIEKTI SKAKGQPREPQVYTLPPSREEMTKNQVSLSCAVKGF YPSDIAVEWESNGQPENNYKTTPPVLDSDGSFFLVSK LTVDKSRWQQGNVFSCSVMHEALHSHYTQKSLSLSP GK |
| 278 | ADC-2 IgG1 HC 2 LALA+DHS constant region without terminal lysine | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCP APEAAGGPSVFLFPPKPKDTLMISRTPEVTCVVVDVSH EDPEVKFNWYVDGVEVHNAKTKPREEQYNSTYRVVS VLTVDHHDWLNGKEYKCKVSNKALPAPIEKTISKAK GQPREPQVYTLPPSREEMTKNQVSLSCAVKGFYPSDI AVEWESNGQPENNYKTTPPVLDSDGSFFLVSKLTVDK SRWQQGNVFSCSVMHEALHSHYTQKSLSLSPG |
| 279 | ADC-2 IgG4_S228P HC 2 constant region with terminal lysine | ASTKGPSVFPLAPCSRSTSESTAALGCLVKDYFPEPVT VSWNSGALTSGVHTFPAVLQSSGLYSLSSVVTVPSSSL GTKTYTCNVDHKPSNTKVDKRVESKYGPPCPPCPAPE FLGGPSVFLFPPKPKDTLMISRTPEVTCVVVDVSQEDP EVQFNWYVDGVEVHNAKTKPREEQFNSTYRVVSVLT VLHQDWLNGKEYKCKVSNKGLPSSIEKTISKAKGQPR EPQVYTLPPSQEEMTKNQVSLSCAVKGFYPSDIAVEW ESNGQPENNYKTTPPVLDSDGSFFLVSRLTVDKSRWQ EGNVFSCSVMHEALHNHYTQKSLSLSLGK |
| 280 | ADC-2 IgG4_S228P HC 2 constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 281 | ADC-2 IgG4_S228P HC 2 STR constant | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSOE |
| | region with terminal lysine | DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 282 | ADC-2 IgG4_S228P HC 2 STR constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 283 | ADC-2 IgG4_S228P HC 2 FALA constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 284 | ADC-2 IgG4_S228P HC 2 FALA constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 285 | ADC-2 IgG4_S228P HC 2 YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 286 | ADC-2 IgG4_S228P HC 2 YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI |
| | | AVEWESNGQPENNYKTTPPVLDSDGSFFLVSRLTVD KSRWQEGNVFSCSVMHEALHNHYTQKSLSLSLG |
| 287 | ADC-2 IgG4_S228P HC 2 STR+YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 288 | ADC-2 IgG4_S228P HC 2 STR+YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 289 | ADC-2 IgG4_S228P HC 2 FALA+YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLYITREPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 290 | ADC-2 IgG4_S228P HC 2 FALA+YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLYITREPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 291 | ADC-2 IgG4_S228P HC 2 REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLGK |
| 292 | ADC-2 IgG4_S228P HC 2 REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLG |
| 293 | ADC-2 IgG4_S228P HC 2 STR+REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLGK |
| 294 | ADC-2 IgG4_S228P HC 2 STR+REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLG |
| 295 | ADC-2 IgG4_S228P HC 2 FALA+REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLGK |
| 296 | ADC-2 IgG4_S228P HC 2 FALA+REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLG |
| 297 | ADC-2 IgG4_S228P HC 2 LS constant region | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA |
| | with terminal lysine | PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLGK |
| 298 | ADC-2 IgG4_S228P HC 2 LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLG |
| 299 | ADC-2 IgG4_S228P HC 2 STR+LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLGK |
| 300 | ADC-2 IgG4_S228P HC 2 STR+LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLG |
| 301 | ADC-2 IgG4_S228P HC 2 FALA+LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLGK |
| 302 | ADC-2 IgG4_S228P HC 2 FALA+LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK |
| | | GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLG |
| 303 | ADC-2 IgG4_S228P HC 2 QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLGK |
| 304 | ADC-2 IgG4_S228P HC 2 QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLG |
| 305 | ADC-2 IgG4_S228P HC 2 STR+QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDQLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLGK |
| 306 | ADC-2 IgG4_S228P HC 2 STR+QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDQLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLG |
| 307 | ADC-2 IgG4_S228P HC 2 FALA+QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLGK |
| 308 | ADC-2 IgG4_S228P HC 2 FALA+QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLG |
| 309 | ADC-2 IgG4_S228P HC 2 DHS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHSHYTQKSLSLSLGK |
| 310 | ADC-2 IgG4_S228P HC 2 DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEFLGGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHSHYTQKSLSLSLG |
| 311 | ADC-2 IgG4_S228P HC 2 STR+DHS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHSHYTQKSLSLSLGK |
| 312 | ADC-2 IgG4_S228P HC 2 STR+DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PESTRGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHSHYTQKSLSLSLG |
| 313 | ADC-2 IgG4_S228P HC 2 FALA+DHS | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA |
| | constant region with terminal lysine | PEAAGGPSVFLFPPKPKDTLMISRTPEVTCVVVDVSQ EDPEVQFNWYVDGVEVHNAKTKPREEQFNSTYRVV SVLTVDHHDWLNGKEYKCKVSNKGLPSSIEKTISKA KGQPREPQVYTLPPSQEEMTKNQVSLSCAVKGFYPS DIAVEWESNGQPENNYKTTPPVLDSDGSFFLVSRLTV DKSRWQEGNVFSCSVMHEALHSHYTQKSLSLSLGK |
| 314 | ADC-2 IgG4_S228P HC 2 FALA+DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPPCPA PEAAGGPSVFLFPPKPKDTLMISRTPEVTCVVVDVSQ EDPEVQFNWYVDGVEVHNAKTKPREEQFNSTYRVV SVLTVDHHDWLNGKEYKCKVSNKGLPSSIEKTISKA KGQPREPQVYTLPPSQEEMTKNQVSLSCAVKGFYPS DIAVEWESNGQPENNYKTTPPVLDSDGSFFLVSRLTV DKSRWQEGNVFSCSVMHEALHSHYTQKSLSLSLG |
| 315 | ADC-2 IgG4_HC 2 constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 316 | ADC-2 IgG4_HC 2 constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 317 | ADC-2 IgG4_HC 2 STR constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 318 | ADC-2 IgG4_HC 2 STR constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK |
| | | GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 319 | ADC-2 IgG4_HC 2 FALA constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 320 | ADC-2 IgG4_HC 2 FALA constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 321 | ADC-2 IgG4_HC 2 YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 322 | ADC-2 IgG4_HC 2 YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 323 | ADC-2 IgG4_HC 2 STR+YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 324 | ADC-2 IgG4_HC 2 STR+YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLYITREPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 325 | ADC-2 IgG4_HC 2 FALA+YTE constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLYITREPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLGK |
| 326 | ADC-2 IgG4_HC 2 FALA+YTE constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLYITREPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHNHYTQKSLSLSLG |
| 327 | ADC-2 IgG4_HC 2 REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLGK |
| 328 | ADC-2 IgG4_HC 2 REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLG |
| 329 | ADC-2 IgG4_HC 2 STR+REW constant region | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA |
| | with terminal lysine | PESTRGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLGK |
| 330 | ADC-2 IgG4_HC 2 STR+REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLG |
| 331 | ADC-2 IgG4_HC 2 FALA+REW constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLGK |
| 332 | ADC-2 IgG4_HC 2 FALA+REW constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHRDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVEHEALHWHYTQKSLSLSLG |
| 333 | ADC-2 IgG4_HC 2 LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLGK |
| 334 | ADC-2 IgG4_HC 2 LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK |
| | | GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLG |
| 335 | ADC-2 IgG4_HC 2 STR+LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLGK |
| 336 | ADC-2 IgG4_HC 2 STR+LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLG |
| 337 | ADC-2 IgG4_HC 2 FALA+LS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLGK |
| 338 | ADC-2 IgG4_HC 2 FALA+LS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHSHYTQKSLSLSLG |
| 339 | ADC-2 IgG4_HC 2 QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLGK |
| 340 | ADC-2 IgG4_HC 2 QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLG |
| 341 | ADC-2 IgG4_HC 2 STR+QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDQLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLGK |
| 342 | ADC-2 IgG4_HC 2 STR+QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDQLMISRTPEVTCVVVDVSQE DPEVQFNWYVDGVEVHNAKTKPREEQFNSTYRVVS VLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLG |
| 343 | ADC-2 IgG4_HC 2 FALA+QL constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLGK |
| 344 | ADC-2 IgG4_HC 2 FALA+QL constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDQLMISRTPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVLHEALHNHYTQKSLSLSLG |
| 345 | ADC-2 IgG4_HC 2 DHS constant | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA |
| | region with terminal lysine | PEFLGGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHSHYTQKSLSLSLGK |
| 346 | ADC-2 IgG4_HC 2 DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEFLGGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHSHYTQKSLSLSLG |
| 347 | ADC-2 IgG4_HC 2 STR+DHS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHSHYTQKSLSLSLGK |
| 348 | ADC-2 IgG4_HC 2 STR+DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PESTRGPSVFLFPPKPKDTLMISRTPEVTCVVVDVSQE DPEVQFNWYVDGVEVHNAKTKPREEQFNSTYRVVS VLTVDHHDWLNGKEYKCKVSNKGLPSSIEKTISKAK GQPREPQVYTLPPSQEEMTKNQVSLSCAVKGFYPSDI AVEWESNGQPENNYKTTPPVLDSDGSFFLVSRLTVD KSRWQEGNVFSCSVMHEALHSHYTQKSLSLSLG |
| 349 | ADC-2 IgG4_HC 2 FALA+DHS constant region with terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVDHHDWLNGKEYKCKVSNKGLPSSIEKTISKA KGQPREPQVYTLPPSQEEMTKNQVSLSCAVKGFYPS DIAVEWESNGQPENNYKTTPPVLDSDGSFFLVSRLTV DKSRWQEGNVFSCSVMHEALHSHYTQKSLSLSLGK |
| 350 | ADC-2 IgG4_HC 2 FALA+DHS constant region without terminal lysine | ASTKGPSVFPLAPCSRSTSESTAALGCLVKDYFPEPVT VSWNSGALTSGVHTFPAVLQSSGLYSLSSVVTVPSSS LGTKTYTCNVDHKPSNTKVDKRVESKYGPPCPSCPA PEAAGGPSVFLFPPKPKDTLMISRTPEVTCVVVDVSQ EDPEVQFNWYVDGVEVHNAKTKPREEQFNSTYRVV SVLTVDHHDWLNGKEYKCKVSNKGLPSSIEKTISKA |
| | | KGQPREPQVYTLPPSQEEMTKNQVSLSCAVKGFYPS DIAVEWESNGQPENNYKTTPPVLDSDGSFFLVSRLTV DKSRWQEGNVFSCSVMHEALHSHYTQKSLSLSLG |

In some embodiments, the anti-FRα bivalent biparatopic antibody of ADC-2 comprises a heavy chain 2 (HC 2 or second polypeptide) comprising (i) the VH of SEQ ID NO: 4 and (ii) an IgG1 heavy chain constant region comprising an amino acid sequence shown as any one of SEQ ID NOs: 243 - 278. In some embodiments, the anti-FRα bivalent biparatopic antibody heavy chain 2 (HC 2) may comprise a wild type IgG1 heavy chain constant region comprising amino acid sequences according to SEQ ID NO 243 (with terminal lysine) and SEQ ID NO 244 (without terminal lysine). In some embodiments, the IgG1 heavy chain 2 constant region comprises Fc silencing mutations selected from L234A, L235A (LALA) or L234S, L235T, G236R (STR). In some embodiments, the anti-FRα bivalent biparatopic antibody heavy chain 2 (HC 2) comprises an IgG1 heavy chain constant region comprising the mutations L234A, L235A (LALA) with or without terminal lysine. In some embodiments, the anti-FRα bivalent biparatopic antibody heavy chain 2 (HC 2) comprises an IgG1 heavy chain constant region comprising the mutations L234S, L235T, G236R (STR) with or without terminal lysine. In some embodiments, the anti-FRα bivalent biparatopic antibody heavy chain 2 (HC 2) may comprise a heavy chain constant region IgG1 comprising half-life extension mutations selected from M252Y, S254T, T256E (YTE); Q311R, M428E, N434W (REW); M428L, N434S (LS); L309D, Q311H, N434S (DHS); or T250Q, M428L (QL). In some embodiments, the anti-FRα bivalent biparatopic antibody heavy chain 2 (HC 2) comprises an IgG1 heavy chain constant region comprising M252Y, S254T, T256E (YTE) with or without terminal lysine. In some embodiments, the anti-FRα bivalent biparatopic antibody heavy chain 2 (HC 2) comprises an IgG1 heavy chain constant region comprising Q311R, M428E, N434W (REW) with or without terminal lysine. In some embodiments, the anti-FRα bivalent biparatopic antibody heavy chain 2 (HC 2) comprises an IgG1 heavy chain constant region comprising M428L, N434S (LS) with or without terminal lysine. In some embodiments, the anti-FRα bivalent biparatopic antibody heavy chain 2 (HC 2) comprises an IgG1 heavy chain constant region comprising L309D, Q311H, N434S (DHS) with or without terminal lysine. In some embodiments, the anti-FRα bivalent biparatopic antibody heavy chain 2 (HC 2) comprises an IgG1 heavy chain constant region comprising T250Q, M428L (QL) with or without terminal lysine. In some embodiments, the anti-FRα bivalent biparatopic antibody heavy chain 2 (HC 2) constant region may comprise both Fc silencing mutations and half-life extension mutations. In some embodiments, the anti-FRα bivalent biparatopic antibody heavy chain 2 (HC 2) comprises an IgG1 heavy chain constant region comprising the mutations L234A, L235A (LALA) and M252Y, S254T, T256E (YTE) with or without terminal lysine. In some embodiments, the anti-FRα bivalent biparatopic antibody heavy chain 2 (HC 2) comprises an IgG1 heavy chain constant region comprising the mutations L234S, L235T, G236R (STR) and M252Y, S254T, T256E (YTE) with or without terminal lysine. In some embodiments, the anti-FRα bivalent biparatopic antibody heavy chain 2 (HC 2) comprises an IgG1 heavy chain constant region comprising the mutations L234A, L235A (LALA) and Q311R, M428E, N434W (REW) with or without terminal lysine. In some embodiments, the anti-FRα bivalent biparatopic antibody heavy chain 2 (HC 2) comprises an IgG1 heavy chain constant region comprising the mutations L234S, L235T, G236R (STR) and Q311R, M428E, N434W (REW) with or without terminal lysine. In some embodiments, the anti-FRα bivalent biparatopic antibody heavy chain 2 (HC 2) comprises an IgG1 heavy chain constant region comprising the mutations L234A, L235A (LALA) and M428L, N434S (LS) with or without terminal lysine. In some embodiments, the anti-FRα bivalent biparatopic antibody heavy chain 2 (HC 2) comprises an IgG1 heavy chain constant region comprising the mutations L234S, L235T, G236R (STR) and M428L, N434S (LS) with or without terminal lysine. In some embodiments, the anti-FRα bivalent biparatopic antibody heavy chain 2 (HC 2) comprises an IgG1 heavy chain constant region comprising the mutations L234A, L235A (LALA) and L309D, Q311H, N434S (DHS) with or without terminal lysine. In some embodiments, the anti-FRα bivalent biparatopic antibody heavy chain 2 (HC 2) comprises an IgG1 heavy chain constant region comprising the mutations L234S, L235T, G236R (STR) and L309D, Q311H, N434S (DHS) with or without terminal lysine. In some embodiments, the anti-FRα bivalent biparatopic antibody heavy chain 2 (HC 2) comprises an IgG1 heavy chain constant region comprising the mutations L234A, L235A (LALA) and T250Q, M428L (QL) with or without terminal lysine. In some embodiments, the anti-FRα bivalent biparatopic antibody heavy chain 2 (HC 2) comprises an IgG1 heavy chain constant region comprising the mutations L234S, L235T, G236R (STR) and T250Q, M428L (QL) with or without terminal lysine. In some embodiments, the position of the mutations is according to EU numbering.

In some embodiments, the anti-FRα bivalent biparatopic antibody of ADC-2 comprises a heavy chain 2 (HC 2 or second polypeptide) comprising (i) the VH of SEQ ID NO: 4 and (ii) an IgG4 heavy chain constant region comprising an amino acid sequence shown as any one of SEQ ID NOs: 279 - 350. In some embodiments, the anti-FRα bivalent biparatopic antibody heavy chain 2 (HC 2) may comprise a wild type IgG4 heavy chain constant region comprising amino acid sequences according to SEQ ID NO 315 (with terminal lysine) and SEQ ID NO 316 (without terminal lysine). In some embodiments, the anti-FRα bivalent biparatopic antibody heavy chain 2 (HC 2) may comprise the IgG4 heavy chain constant region further comprising an S228P mutation according to SEQ ID NO 279 (with terminal lysine) and SEQ ID NO 280 (without terminal lysine). In some embodiments, the IgG4 heavy chain constant region comprises Fc silencing mutations selected from F234A, L235A (FALA) or L234S, L235T, G236R (STR). In some embodiments, the anti-FRα bivalent biparatopic antibody heavy chain 2 (HC 2) comprises an IgG4 heavy chain constant region comprising the mutations F234A, L235A (FALA) with or without terminal lysine and with or without an S228P mutation. In some embodiments, the anti-FRα bivalent biparatopic antibody heavy chain 2 (HC 2) comprises an IgG4 heavy chain constant region comprising the mutations L234S, L235T, G236R (STR) with or without terminal lysine and with or without an S228P mutation. In some embodiments, the anti-FRα bivalent biparatopic antibody heavy chain 2 (HC 2) may comprise an IgG4 heavy chain constant region comprising half-life extension mutations selected from M252Y, S254T, T256E (YTE); Q311R, M428E, N434W (REW); M428L, N434S (LS); L309D, Q311H, N434S (DHS); or T250Q, M428L (QL). In some embodiments, the anti-FRα bivalent biparatopic antibody heavy chain 2 (HC 2) comprises an IgG4 heavy chain constant region comprising M252Y, S254T, T256E (YTE) with or without terminal lysine and with or without an S228P mutation. In some embodiments, the anti-FRα bivalent biparatopic antibody heavy chain 2 (HC 2) comprises an IgG4 heavy chain constant region comprising Q311R, M428E, N434W (REW) with or without terminal lysine and with or without an S228P mutation. In some embodiments, the anti-FRα bivalent biparatopic antibody heavy chain 2 (HC 2) comprises an IgG4 heavy chain constant region comprising M428L, N434S (LS) with or without terminal lysine and with or without an S228P mutation. In some embodiments, the anti-FRα bivalent biparatopic antibody heavy chain 2 (HC 2) comprises an IgG4 heavy chain constant region comprising L309D, Q311H, N434S (DHS) with or without terminal lysine and with or without an S228P mutation. In some embodiments, the anti-FRα bivalent biparatopic antibody heavy chain 2 (HC 2) comprises an IgG4 heavy chain constant region comprising T250Q, M428L (QL) with or without terminal lysine and with or without an S228P mutation. In some embodiments, the anti-FRα bivalent biparatopic antibody heavy chain 2 (HC 2) constant region may comprise both Fc silencing mutations and half-life extension mutations. In some embodiments, the anti-FRα bivalent biparatopic antibody heavy chain 2 (HC 2) comprises an IgG4 heavy chain constant region comprising the mutations F234A, L235A (FALA) and M252Y, S254T, T256E (YTE) with or without terminal lysine and with or without an S228P mutation. In some embodiments, the anti-FRα bivalent biparatopic antibody heavy chain 2 (HC 2) comprises an IgG4 heavy chain constant region comprising the mutations L234S, L235T, G236R (STR) and M252Y, S254T, T256E (YTE) with or without terminal lysine and with or without an S228P mutation. In some embodiments, the anti-FRα bivalent biparatopic antibody heavy chain 2 (HC 2) comprises an IgG4 heavy chain constant region comprising the mutations F234A, L235A (FALA) and Q311R, M428E, N434W (REW) with or without terminal lysine and with or without an S228P mutation. In some embodiments, the anti-FRα bivalent biparatopic antibody heavy chain 2 (HC 2) comprises an IgG4 heavy chain constant region comprising the mutations L234S, L235T, G236R (STR) and Q311R, M428E, N434W (REW) with or without terminal lysine and with or without an S228P mutation. In some embodiments, the anti-FRα bivalent biparatopic antibody heavy chain 2 (HC 2) comprises an IgG4 heavy chain constant region comprising the mutations F234A, L235A (FALA) and M428L, N434S (LS) with or without terminal lysine and with or without an S228P mutation. In some embodiments, the anti-FRα bivalent biparatopic antibody heavy chain 2 (HC 2) comprises an IgG4 heavy chain constant region comprising the mutations L234S, L235T, G236R (STR) and M428L, N434S (LS) with or without terminal lysine and with or without an S228P mutation. In some embodiments, the anti-FRα bivalent biparatopic antibody heavy chain 2 (HC 2) comprises an IgG4 heavy chain constant region comprising the mutations F234A, L235A (FALA) and L309D, Q311H, N434S (DHS) with or without terminal lysine and with or without an S228P mutation. In some embodiments, the anti-FRα bivalent biparatopic antibody heavy chain 2 (HC 2) comprises an IgG4 heavy chain constant region comprising the mutations L234S, L235T, G236R (STR) and L309D, Q311H, N434S (DHS) with or without terminal lysine and with or without an S228P mutation. In some embodiments, the anti-FRα bivalent biparatopic antibody heavy chain 2 (HC 2) comprises an IgG4 heavy chain constant region comprising the mutations F234A, L235A (FALA) and T250Q, M428L (QL) with or without terminal lysine and with or without an S228P mutation. In some embodiments, the anti-FRα bivalent biparatopic antibody heavy chain 2 (HC 2) comprises an IgG4 heavy chain constant region comprising the mutations L234S, L235T, G236R (STR) and T250Q, M428L (QL) with or without terminal lysine and with or without an S228P mutation. In some embodiments, the position of the mutations is according to EU numbering.

In some embodiments, the ADC-2 anti-FRα bivalent biparatopic antibody comprises (i) a first polypeptide (heavy chain 1 or HC 1) comprising the scFv-hinge polypeptide of SEQ ID NO: 351 and an Fc region, (ii) a second polypeptide (heavy chain 2 or HC 2) comprising the VH of SEQ ID NO: 4 and an heavy chain constant region, and (iii) a light chain of SEQ ID NO: 11, wherein the Fc region comprises an amino acid sequence shown as any one of SEQ ID NO: 135 - 242 and the heavy chain constant region comprises an amino acid sequence shown as any one of SEQ ID NO: 243-350. In some embodiments, the Fc region and the heavy chain constant region comprise the amino acid sequences shown as SEQ ID NO: 135 and 243, respectively. In some embodiments, the Fc region and the heavy chain constant region comprise the amino acid sequences shown as SEQ ID NO: 136 and 244, respectively. In some embodiments, the Fc region and the heavy chain constant region comprise the amino acid sequences shown as SEQ ID NO: 137 and 245, respectively. In some embodiments, the Fc region and the heavy chain constant region comprise the amino acid sequences shown as SEQ ID NO: 138 and 246, respectively. In some embodiments, the Fc region and the heavy chain constant region comprise the amino acid sequences shown as SEQ ID NO: 139 and 247, respectively. In some embodiments, the Fc region and the heavy chain constant region comprise the amino acid sequences shown as SEQ ID NO: 140 and 248, respectively. In some embodiments, the Fc region and the heavy chain constant region comprise the amino acid sequences shown as SEQ ID NO: 141 and 249, respectively. In some embodiments, the Fc region and the heavy chain constant region comprise the amino acid sequences shown as SEQ ID NO: 142 and 250, respectively. In some embodiments, the Fc region and the heavy chain constant region comprise the amino acid sequences shown as SEQ ID NO: 143 and 251, respectively. In some embodiments, the Fc region and the heavy chain constant region comprise the amino acid sequences shown as SEQ ID NO: 144 and 252, respectively. In some embodiments, the Fc region and the heavy chain constant region comprise the amino acid sequences shown as SEQ ID NO: 145 and 253, respectively. In some embodiments, the Fc region and the heavy chain constant region comprise the amino acid sequences shown as SEQ ID NO: 146 and 254, respectively. In some embodiments, the Fc region and the heavy chain constant region comprise the amino acid sequences shown as SEQ ID NO: 147 and 255, respectively. In some embodiments, the Fc region and the heavy chain constant region comprise the amino acid sequences shown as SEQ ID NO: 148 and 256, respectively. In some embodiments, the Fc region and the heavy chain constant region comprise the amino acid sequences shown as SEQ ID NO: 149 and 257, respectively. In some embodiments, the Fc region and the heavy chain constant region comprise the amino acid sequences shown as SEQ ID NO: 150 and 258, respectively. In some embodiments, the Fc region and the heavy chain constant region comprise the amino acid sequences shown as SEQ ID NO: 151 and 259, respectively. In some embodiments, the Fc region and the heavy chain constant region comprise the amino acid sequences shown as SEQ ID NO: 152 and 260, respectively. In some embodiments, the Fc region and the heavy chain constant region comprise the amino acid sequences shown as SEQ ID NO: 153 and 261, respectively. In some embodiments, the Fc region and the heavy chain constant region comprise the amino acid sequences shown as SEQ ID NO: 154 and 262, respectively. In some embodiments, the Fc region and the heavy chain constant region comprise the amino acid sequences shown as SEQ ID NO: 155 and 263, respectively. In some embodiments, the Fc region and the heavy chain constant region comprise the amino acid sequences shown as SEQ ID NO: 156 and 264, respectively. In some embodiments, the Fc region and the heavy chain constant region comprise the amino acid sequences shown as SEQ ID NO: 157 and 265, respectively. In some embodiments, the Fc region and the heavy chain constant region comprise the amino acid sequences shown as SEQ ID NO: 158 and 266, respectively. In some embodiments, the Fc region and the heavy chain constant region comprise the amino acid sequences shown as SEQ ID NO: 159 and 267, respectively. In some embodiments, the Fc region and the heavy chain constant region comprise the amino acid sequences shown as SEQ ID NO: 160 and 268, respectively. In some embodiments, the Fc region and the heavy chain constant region comprise the amino acid sequences shown as SEQ ID NO: 161 and 269, respectively. In some embodiments, the Fc region and the heavy chain constant region comprise the amino acid sequences shown as SEQ ID NO: 162 and 270, respectively. In some embodiments, the Fc region and the heavy chain constant region comprise the amino acid sequences shown as SEQ ID NO: 163 and 271, respectively. In some embodiments, the Fc region and the heavy chain constant region comprise the amino acid sequences shown as SEQ ID NO: 164 and 272, respectively. In some embodiments, the Fc region and the heavy chain constant region comprise the amino acid sequences shown as SEQ ID NO: 165 and 273, respectively. In some embodiments, the Fc region and the heavy chain constant region comprise the amino acid sequences shown as SEQ ID NO: 166 and 274, respectively. In some embodiments, the Fc region and the heavy chain constant region comprise the amino acid sequences shown as SEQ ID NO: 167 and 275, respectively. In some embodiments, the Fc region and the heavy chain constant region comprise the amino acid sequences shown as SEQ ID NO: 168 and 276, respectively. In some embodiments, the Fc region and the heavy chain constant region comprise the amino acid sequences shown as SEQ ID NO: 169 and 277, respectively. In some embodiments, the Fc region and the heavy chain constant region comprise the amino acid sequences shown as SEQ ID NO: 170 and 278, respectively. In some embodiments, the Fc region and the heavy chain constant region comprise the amino acid sequences shown as SEQ ID NO: 171 and 279, respectively. In some embodiments, the Fc region and the heavy chain constant region comprise the amino acid sequences shown as SEQ ID NO: 172 and 280, respectively. In some embodiments, the Fc region and the heavy chain constant region comprise the amino acid sequences shown as SEQ ID NO: 173 and 281, respectively. In some embodiments, the Fc region and the heavy chain constant region comprise the amino acid sequences shown as SEQ ID NO: 174 and 282, respectively. In some embodiments, the Fc region and the heavy chain constant region comprise the amino acid sequences shown as SEQ ID NO: 175 and 283, respectively. In some embodiments, the Fc region and the heavy chain constant region comprise the amino acid sequences shown as SEQ ID NO: 176 and 284, respectively. In some embodiments, the Fc region and the heavy chain constant region comprise the amino acid sequences shown as SEQ ID NO: 177 and 285, respectively. In some embodiments, the Fc region and the heavy chain constant region comprise the amino acid sequences shown as SEQ ID NO: 178 and 286, respectively. In some embodiments, the Fc region and the heavy chain constant region comprise the amino acid sequences shown as SEQ ID NO: 179 and 287, respectively. In some embodiments, the Fc region and the heavy chain constant region comprise the amino acid sequences shown as SEQ ID NO: 180 and 288, respectively. In some embodiments, the Fc region and the heavy chain constant region comprise the amino acid sequences shown as SEQ ID NO: 181 and 289, respectively. In some embodiments, the Fc region and the heavy chain constant region comprise the amino acid sequences shown as SEQ ID NO: 182 and 290, respectively. In some embodiments, the Fc region and the heavy chain constant region comprise the amino acid sequences shown as SEQ ID NO: 183 and 291, respectively. In some embodiments, the Fc region and the heavy chain constant region comprise the amino acid sequences shown as SEQ ID NO: 184 and 292, respectively. In some embodiments, the Fc region and the heavy chain constant region comprise the amino acid sequences shown as SEQ ID NO: 185 and 293, respectively. In some embodiments, the Fc region and the heavy chain constant region comprise the amino acid sequences shown as SEQ ID NO: 186 and 294, respectively. In some embodiments, the Fc region and the heavy chain constant region comprise the amino acid sequences shown as SEQ ID NO: 187 and 295, respectively. In some embodiments, the Fc region and the heavy chain constant region comprise the amino acid sequences shown as SEQ ID NO: 188 and 296, respectively. In some embodiments, the Fc region and the heavy chain constant region comprise the amino acid sequences shown as SEQ ID NO: 189 and 297, respectively. In some embodiments, the Fc region and the heavy chain constant region comprise the amino acid sequences shown as SEQ ID NO: 190 and 298, respectively. In some embodiments, the Fc region and the heavy chain constant region comprise the amino acid sequences shown as SEQ ID NO: 191 and 299, respectively. In some embodiments, the Fc region and the heavy chain constant region comprise the amino acid sequences shown as SEQ ID NO: 192 and 300, respectively. In some embodiments, the Fc region and the heavy chain constant region comprise the amino acid sequences shown as SEQ ID NO: 193 and 301, respectively. In some embodiments, the Fc region and the heavy chain constant region comprise the amino acid sequences shown as SEQ ID NO: 194 and 302, respectively. In some embodiments, the Fc region and the heavy chain constant region comprise the amino acid sequences shown as SEQ ID NO: 195 and 303, respectively. In some embodiments, the Fc region and the heavy chain constant region comprise the amino acid sequences shown as SEQ ID NO: 196 and 304, respectively. In some embodiments, the Fc region and the heavy chain constant region comprise the amino acid sequences shown as SEQ ID NO: 197 and 305, respectively. In some embodiments, the Fc region and the heavy chain constant region comprise the amino acid sequences shown as SEQ ID NO: 198 and 306, respectively. In some embodiments, the Fc region and the heavy chain constant region comprise the amino acid sequences shown as SEQ ID NO: 199 and 307, respectively. In some embodiments, the Fc region and the heavy chain constant region comprise the amino acid sequences shown as SEQ ID NO: 200 and 308, respectively. In some embodiments, the Fc region and the heavy chain constant region comprise the amino acid sequences shown as SEQ ID NO: 201 and 309, respectively. In some embodiments, the Fc region and the heavy chain constant region comprise the amino acid sequences shown as SEQ ID NO: 202 and 310, respectively. In some embodiments, the Fc region and the heavy chain constant region comprise the amino acid sequences shown as SEQ ID NO: 203 and 311, respectively. In some embodiments, the Fc region and the heavy chain constant region comprise the amino acid sequences shown as SEQ ID NO: 204 and 312, respectively. In some embodiments, the Fc region and the heavy chain constant region comprise the amino acid sequences shown as SEQ ID NO: 205 and 313, respectively. In some embodiments, the Fc region and the heavy chain constant region comprise the amino acid sequences shown as SEQ ID NO: 206 and 314, respectively. In some embodiments, the Fc region and the heavy chain constant region comprise the amino acid sequences shown as SEQ ID NO: 207 and 315, respectively. In some embodiments, the Fc region and the heavy chain constant region comprise the amino acid sequences shown as SEQ ID NO: 208 and 316, respectively. In some embodiments, the Fc region and the heavy chain constant region comprise the amino acid sequences shown as SEQ ID NO: 209 and 317, respectively. In some embodiments, the Fc region and the heavy chain constant region comprise the amino acid sequences shown as SEQ ID NO: 210 and 318, respectively. In some embodiments, the Fc region and the heavy chain constant region comprise the amino acid sequences shown as SEQ ID NO: 211 and 319, respectively. In some embodiments, the Fc region and the heavy chain constant region comprise the amino acid sequences shown as SEQ ID NO: 212 and 320, respectively. In some embodiments, the Fc region and the heavy chain constant region comprise the amino acid sequences shown as SEQ ID NO: 213 and 321, respectively. In some embodiments, the Fc region and the heavy chain constant region comprise the amino acid sequences shown as SEQ ID NO: 214 and 322, respectively. In some embodiments, the Fc region and the heavy chain constant region comprise the amino acid sequences shown as SEQ ID NO: 215 and 323, respectively. In some embodiments, the Fc region and the heavy chain constant region comprise the amino acid sequences shown as SEQ ID NO: 216 and 324, respectively. In some embodiments, the Fc region and the heavy chain constant region comprise the amino acid sequences shown as SEQ ID NO: 217 and 325, respectively. In some embodiments, the Fc region and the heavy chain constant region comprise the amino acid sequences shown as SEQ ID NO: 218 and 326, respectively. In some embodiments, the Fc region and the heavy chain constant region comprise the amino acid sequences shown as SEQ ID NO: 219 and 327, respectively. In some embodiments, the Fc region and the heavy chain constant region comprise the amino acid sequences shown as SEQ ID NO: 220 and 328, respectively. In some embodiments, the Fc region and the heavy chain constant region comprise the amino acid sequences shown as SEQ ID NO: 221 and 329, respectively. In some embodiments, the Fc region and the heavy chain constant region comprise the amino acid sequences shown as SEQ ID NO: 222 and 330, respectively. In some embodiments, the Fc region and the heavy chain constant region comprise the amino acid sequences shown as SEQ ID NO: 223 and 331, respectively. In some embodiments, the Fc region and the heavy chain constant region comprise the amino acid sequences shown as SEQ ID NO: 224 and 332, respectively. In some embodiments, the Fc region and the heavy chain constant region comprise the amino acid sequences shown as SEQ ID NO: 225 and 333, respectively. In some embodiments, the Fc region and the heavy chain constant region comprise the amino acid sequences shown as SEQ ID NO: 226 and 334, respectively. In some embodiments, the Fc region and the heavy chain constant region comprise the amino acid sequences shown as SEQ ID NO: 227 and 335, respectively. In some embodiments, the Fc region and the heavy chain constant region comprise the amino acid sequences shown as SEQ ID NO: 228 and 336, respectively. In some embodiments, the Fc region and the heavy chain constant region comprise the amino acid sequences shown as SEQ ID NO: 229 and 337, respectively. In some embodiments, the Fc region and the heavy chain constant region comprise the amino acid sequences shown as SEQ ID NO: 230 and 338, respectively. In some embodiments, the Fc region and the heavy chain constant region comprise the amino acid sequences shown as SEQ ID NO: 231 and 339, respectively. In some embodiments, the Fc region and the heavy chain constant region comprise the amino acid sequences shown as SEQ ID NO: 232 and 340, respectively. In some embodiments, the Fc region and the heavy chain constant region comprise the amino acid sequences shown as SEQ ID NO: 233 and 341, respectively. In some embodiments, the Fc region and the heavy chain constant region comprise the amino acid sequences shown as SEQ ID NO: 234 and 342, respectively. In some embodiments, the Fc region and the heavy chain constant region comprise the amino acid sequences shown as SEQ ID NO: 235 and 343, respectively. In some embodiments, the Fc region and the heavy chain constant region comprise the amino acid sequences shown as SEQ ID NO: 236 and 344, respectively. In some embodiments, the Fc region and the heavy chain constant region comprise the amino acid sequences shown as SEQ ID NO: 237 and 345, respectively. In some embodiments, the Fc region and the heavy chain constant region comprise the amino acid sequences shown as SEQ ID NO: 238 and 346, respectively. In some embodiments, the Fc region and the heavy chain constant region comprise the amino acid sequences shown as SEQ ID NO: 239 and 347, respectively. In some embodiments, the Fc region and the heavy chain constant region comprise the amino acid sequences shown as SEQ ID NO: 240 and 348, respectively. In some embodiments, the Fc region and the heavy chain constant region comprise the amino acid sequences shown as SEQ ID NO: 241 and 349, respectively. In some embodiments, the Fc region and the heavy chain constant region comprise the amino acid sequences shown as SEQ ID NO: 242 and 350, respectively.

### TOPOISOMERASE 1 INHIBITORS (TOP1I)

Topoisomerase 1 (TOP1) removes supercoils formed during DNA replication. TOP1 inhibitors (TOP1i) can bind and stabilize TOP1-DNA complexes, inducing DNA strand breakage and apoptosis.

In some embodiments, provided herein is a topoisomerase I inhibitor drug ("TOP1i drug") according to structural formula (I), which may be purposed for targeted delivery to cells by conjugation to an anti-FRα antibody.

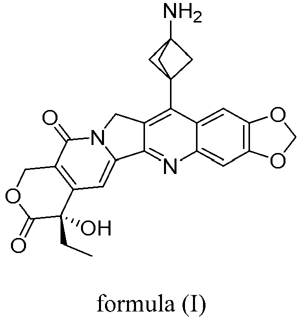

In some embodiments, the TOP1i drug is (7*S*)-14-(3-aminobicyclo[1.1.1]pentan-1-yl)-7-ethyl-7-hydroxy-2*H*,10*H*-[1,3]dioxolo[4,5-*g*]pyrano[3',4':6,7]indolizino[1,2-*b*]quinoline-8,11(7*H*,13*H*)-dione.

In some embodiments, the TOP1i drug as contemplated herein may be conjugated to an antibody via a linker as shown in structural formula (II): 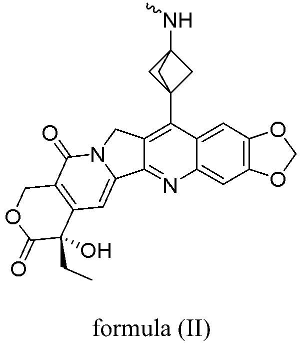 wherein 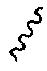 represents the point of attachment of a linker to the TOP1i drug.

### LINKER DRUG LD1 (STRUCTURAL FORMULA (III))

In some embodiments, a TOP1i linker drug (LD1) is a compound according to formula (III). In some embodiments, LD1 is (2*S*)-2-(2-bromoacetamido)-*N*-[(2*S*)-1-({3-[(7*S*)-7-ethyl-7-hydroxy-8,11-dioxo-7,8,11,13-tetrahydro-2*H*,10*H*-[1,3]dioxolo[4,5-*g*]pyrano[3',4':6,7]indolizino[1,2-*b*]quinolin-14-yl]bicyclo[1.1.1]pentan-1-yl}amino)-1-oxopropan-2-yl]-3-methylbutanamide.

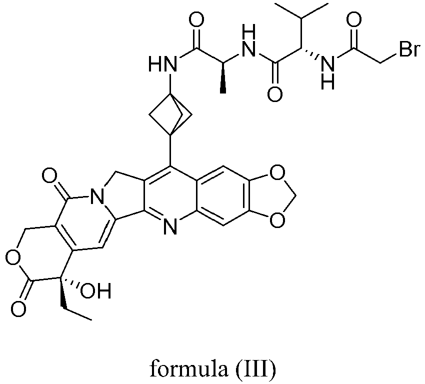

### ANTI-FRα ADCs

In some embodiments, the TOP1i drug as described herein (*i.e*., formula (I)) may be conjugated to an anti-FRα antibody to form an anti-FRα TOP1i antibody drug conjugate. Antibody-drug conjugates selectively deliver one or more drug moiety(s) to target tissues, such as FRα expressing tumors.

In some embodiments, TOP1i drugs are conjugated to the anti-FRα antibody by way of a linker moiety. In some embodiments, the linkers connect the TOP1i drug to the anti-FRα antibody by forming a covalent linkage to the TOP1i drug at one location and a covalent linkage to the antibody at another. In some embodiments, the covalent linkages are formed by reaction between functional groups on the linker and functional groups on the TOP1i drug and the anti-FRα antibody. In some embodiments, the anti-FRα ADC of the present disclosure is comprised of an anti-FRα antibody conjugated to the TOP1i linker drug of formula (III).

In some embodiments, "Ab" or "antibody" refer to an anti-FRα antibody comprising a heavy chain having an amino acid sequence shown as SEQ ID NO: 5 or SEQ ID NO: 6 and a light chain having an amino acid sequence shown as SEQ ID NO: 11. In some embodiments, Ab is conjugated to the TOP1i linker drug of formula (III).

In some embodiments, "Ab" or "antibody" refer to an anti-FRα antibody comprising a light chain having an amino acid sequence shown as SEQ ID NO: 11, a first heavy chain having an amino acid sequence shown as SEQ ID NO: 12, and a second heavy chain having an amino acid sequence shown as SEQ ID NO: 13. In some embodiments, Ab is conjugated to the TOP1i linker drug of formula (III).

### ANTI-FRα ANTIBODIES

In some embodiments, Ab is a humanized anti-FRα IgG1 monoclonal antibody. In some embodiments, Ab comprises variable regions and CDRs (complementary determining regions) identified according to rules developed in the art and/or by aligning sequences against a database of known variable regions. Methods for identifying these regions are described in Kontermann and Dubel, eds., Antibody Engineering, Springer, New York, N.Y., 2001 and Dinarello et al., Current Protocols in Immunology, John Wiley and Sons Inc., Hoboken, N.J., 2000. For example, CDRs may be identified in accordance with one of the schemes provided by Kabat et al. (1991) Sequences of Proteins of Immunological Interest (5th Ed.), U.S. Dept. of Health and Human Services, PHS, NIH, NIH Publication No. 91-3242 (referred to herein as "Kabat"); or in accordance with AbM (Oxford Molecular/MSI Pharmacopia) (referred to herein as "AbM"). AbM can be obtained from the Abysis database at www.bioinf.org.uk/abs (maintained by A.C. Martin in the Department of Biochemistry & Molecular Biology University College London).

In some embodiments, an ADC-1 anti-FRα immunoconjugate comprises a heavy chain having an amino acid sequence shown as SEQ ID NO: 5 or 6 (variable region is **bold** and has an amino acid sequence shown as SEQ ID NO: 4; constant region is *italicized;* CDRs are underlined and have amino acid sequences shown as SEQ ID NOs: 1, 2 and 3 (Kabat system) respectively, in order of appearance):
**QVQLVQSGAEVVKPGASVKISCKASGYTFTGYFMNWVKQSPGQSLEWIGRIHPYDGDTFYNQ KFQGKATLTVDKSSNTAHMELLSLTSEDFAVYYCTRYDGSRAMDYWGQGTTVTVSS***ASTKGP SVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPELLGGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALH NHYTQKSLSLSPGK* (SEQ ID NO: 5 (SEQ ID NO: 6 is identical to SEQ ID NO: 5 except lacking the C-terminal "K" (lysine residue)).
AbM identified CDRs are shown as SEQ ID NO: 23 and SEQ ID NO: 24.

In some embodiments, an ADC-1 anti-FRα immunoconjugate comprises a light chain having an amino acid sequence shown as SEQ ID NO: 11 (variable region is bold and has an amino acid sequence shown as SEQ ID NO: 10; constant region is *italicized;* CDRs are underlined and have amino acid sequences shown as SEQ ID NOs: 7, 8 and 9 (Kabat system) respectively, in order of appearance):

DIVLTQSPLSLAVSLGQPAIISCKASQSVSFAGTSLMHWYHQKPGQQPRLLIYRASNLEAGVPDRFSG
SGSKTDFTLTISPVEAEDAATYYCQQSREYPYTFGGGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC (SEQ ID NO: 11).

In some embodiments, an ADC-1 anti-FRα immunoconjugate comprises two heavy chains each having an amino acid sequence shown as SEQ ID NO: 5 or 6 or SEQ ID NO: 5 and 6 and two light chains each having an amino acid sequence shown as SEQ ID NO: 11.

In some embodiments, an ADC-1 anti-FRα immunoconjugate comprises a CDR-H1 having an amino acid sequence shown as SEQ ID NO: 1; a CDR-H2 having an amino acid sequence shown as SEQ ID NO: 2; a CDR-H3 having an amino acid sequence shown as SEQ ID NO 3; a CDR-L1 having an amino acid sequence shown as SEQ ID NO: 7; a CDR-L2 having an amino acid sequence shown as SEQ ID NO 8; and a CDR-L3 having an amino acid sequence shown as SEQ ID NO 9. The amino acid sequence of the CDRs was identified using the Kabat definition.

In some embodiments, an ADC-2 anti-FRα immunoconjugate comprises a light chain (third polypeptide) having an amino acid sequence shown as SEQ ID NO: 11, a first heavy chain (HC 1 or first polypeptide) having the amino acid sequence shown as SEQ ID NO: 12, and a second heavy chain (HC 2 or second polypeptide) having the amino acid sequence shown as SEQ ID NO: 13.

In some embodiments, an ADC-2 anti-FRα immunoconjugate comprises a light chain having the amino acid sequence shown as SEQ ID NO: 11 (variable regions is **bold** and has an amino acid sequence shown as SEQ ID NO:10; constant region is *italicized;* CDRs are underlined and have amino acid sequences shown as SEQ ID NOs: 7, 8 and 9 respectively, in order of appearance):

DIVLTQSPLSLAVSLGQPAIISCKASQSVSFAGTSLMHWYHQKPGQQPRLLIYRASNLEAGV
PDRFSGSGSKTDFTLTISPVEAEDAATYYCQQSREYPYTFGGGTKLEIKRTVAAPSVFIFPP
SDEQLKSGTASVVCLLNNFYPREAKVQWKVDNALQSGNSQESVTEQDSKDSTYSLSSTLTLS
KADYEKHKVYACEVTHQGLSSPVTKSFNRGEC (SEQ ID NO: 11).

In some embodiments, an ADC-2 anti-FRα immunoconjugate comprises a first heavy chain (HC1 or first polypeptide) comprising an scFv-Fc fusion polypeptide ("Heavy Chain 1: scFv-Fc-knob") and having the amino acid sequence shown as SEQ ID NO: 12 (variable regions are indicated in bold and have amino acid sequences shown as SEQ ID NO: 15 (VL) and SEQ ID NO: 14 (VH) (connected by Gly-Ser linker "GGGGSGGGGSGGGGSGGGGS" (SEQ ID NO: 352)), Fc region is *italicized*, and CDRs are underlined and have amino acid sequences shown as SEQ ID NOs: 16, 17, 18, 19, 20, and 21 respectively, in order of appearance): 
EIVLTQSPATLSVTPGDRVSLSCRASQNINNNLHWYQQKPGQSPRLLIKYVSQSVSGIPDRF
SGSGSGTDFTLSISSVEPEDEGMYFCQQSNSWPHYTFGCGTKLEIKGGGGSGGGGSGGGGSG
GGGSEVQLVQSGGGLVQPGGSRRLSCAASGFTFSSFGMHWVRQAPGKCLEWVAYISSGSSTI
SYADSVKGRFTISRDNSKKTLLLQMTSLRAEDTAMYYCAREAYGSSMEYWGQGTLVTVSSGS
EPKSSDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKA
KGQPREPQVYTLPPSREEMTKNQVSLWCLVKGFYPSDIAVEWESNGQPENNYKTTPPVLDSD
GSFFLYSKLTVDKSRWQQGNVFSCSVMHEALHNHYTQKSLSLSPG (SEQ ID NO: 12)
 AbM identified CDRs are shown as SEQ ID NO: 25 and SEQ ID NO: 26.

In some embodiments, an ADC-2 anti-FRα immunoconjugate comprises a second heavy chain (HC 2 or second polypeptide) having an amino acid sequence shown as SEQ ID NO: 13 (variable region is **bold** and has an amino acid sequence shown as SEQ ID NO: 4; constant region is *italicized*; CDRs are underlined and have amino acid sequences shown as SEQ ID NOs: 1, 2 and 3 (Kabat system) respectively, in order of appearance): 
QVQLVQSGAEVVKPGASVKISCKASGYTFTGYFMNWVKQSPGQSLEWIGRIHPYDGDTFYNQ
KFQGKATLTVDKSSNTAHMELLSLTSEDFAVYYCTRYDGSRAMDYWGQGTTVTVSSASTKGP
SVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPELLGGPSVFLFPPKP
KDTLMISRTPEVTCVVVDVSHEDPEVKFNWYVDGVEVHNAKTKPREEQYNSTYRVVSVLTVL
 
HQDWLNGKEYKCKVSNKALPAPIEKTISKAKGQPREPQVYTLPPSREEMTKNQVSLSCAVKG
FYPSDIAVEWESNGQPENNYKTTPPVLDSDGSFFLVSKLTVDKSRWQQGNVFSCSVMHEALH
NHYTQKSLSLSPG (SEQ ID NO: 13).
 AbM identified CDRs are shown as SEQ ID NO: 23 and SEQ ID NO: 24.

In some embodiments, an ADC-2 anti-FRα immunoconjugate comprises: a first CDR-H1 having an amino acid sequence shown as SEQ ID NO: 19; a first CDR-H2 having an amino acid sequence shown as SEQ ID NO: 20; a first CDR-H3 having an amino acid sequence shown as SEQ ID NO 21; a first CDR-L1 having an amino acid sequence shown as SEQ ID NO: 16; a first CDR-L2 having an amino acid sequence shown as SEQ ID NO 17; and a first CDR-L3 having an amino acid sequence shown as SEQ ID NO 18; a second CDR-H1 having an amino acid sequence shown as SEQ ID NO: 1; a second CDR-H2 having an amino acid sequence shown as SEQ ID NO: 2; a second CDR-H3 having an amino acid sequence shown as SEQ ID NO: 3; a second CDR-L1 having an amino acid sequence shown as SEQ ID NO: 7; a second CDR-L2 having an amino acid sequence shown as SEQ ID NO 8; a second CDR-L3 having an amino acid sequence shown as SEQ ID NO 9,.

### NUMBER OF LINKED DRUGS

In some embodiments, the ADCs described herein comprise drug molecules linked to antibody moieties in various stoichiometric molar ratios depending on the configuration of the antibody and, at least in part, the method used to effect conjugation.

The terms "drug load" or "drug loading" refer to the number of drug molecules per antibody in an individual ADC molecule. The number of TOP1i drugs linked to an anti-FRα ADC may vary and will be limited by the number of available attachments sites on the anti-FRα antibody. As contemplated for the anti-FRα ADCs of the invention, the linker will link a single TOP1i drug to the antibody in an anti-FRα ADC. As long as the anti-FRα ADC does not exhibit unacceptable levels of aggregation under the conditions of use and/or storage, anti-FRα ADCs (*i.e*., formula (IV)) having an n of up to 10 are contemplated. In some embodiments, the anti-FRα ADCs have an n of in the range of from 1-10. In some embodiments, the anti-FRα ADCs have an n selected from 1, 2, 3, 4, 5, 6, 7, 8, 9, or 10. In some embodiments, n is 2, 3, 4, 5 or 6. In some embodiments, n is 2, 4, 6, 8, or 10. In some embodiments, n is 2, 4, or 6. In some embodiments, n is 4. In some embodiments, n is about 4. In some embodiments, the drug loading may comprise 1 drug molecule, 2 drug molecules, 3 drug molecules, 4 drug molecules, 5 drug molecules, 6 drug molecules, 7 drug molecules, 8 drug molecules, 9 drug molecules, or 10 drug molecules.

Provided herein is a conjugation method of producing an anti-FRα ADC composition with the predominant species of ADC having an n of 4, and with the composition having a drug-antibody ratio (DAR) of 4 or about 4. The DAR is the average number of drugs linked to each antibody in the composition. Other methods of conjugation are known in the art and can be utilized to produce ADCs having different numbers of conjugated drugs (with an n of, *e.g*., 2, 3, 4, 5, 6, 7, 8, 9, 10, about 2, about 3, about 4, about 5, about 6, about 7, about 8, about 9, or about 10,) and compositions of different DARs (*e.g*., a DAR of 2, 3, 4, 5, 6, 7, 8, 9, 10, about 2, about 3, about 4, about 5, about 6, about 7, about 8, about 9, or about 10).

### EXEMPLARY ADCS

**In** some embodiments, an ADC-1 anti-FRα antibody drug conjugate of formula (IV) is provided: 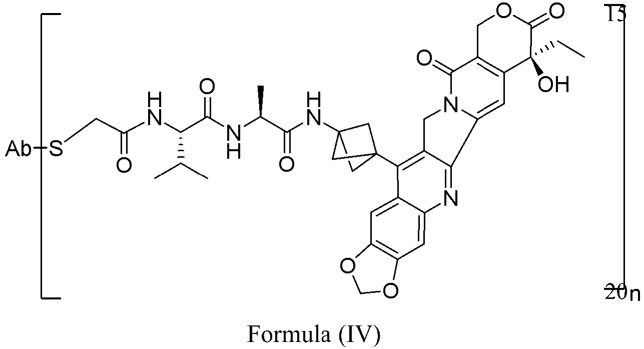 wherein antibody "Ab" comprises two heavy chains each having an amino acid sequence shown as SEQ ID NO: 5 or 6 or SEQ ID NO: 5 and 6 and two light chains each having an amino acid sequence shown as SEQ ID NO: 11. In some embodiments, conjugation of the linker-drug to the antibody is via a linkage formed with a sulfhydryl group of a cysteine residue of the antibody. In some embodiments, n has a value of 4 or about 4.

In some embodiments, "ADC-1" is an ADC composition comprising ADCs according to structural formula (IV), wherein the Ab has two heavy chains each having an amino acid sequence shown as SEQ ID NO: 5 or 6 or SEQ ID NO: 5 and 6 and two light chains each having an amino acid sequence shown as SEQ ID NO: 11 and a drug-antibody ratio (DAR) of about 4, with the predominant species having an n of 4. In an embodiment, ADC-1 is a composition comprising a plurality of ADC-1 species with two or more different values of n. In some embodiments, the composition of ADC-1 has an average drug-antibody ratio (DAR) of about 3.5, about 3.6, about 3.7, about 3.8, about 3.9, about 4.0, about 4.1, about 4.2, about 4.3, about 4.4, or about 4.5.

A procedure used to make ADC-1 is described in the Examples below.

In some embodiments, the composition of ADC-1 has an average of four molecules of the topoisomerase 1 inhibitor (Top1) conjugated per anti-FRα antibody. In some embodiments, the predominant species of ADC-1 in the composition has n = 4. In some embodiments, ADC-1 has an average DAR of about 4.

In some embodiments, an ADC-2 anti-FRα antibody drug conjugate of formula (IV) is provided: 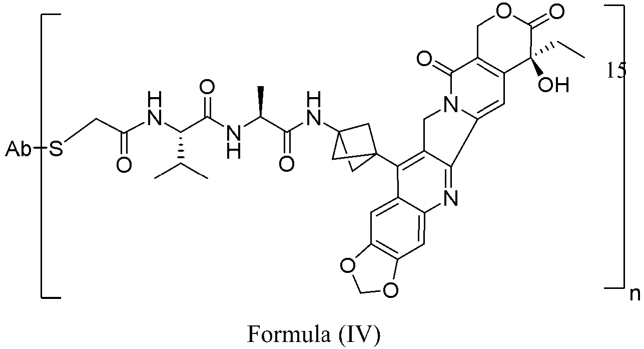 wherein antibody "Ab" comprises two heavy chains and one light chain, wherein a first heavy chain (comprising an scFv FRα antigen binding domain) has an amino acid sequence shown as SEQ ID NO: 12, a second heavy chain has an amino acid sequence shown as SEQ ID NO: 13, and a light chain has an amino acid sequence shown as SEQ ID NO: 11. In some embodiments, conjugation of the linker-drug to the antibody is via a linkage formed with a sulfhydryl group of a cysteine residue of the antibody. In some embodiments, n has a value of 4 or about 4.

In some embodiments, "ADC-2" is an ADC composition comprising ADCs according to structural formula (IV), wherein the Ab has two heavy chains and a light chain, wherein a first heavy chain (comprising an scFv FRα antigen binding domain) has an amino acid sequence shown as SEQ ID NO: 12, a second heavy chain has an amino acid sequence shown as SEQ ID NO: 13, and a light chain has an amino acid sequence shown as SEQ ID NO: 11, and a drug-antibody ratio (DAR) of 4 or about 4, with the predominant species having an n of 4. A procedure used to make ADC-2 is described in the Examples below. In an embodiment, ADC-2 is a composition comprising a plurality of ADC-2 species with two or more different values of n. In some embodiments, the composition of ADC-2 has an average drug-antibody ratio (DAR) of about 3.5, about 3.6, about 3.7, about 3.8, about 3.9, about 4.0, about 4.1, about 4.2, about 4.3, about 4.4, or about 4.5.

In some embodiments, the composition of ADC-2 has an average of four molecules of the topoisomerase 1 inhibitor (Top1) conjugated per anti-FRα antibody. In some embodiments, the predominant species of ADC-2 in the composition has n = 4. In some embodiments, ADC-2 has an average DAR of about 4.

### METHODS OF USE

In some embodiments, methods for treating cancer (e.g., FRα-expressing cancers) such as, gynecological cancers, not limited to, ovarian, peritoneal (primary peritoneal), fallopian tube, endometrial, and cervical cancers comprise administering to a subject in need thereof a therapeutically effective amount of an ADC of formula (IV) are provided. In some embodiments, the ADC is ADC-1 described herein. In some embodiments, the ADC is ADC-2 described herein.

The term "subject," as used herein, refers to a human. The terms "human," "patient," and "subject" are used interchangeably herein.

The terms "treat," "treating," and "treatment," as used herein, refer to a method of alleviating or abrogating a disease and/or its attendant symptoms.

The phrase "therapeutically effective amount" refers to an amount of an ADC sufficient to prevent the development of or to alleviate to some extent one or more of the symptoms of the condition or disorder being treated when administered for treatment in a particular subject or subject population.

### EXAMPLES

### Example 1: Procedure for Conjugation (ADC-1) Mirvetuximab ADC

Synthesis of linker-drug is described in International Publication Number WO 2024/042497 A1 (International Application Number PCT/IB2023/058446, International Filing Date 25-August-2023) and in International Publication Number WO 2022/232834 A1 (International Application Number PCT/US2022/072008, International Filing Date 29-April-2022) each of which are hereby incorporated by reference in their entireties.

Mirvetuximab was conjugated to the linker-drug as described below to form antibody-drug conjugate ADC-1.

The Mirvetuximab antibody was partially reduced with 2.5 equivalents of reducing agent and then conjugated with an excess of linker drug. This resulted in the final product comprising the antibody with a distribution of 0-8 linker-drugs conjugated to cysteine residues with an average drug-to-antibody ratio (DAR) of 4.

A solution of Mirvetuximab antibody, 10 mg/mL in phosphate-buffered sodium buffer at 4 °C, was treated with ethylenediaminetetraacetic acid to a final concentration of 5 mM. Next, 2.5 molar equivalents of tris (2-carboxyethyl) phosphine (TCEP, 10 mM, Bond-Breaker^{®}, Thermo Scientific^{®}) was added to the antibody solution, which was mixed by gentle inversion, and incubated at 4 °C for 18-24 hours.

Before the conjugation reaction, the antibody solution was brought to ambient temperature and the pH was adjusted to 8.0-8.4 by addition of 1 M borate solution, pH 8.0, to a final concentration of 100 mM borate. Linker-drug solution (8.0 molar equivalents, 10 mM in N,N-dimethylacetamide (DMA)) was added to the antibody solution and the mixture was combined by gentle inversion. The reaction was allowed to proceed at room temperature for 1 hour.

Ultrafiltration Diafiltration (UF/DF) was applied to remove residual small molecules from the resulting antibody-drug conjugate, and to buffer exchange the antibody-drug conjugate. Using a Pellicon^{®} 3 membrane (0.11m²) for Tangential Flow Filtration (TFF), the antibody-drug conjugate was buffer exchanged with 10 diavolumes of 7% DMA in DPBS (Dulbecco's phosphate-buffered saline)), 10 diavolumes DPBS, and finally 20 diavolumes of 15 mM histidine, 7% sucrose, pH 6.0 buffer. The final ADC solution was sterile-filtered with a 0.22 µm filter before being stored at -80 °C.

### Example 2: Procedure for Conjugation (ADC-2) Bivalent Biparatopic ADC

The bivalent biparatopic antibody was conjugated to the linker-drug as described below to form antibody-drug conjugate ADC-2.

The bivalent biparatopic antibody was partially reduced with 2.9 equivalents of reducing agent and then conjugated with an excess of linker drug. This resulted in the final product comprising the antibody with a distribution of 0-8 linker-drugs conjugated to cysteine residues with an average drug-to-antibody ratio (DAR) of 4.

A solution of the bivalent biparatopic antibody, 10 mg/mL in phosphate-buffered sodium buffer at 4 °C, was treated with ethylenediaminetetraacetic acid to a final concentration of 5 mM. Next, 2.9 molar equivalents of tris (2-carboxyethyl) phosphine (TCEP, 10 mM, Bond-Breaker^{®}, Thermo Scientific^{®}) was added to the antibody solution, which was mixed by gentle inversion, and incubated at 4 °C for 18-24 hours.

Before the conjugation reaction, the antibody solution was brought to ambient temperature and the pH was adjusted to 8.0-8.4 by addition of 1 M borate solution, pH 8.0, to a final concentration of 100 mM borate. Linker-drug solution (8.0 molar equivalents, 10 mM in *N*,*N*-dimethylacetamide (DMA)) was added to the antibody solution and the mixture was combined by gentle inversion. The reaction was allowed to proceed at room temperature for 1 hour.

Ultrafiltration Diafiltration (UF/DF) was applied to remove residual small molecules from the resulting antibody-drug conjugate, and to buffer exchange the antibody-drug conjugate. Using a Pellicon^{®} 3 membrane (0.11m²) for Tangential Flow Filtration (TFF), the antibody-drug conjugate was buffer exchanged with 10 diavolumes of 7% DMA in DPBS (Dulbecco's phosphate-buffered saline), 10 diavolumes DPBS, and finally 20 diavolumes of 15 mM histidine, 7% sucrose, pH 6.0 buffer. The final ADC solution was sterile-filtered with a 0.22 µm filter before being stored at -80 °C.

### Example 3: DAR (Drug Antibody Ratio) Determination

DAR was determined by LC-MS. LC-MS analysis was performed using an Agilent 1290 Infinity II HPLC system interfaced to an Agilent 6230B TOF 6220 ESI mass spectrometer. The ADC was reduced with 5 mM (final concentration) Bond-Breaker^{®} TCEP solution (Thermo Scientific^{®}, Rockford, IL), loaded onto an OPTI-TRAP^{™} 1 mm MICRO, PROTEIN 5 uL desalting catridge (Optimize Technologies, Auburn, CA) and eluted with a gradient of 10% B to 75% B in 0.2 minutes at ambient temperature. Mobile phase A was H₂O with 0.1% formic acid (FA), mobile phase B was acetonitrile with 0.1% FA, and the flow rate was 0. 4 mL/minute. Electrospray-ionization time-of-flight mass spectra of the co-eluting light and heavy chains were acquired using Agilent MassHunter^{®} acquisition software. The extracted intensity vs. *m*/*z* spectrum was deconvoluted using the Maximum Entropy feature of MassHunter^{®} software to determine the mass of each reduced antibody fragment. DAR was calculated from the deconvoluted spectrum by summing intensities of the naked and modified peaks for the light chain and heavy chain, normalized by multiplying intensity by the number of drugs attached. The summed, normalized intensities were divided by the sum of the intensities, and the summing results for two light chains and two heavy chains produced a final average DAR value for the full ADC.

| **ADC** | **DAR value** |
|---|---|
| ADC-1 | 4 |
| ADC-2 | 4 |

### Example 4. In vitro binding kinetics of Mirvetuximab and bivalent biparatopic antibody with Human and Cynomolgus monkey FOLR1 as measured by Surface Plasmon Resonance

The antibodies and antibody drug conjugates (ADCs) used for *in vitro* and *in vivo* testing are shown in Table 10.

**Table 10. Antibodies and ADCs used for in vitro studies**

| **ADC** | **Antibody** | **Heavy Chain SEQ ID NO:** | **Heavy Chain 2 SEQ ID NO:** | **Light Chain SEQ ID NO:** |
|---|---|---|---|---|
| ADC-1 | Mirvetuximab | 5 or 6 | | 11 |
| ADC-2 | Bivalent biparatopic | 12 | 13 | 11 |

ADC-3, a control ADC, was also utilized for *in vitro* testing. ADC-3 uses the human monoclonal antibody MSL109, which is an anti-cytomegalovirus antibody (Nokta, M. et al. Antiviral Res. 1994 May;24(1):17-26. doi: 10.1016/0166-3542(94)90048-5). The ADC utilizes the drug Top1 inhibitor payload and has a DAR of 4.

### Surface Plasmon Resonance Binding Assay (SPR)

A Biacore^{™} T200 SPR instrument (Cytiva, Marlborough, MA, USA) was used to measure the binding kinetics of human and cynomolgus monkey FOLR1 extracellular domains (ECDs) (analyte) binding to mirvetuximab antibody or the bivalent biparatopic antibody (ligands). The ECDs of both human and cynomolgus monkey FOLR1 were composed of residues 25-233 and included carboxy-terminal 6-histidine tags. The assay format was based on fragment crystallizable (Fc) capture via immobilized anti-human Fc (Thermo Fisher Scientific, Waltham, MA, USA). A standard amine coupling protocol was used to immobilize the anti-Fc capture antibody via primary amines to the carboxymethyl dextran surface of CM5 sensorchips (Cytiva); capture antibodies were coupled to a level of approximately 2000 RU. Chip preparation and binding kinetic measurements were made in the assay buffer HBS-EP+ (Cytiva; 10 mM HEPES, pH 7.4, 150 mM NaCl, 3 mM EDTA, 0.05% Tween^{®} 20). Each assay cycle consisted of the following steps: 1) capture of ligand to approximately 100 RU; 2) analyte injection over both reference and test surface; 240 µL at 80 µL/minute, after which dissociation was monitored for 15 minutes at 80 µL/minute; and 3) regeneration of capture surface with 10 mM glycine, pH 1.5. During the assay, all measurements were referenced against the capture surface alone (*i.e.,* with no captured ligand), and buffer-only injections were used for double referencing. For kinetic determinations, analyte injections were a 5-fold dilution series starting at 2.5 mM for a total of four concentrations; buffer-only injections were included for secondary referencing. Data were processed and fitted globally to a 1:1 binding model using Biacore T200 Evaluation Software to determine the binding kinetic rate constants, kₐ (M-1s-1, on-rate) and k_{d} (s-1, off-rate) and the equilibrium dissociation constant K_{D} (M, affinity).

Binding kinetics for human and cynomolgus FOLR1 are similar, as shown in Table 10 A and B. Mirvetuximab binds to human FOLR1 ECD with K_{D} = 4.5e-10 M and to cynomolgus FOLR1 ECD with K_{D} = 3.2e-10 M. The bivalent biparatopic antibody binds to human FOLR1 ECD with K_{D} = 8.5e-10 and to cynomolgus FOLR1 ECD with K_{D} = 5.1e-10.

### Table 11 A and B: Binding kinetics of human and cynomolgus FOLR1 to anti-FOLR1 mirvetuximab and bivalent biparatopic antibody as determined by SPR

**Table 11 A.**

| **Antibody** | **Human FOLR1 (25-233-His6)** | | | |
|---|---|---|---|---|
| | **ka (1/Ms)** | **kd (1/s)** | **KD (M)** | **Stoichiometry** |
| **Mirvetuximab** | 3.6e5 | 1.6e-4 | 4.5e-10 | 2.1 |
| **Bivalent biparatopic** | 2.8e5 | 2.3e-4 | 8.5e-10 | 1.9 |

**Table 11 B.**

| **Antibody** | **Cynomolgus FOLR1 (25-233-His6)** | | | |
|---|---|---|---|---|
| | **ka (1/Ms)** | **kd (1/s)** | **KD (M)** | **Stoichiometry** |
| **Mirvetuximab** | 4.4e5 | 1.4e-4 | 3.2e-10 | 2.1 |
| **Bivalent biparatopic** | 3.5e5 | 1.7e-4 | 5.1e-10 | 1.9 |

### Example 5: FOLR1 density on gynecological cancer cell lines and their response to ADCs

### Determination of Receptor Density

FOLR1 cell surface density (Antibody Binding Capacity, ABC) was determined by indirect immunofluorescent staining of surface antigens on cultured cells using QIFIKIT^{®} (Agilent/Dako, Santa Clara, CA, USA). Cells were detached with 0.25% Trypsin-EDTA at room temperature and plated in round-bottom 96-well plates at 100,000 cells/well in 100 µL of complete growth media. Plated cells were centrifuged at 200 x g at 4 °C for 5 minutes.

Supernatant was removed, and cells were resuspended in 200 µL Staining Buffer (BD Pharmingen^{™}, Milpitas, CA, USA). Cells were washed twice by pelleting at 200 x g, removing the supernatant, and resuspending in 200 µL Staining Buffer. Human FOLR1 antibodies were diluted starting from 300 nM followed by a 1:3 serial dilution in Staining Buffer. After a final wash, supernatant was removed, and 100 µL of diluted antibody were added to cells. Plates were incubated at 4 °C for one hour. During incubation, QIFIKIT^{®} beads were washed as recommended in the manufacturer's instructions; cells were washed as above. Following a final wash, supernatant was removed, and cells were resuspended in 100 µL of an anti-human IgG-PE antibody (Southern Biotech, Birmingham, AL, USA) along with Far Red Live/Dead Stain (Invitrogen/ThermoFisher, Waltham, MA, USA) diluted in Staining Buffer at 1 µL/mL. Beads were resuspended in 100 µL of anti-mouse IgG-PE antibody (Southern Biotech) diluted in Staining Buffer. The plate was incubated, washed, and samples were resuspended as described above. Samples were analyzed on a FACS Canto II flow cytometer (Becton Dickinson, Franklin Lakes, NJ, USA) with a minimum of 10,000 events counted per cell line, and data were analyzed using FloJo^{™} software (Ashland, OR, USA). Calculations based on a standard curve from QIFIKIT^{®} calibration beads were generated using GraphPad Prism software (Dotmatics, Boston, MA, USA).

To interrogate a potential correlation of surface FOLR1 expression and sensitivity to ADC-1 and ADC-2, proliferation of a panel of cancer cell lines was evaluated *in vitro.* Cell lines were sourced, as shown, and grown in 37 °C incubators with 5% CO₂. All cultures were supplemented with 10% FBS in the specified media: COV318 [European Collection of Authenticated Cell Cultures (ECACC)], RPMI; IGROV-1 [Sigma Aldrich (MO, USA)], DMEM; KB [American Type Culture Collection (ATCC, Manassas, VA, USA)], Eagle's MEM; OV-90 (ATCC), RPMI; OVSAHO(JCRB), RPMI.

Flow cytometry demonstrated that this panel of cancer cell lines expresses a range of FOLR1 levels as quantified by antibody binding capacity.

### In Vitro Cytotoxicity Assay

Cells were detached with 0.25% Trypsin-EDTA at room temperature and plated in 96-well black, round-bottom Spheroid Microplates (Corning Scientific, Corning, NY, USA) in 100 µL growth media at 1000 to 5000 cells/well. The cells were incubated overnight at 37 °C with 5% CO₂. On day 2, 3-fold dilutions of treatments, starting from 300 nM final concentration, were added to the plate (10 µL/well). Untreated control wells (for 0% control) were included on each plate. The plates were incubated for 10 days at 37°C with 5% CO₂. CellTiter-Glo^{®} 3D Cell Viability Solution (Promega, Madison, WI, USA) was added to each well at 1:1 dilution (100 µL:100 µL), and the plates were then incubated, shaking at 600 RPM, for 30 minutes under ambient conditions. Plates were read on the PerkinElmer (Waltham, MA, USA) EnVision plate reader with luminescence setting. Percent of control was calculated using the following formula: 100x (treated)/(control). Half-maximal inhibition values (EC₅₀) were calculated using GraphPad Prism Software (Dotmatics), where n = 2 in two independent experiments.

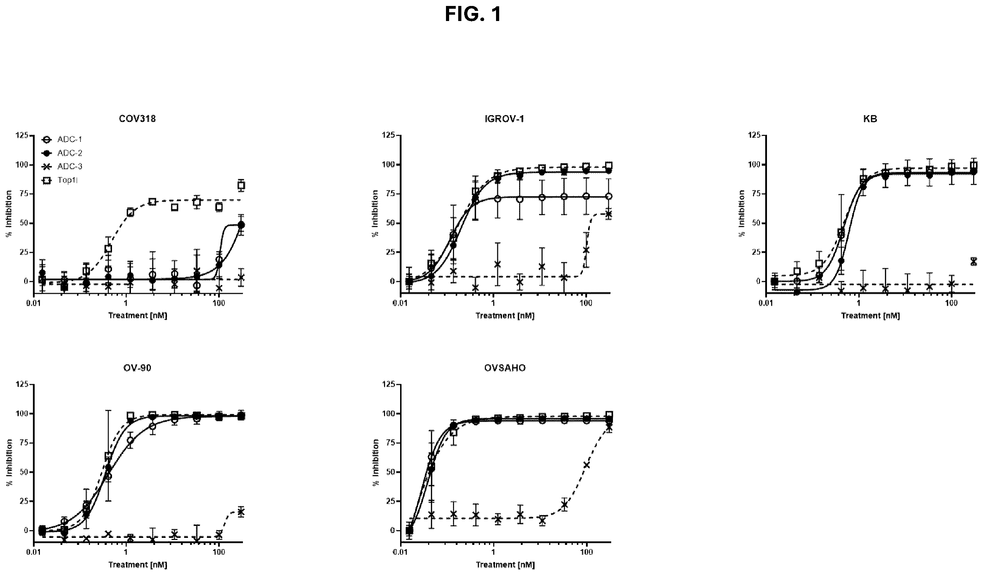

Table 11 and Figure 1 illustrate *in vitro* activities for the Top1 inhibitor and related ADCs. Each of the cancer cell lines was sensitive to the Top1 inhibitor payload alone with sub-nanomolar potency, but there is a distinction among cell lines. ADC-1 and ADC-2 were both active against cells with surface expression estimated at >25,000 FOLR1/cell (illustrated with OV-90 cells) and insensitive on COV318 cells with a lower FOLR1 level. These responses were antigen (FOLR1)-dependent, since none of the cell lines showed activity when exposed to the non-targeting ADC-3.

**Table 12. Cancer cell lines, FOLR1 receptor density, and cell proliferation potency in the presence of Top1 inhibitor payload or ADCs**

| **Cells** | **Indication** | **FOLR1/Cell^{a}** | **CellTiter-Glo EC50 (nM)** | | | |
|---|---|---|---|---|---|---|
| | | | **Payload** | **ADC-1** | **ADC-2** | **ADC-3** |
| COV318 | Ovarian | 3,500 | 0.4 | >100 | >100 | >300 |
| IGROV-1 | Ovarian | >583,000^{b} | 0.2 | 0.1 | 0.2 | >100 |
| KB | Cervical | >583,000^{b} | 0.5 | 0.5 | 0.6 | >300 |
| OV-90 | Ovarian | 25,000 | 0.3 | 0.4 | 0.4 | >100 |
| OVSAHO | Ovarian | 156,500 | 0.1 | 0.04^{c} | 0.05^{c} | >90 |

| | | | | | | |
|---|---|---|---|---|---|---|
| **^{a}** Surface-expressed FOLR1 molecules/cell as defined by ABC and detected with Mirvetuximab ^{b} 583,000 is the highest expression projected with QIFITKIT^{®} assay as conducted. ^{c} Estimated value observed in replicates | | | | | | |

### Example 6: Inhibition of CDX and PDX tumor proliferation by ADC-1 and ADC-2 in mice

### Cells and Culturing

The cell line, OV-90 was obtained from the American Type Culture Collection (ATCC, Manassas, VA, USA) and IGROV-1 was obtained from Sigma Aldrich (Sigma Alrich, Saint Louis, MO, USA). The cell lines were maintained in RPMI 1640 media (Gibco, Fisher Scientific, Waltham, MA, USA) supplemented with 10% human serum (Corning Life Sciences, Corning, NY, USA). Patient-derived xenograft (PDX) ovarian models, OV336 and OV89, were established at AbbVie Inc. (North Chicago, IL, USA). PDX cells for dosing experimental mice were expanded in NSG mice obtained from The Jackson Laboratory (Bar Harbor, ME, USA).

### Mice Husbandry

Female CB17/SCID mice at six to eight weeks of age were obtained from Charles River (Wilmington, MA, USA) and the NSG mice at six to eight weeks of age were obtained from The Jackson Laboratory and housed at a maximum of 5 per cage. The mice weighed from 18 to 20 g upon arrival. Food and water were available *ad libitum.* Mice were acclimated to the animal facilities for a period of 72 hours prior to commencement of experiments. Animals were treated during the light phase of a 12-hour light: 12-hour dark schedule (lights on at 06:00 hours). All experiments were conducted in compliance with AbbVie's Institutional Animal Care and Use Committee and the National Institutes of Health Guide for Care and Use of Laboratory Animals guidelines.

### Generation of Tumor-Bearing Mice and Growth Inhibition of Xenografted Cells

For each subcutaneous xenograft study, viable cells were inoculated at 5 million cells into the right flank of CB17/SCID mice for cell line xenograft (CDX) models and 50 thousand cells into the mammary fat pad region of NSG mice for the PDX models. The injection volume was 0.1 mL composed of cells in a 1:1 mixture of Hanks' Balanced Salt Solution (Fisher Scientific) and Matrigel (Corning Scientific). Tumors were size matched at approximate volumes of 150 to 200 mm³. Therapy typically commenced within 24 hours following randomization and size matching of tumors into required cohorts. Mice weighed approximately 20 to 26 g at the onset of therapy. Tumor volume was estimated one to two times weekly by measurements of length (L) and width (W) of the tumor, which were obtained via electronic calipers and volume was calculated according to the following equation: V = (L x W²)/2. Mice were euthanized when tumor volume reached a maximum of 2,000 mm³ or if animal health was compromised, per institutional guidelines.

Mice were dosed intraperitoneally (IP) with a single low or high dose of the ADC in an appropriate buffer at the beginning of treatment. All IP doses were tolerated, and no body weight reductions were observed. Tumor measurements and efficacy were followed for a maximum of 90 days or when institutional guidelines were met.

### Compounds and Formulations

### In vivo Cell Line-Derived Xenograft (CDX) Studies

CB17/SCID mice were evaluated twice weekly to determine inhibition of CDX tumor proliferation by ADC-1 and ADC-2. In the mice inoculated with the IGROV1 cell line, which highly expresses FOLR1 (see Example 5), both ADC-1 and ADC-2 have comparable potency. This inhibition was shown at both low and high dose levels of the ADCs. The growth inhibition was shown after administration of one dose of the ADC. In contrast, mice inoculated with the low FOLR1 expressing cell line OV90 (see Example 5) showed different results. In these mice, ADC-2 was more efficacious than ADC-1 at limiting tumor growth at both low and high dose levels.

### In vivo Patient-Derived Xenograft (PDX) Studies

NSG mice were evaluated twice weekly to determine inhibition of PDX tumor proliferation by ADC-1 and ADC-2. In these studies, mice were inoculated with cells of two high expressing FOLR1 ovarian models, OV336 and OV89. In the OV336-inoculated mice, a strong dose response was seen for both ADC-1 and ADC-2. However, ADC-1 was more active at preventing growth in tumor size. In contrast, OV89-inoculated mice showed a mild dose relationship in proliferation control. The efficacy of ADC-1 is higher or comparable to ADC-2, administered as a single dose, at both low and high dose levels in both PDX studies.

In summary, ADC-1 showed slightly greater efficacy in mouse CDX and PDX models where FOLR1 expression was high. However, in mouse models with low FOLR1 expression, ADC-2 was more efficacious than ADC-1.

### Example 7: Efficacy and Half-Life of Anti-FRα Antibodies Conjugated with Topoisomerase Inhibitors Compared to Anti-FRα Antibodies Conjugated with Maytansinoids

Anti-tumor efficacy and *in vivo* half-life of TOP1i-conjugated anti-FRα antibodies were compared to corresponding anti-FRα maytansinoid-conjugated antibodies.

Anti-tumor efficacy of TOP1i antibodies and a maytansinoid conjugated antibody was assessed in mouse model systems such as described in Example 6.

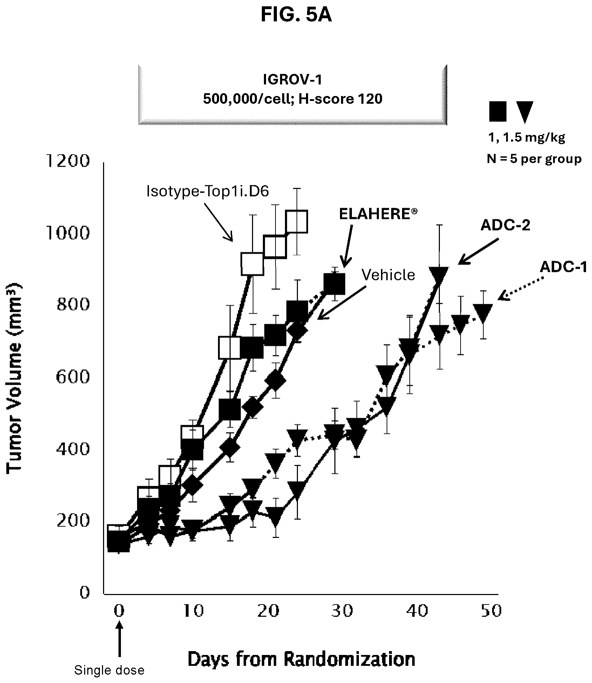

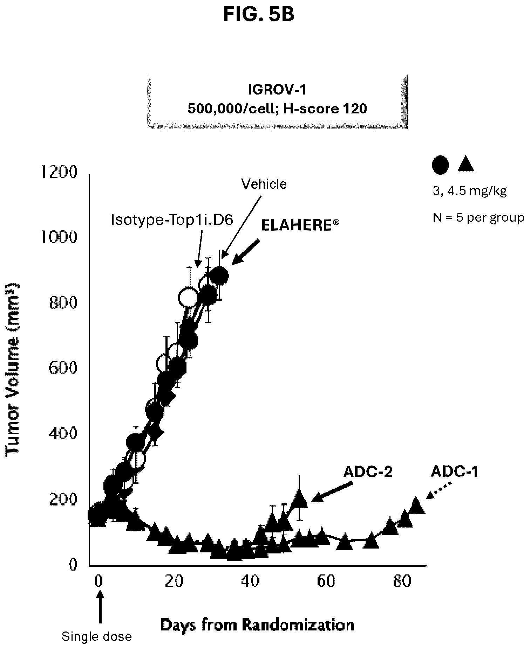

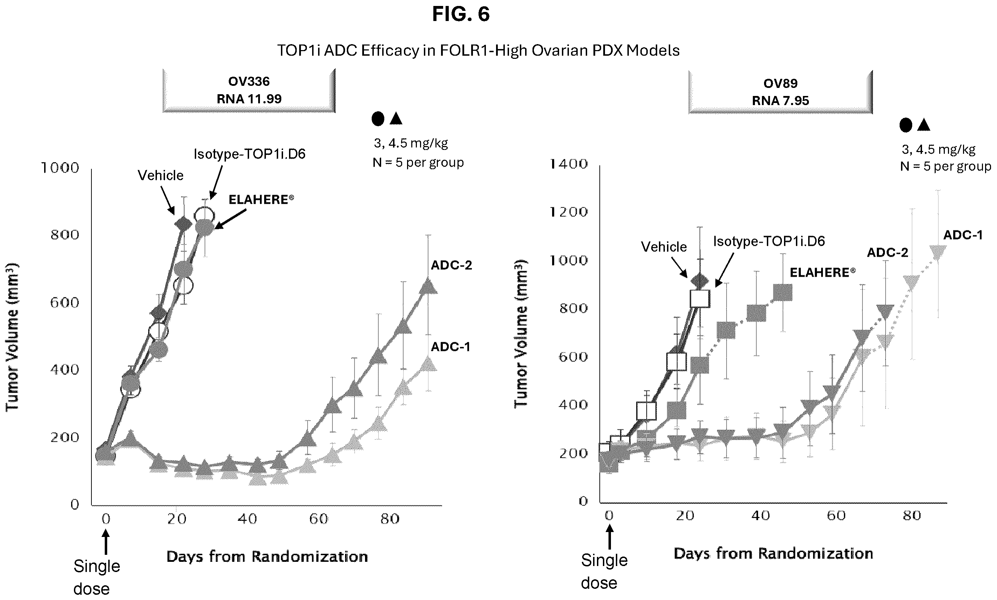

In ovarian cancer models utilizing IGROV-1, OV336 or OV89 cells (high FRα expression levels), experiments showed TOP1i-conjugated ADCs (ADC-1 and ADC-2) exhibited improved efficacy at inhibiting tumor progression as compared to maytansinoid conjugated anti-FRα antibody mirvetuximab soravtansine (ELAHERE^{®}). See, FIG. 5-6.

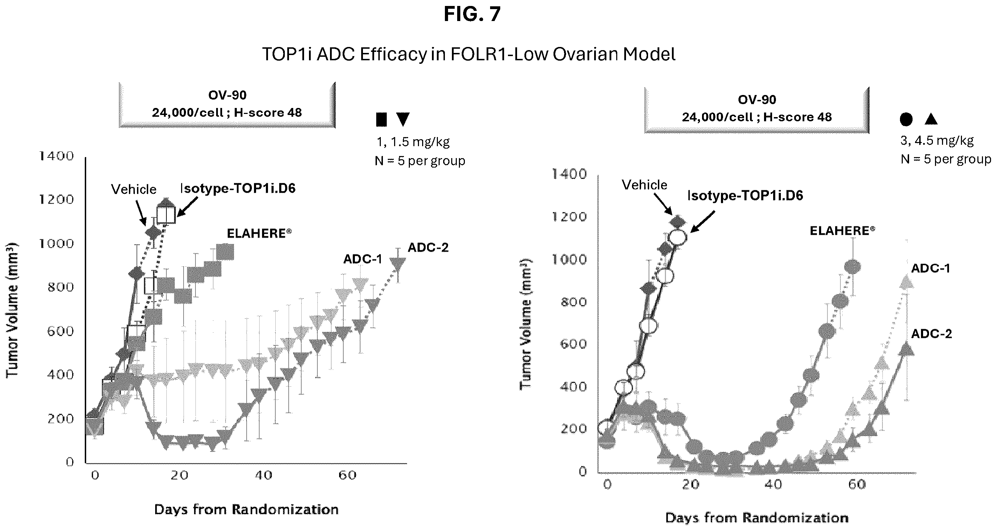

Furthermore, in ovarian cancer models utilizing OV90 cells (low FRα expression levels), experiments again showed that TOP1i-conjugated ADCs (ADC-1 and ADC-2) exhibited improved efficacy at inhibiting tumor progression as compared to maytansinoid conjugated anti-FRα antibody mirvetuximab soravtansine (ELAHERE^{®}). See, FIG. 7. Moreover, ADC-1 and ADC-2 at equivalent doses showed comparable potencies. See, FIGs. 5-7.

**Table 13: Selected Sequences**

| **SEQ ID NO:** | **Description** | **Sequence** |
|---|---|---|
| 1 | ADC-1 Ab CDR-H1/ ADC-2 Ab second CDR-H1 (Kabat) | GYFMN |
| 2 | ADC-1 Ab CDR-H2/ ADC-2 Ab second CDR-H2 (Kabat) | RIHPYDGDTFYNQKFQG |
| 3 | ADC-1 Ab CDR-H3/ ADC-2 Ab second CDR-H3 (Kabat) | YDGSRAMDY |
| 4 | ADC-1 Ab Heavy Chain Variable Region/ ADC-2 Ab Second Heavy Chain Variable Region (VH) | QVQLVQSGAEVVKPGASVKISCKASGYT FTGYFMNWVKQSPGQSLEWIGRIHPYDG DTFYNQKFQGKATLTVDKSSNTAHMELL SLTSEDFAVYYCTRYDGSRAMDYWGQG TTVTVSS |
| 5 | ADC-1 Ab Heavy Chain (HC) | QVQLVQSGAEVVKPGASVKISCKASGYT FTGYFMNWVKQSPGQSLEWIGRIHPYDG DTFYNQKFQGKATLTVDKSSNTAHMELL SLTSEDFAVYYCTRYDGSRAMDYWGQG TTVTVSSASTKGPSVFPLAPSSKSTSGGTA ALGCLVKDYFPEPVTVSWNSGALTSGVH TFPAVLQSSGLYSLSSVVTVPSSSLGTQTY ICNVNHKPSNTKVDKKVEPKSCDKTHTC PPCPAPELLGGPSVFLFPPKPKDTLMISRT PEVTCVVVDVSHEDPEVKFNWYVDGVE VHNAKTKPREEQYNSTYRVVSVLTVLHQ DWLNGKEYKCKVSNKALPAPIEKTISKA KGQPREPQVYTLPPSRDELTKNQVSLTCL VKGFYPSDIAVEWESNGQPENNYKTTPP VLDSDGSFFLYSKLTVDKSRWQQGNVFS CSVMHEALHNHYTQKSLSLSPGK |
| 6 | ADC-1 Ab Heavy chain (HC)without C-terminal lysine | QVQLVQSGAEVVKPGASVKISCKASGYT FTGYFMNWVKQSPGQSLEWIGRIHPYDG DTFYNQKFQGKATLTVDKSSNTAHMELL SLTSEDFAVYYCTRYDGSRAMDYWGQG TTVTVSSASTKGPSVFPLAPSSKSTSGGTA ALGCLVKDYFPEPVTVSWNSGALTSGVH TFPAVLQSSGLYSLSSVVTVPSSSLGTQTY |
| | | ICNVNHKPSNTKVDKKVEPKSCDKTHTC PPCPAPELLGGPSVFLFPPKPKDTLMISRT PEVTCVVVDVSHEDPEVKFNWYVDGVE VHNAKTKPREEQYNSTYRVVSVLTVLHQ DWLNGKEYKCKVSNKALPAPIEKTISKA KGQPREPQVYTLPPSRDELTKNQVSLTCL VKGFYPSDIAVEWESNGQPENNYKTTPP VLDSDGSFFLYSKLTVDKSRWQQGNVFS CSVMHEALHNHYTQKSLSLSPG |
| 7 | ADC-1 Ab CDR-L1/ ADC-2 Ab second CDR-L1 (Kabat) | KASQSVSFAGTSLMH |
| 8 | ADC-1 Ab CDR-L2/ ADC-2 Ab second CDR-L2 (Kabat) | RASNLEA |
| 9 | ADC-1 Ab CDR-L3/ ADC-2 Ab second CDR-L3 (Kabat) | QQSREYPYT |
| 10 | ADC-1 Ab Light Chain Variable Region/ ADC-2 Ab Second Light Chain Variable Region (VL) | DIVLTQSPLSLAVSLGQPAIISCKASQSVSF AGTSLMHWYHQKPGQQPRLLIYRASNLE AGVPDRFSGSGSKTDFTLTISPVEAEDAA TYYCQQSREYPYTFGGGTKLEIKR |
| 11 | ADC-1 Ab Light chain/ ADC-2 Ab third polypeptide or Light chain (LC) | DIVLTQSPLSLAVSLGQPAIISCKASQSVSF AGTSLMHWYHQKPGQQPRLLIYRASNLE AGVPDRFSGSGSKTDFTLTISPVEAEDAA TYYCQQSREYPYTFGGGTKLEIKRTVAAP SVFIFPPSDEQLKSGTASVVCLLNNFYPRE AKVQWKVDNALQSGNSQESVTEQDSKD STYSLSSTLTLSKADYEKHKVYACEVTH QGLSSPVTKSFNRGEC |
| 12 | ADC-2 Ab first polypeptide or Heavy Chain 1: scFv-Fc-knob | EIVLTQSPATLSVTPGDRVSLSCRASQNIN NNLHWYQQKPGQSPRLLIKYVSQSVSGIP DRFSGSGSGTDFTLSISSVEPEDEGMYFC QQSNSWPHYTFGCGTKLEIKGGGGSGGG GSGGGGSGGGGSEVQLVQSGGGLVQPG GSRRLSCAASGFTFSSFGMHWVRQAPGK CLEWVAYISSGSSTISYADSVKGRFTISRD NSKKTLLLQMTSLRAEDTAMYYCAREA YGSSMEYWGQGTLVTVSSGSEPKSSDKT HTCPPCPAPELLGGPSVFLFPPKPKDTLMI SRTPEVTCVVVDVSHEDPEVKFNWYVDG VEVHNAKTKPREEQYNSTYRVVSVLTVL HQDWLNGKEYKCKVSNKALPAPIEKTIS KAKGQPREPQVYTLPPSREEMTKNQVSL WCLVKGFYPSDIAVEWESNGQPENNYKT TPPVLDSDGSFFLYSKLTVDKSRWQQGN VFSCSVMHEALHNHYTQKSLSLSPG |
| 13 | ADC-2 Ab second polypeptide or Heavy Chain 2: Fc-hole | QVQLVQSGAEVVKPGASVKISCKASGYT FTGYFMNWVKQSPGQSLEWIGRIHPYDG DTFYNQKFQGKATLTVDKSSNTAHMELL SLTSEDFAVYYCTRYDGSRAMDYWGQG TTVTVSSASTKGPSVFPLAPSSKSTSGGTA ALGCLVKDYFPEPVTVSWNSGALTSGVH TFPAVLQSSGLYSLSSVVTVPSSSLGTQTY ICNVNHKPSNTKVDKKVEPKSCDKTHTC PPCPAPELLGGPSVFLFPPKPKDTLMISRT PEVTCVVVDVSHEDPEVKFNWYVDGVE VHNAKTKPREEQYNSTYRVVSVLTVLHQ DWLNGKEYKCKVSNKALPAPIEKTISKA KGQPREPQVYTLPPSREEMTKNQVSLSC AVKGFYPSDIAVEWESNGQPENNYKTTP PVLDSDGSFFLVSKLTVDKSRWQQGNVF SCSVMHEALHNHYTQKSLSLSPG |
| 14 | ADC-2 Ab first Heavy Chain Variable Region (VH): FR57 E6Q; G44C | EVQLVQSGGGLVQPGGSRRLSCAASGFT FSSFGMHWVRQAPGKCLEWVAYISSGSS TISYADSVKGRFTISRDNSKKTLLLQMTS LRAEDTAMYYCAREAYGSSMEYWGQGT LVTVSS |
| 15 | ADC-2 Ab first Light Chain Variable Region (VL): FR57, F83E, Q101C | EIVLTQSPATLSVTPGDRVSLSCRASQNIN NNLHWYQQKPGQSPRLLIKYVSQSVSGIP DRFSGSGSGTDFTLSISSVEPEDEGMYFC QQSNSWPHYTFGCGTKLEIK |
| 16 | ADC-2 Ab first CDR-L1 (Kabat) | RASQNINNNLH |
| 17 | ADC-2 Ab first CDR-L2 (Kabat) | YVSQSVS |
| 18 | ADC-2 Ab first CDR-L3 (Kabat) | QQSNSWPHYT |
| 19 | ADC-2 Ab first CDR-H1 (Kabat) | SFGMH |
| 20 | ADC-2 Ab first CDR-H2 (Kabat) | YISSGSSTISYADSVKG |
| 21 | ADC-2 Ab first CDR-H3 (Kabat) | EAYGSSMEY |
| 22 | ADC-1 Ab Light Chain Variable Region/ ADC-2 Ab Second Light Chain Variable Region (VL) | DIVLTQSPLSLAVSLGQPAIISCKASQSVSF AGTSLMHWYHQKPGQQPRLLIYRASNLE AGVPDRFSGSGSKTDFTLTISPVEAEDAA TYYCQQSREYPYTFGGGTKLEIK |
| 23 | ADC-1 Ab CDR-H1 (AbM) | GYTFTGYFMN |
| 24 | ADC-1 Ab CDR-H2 (AbM) | RIHPYDGDTF |
| 25 | ADC-2 Ab first CDR-H1 (AbM) | GFTFSSFGMH |
| 26 | ADC-2 Ab first CDR-H2 (AbM) | YISSGSSTIS |
| 351 | ADC-2 Ab scFv-hinge fragment | EIVLTQSPATLSVTPGDRVSLSCRASQNIN NNLHWYQQKPGQSPRLLIKYVSQSVSGIP DRFSGSGSGTDFTLSISSVEPEDEGMYFC QQSNSWPHYTFGCGTKLEIKGGGGSGGG GSGGGGSGGGGSEVQLVQSGGGLVQPG GSRRLSCAASGFTFSSFGMHWVRQAPGK CLEWVAYISSGSSTISYADSVKGRFTISRD NSKKTLLLQMTSLRAEDTAMYYCAREA YGSSMEYWGQGTLVTVSSGSEPKSS |
| 352 | Linker | GGGGSGGGGSGGGGSGGGGS |

## Claims

1. An anti-FRα antibody drug conjugate of formula (IV): 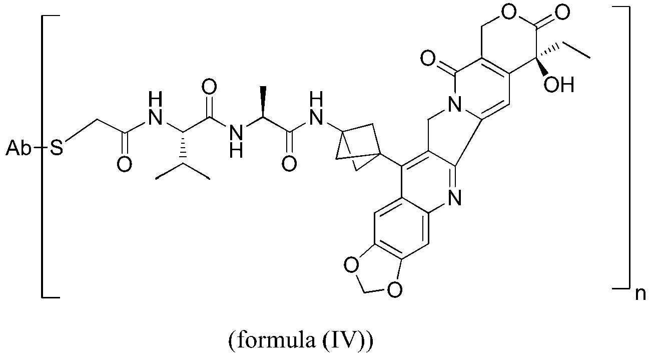 wherein n is an integer from 3 to 5, and wherein Ab is an anti-FRα antibody comprising a heavy chain variable region comprising a CDR-H1, a CDR-H2, and a CDR-H3; and a light chain variable region comprising a CDR-L1, a CDR-L2, and a CDR-L3, wherein
CDR-H1 has the amino acid sequence shown as SEQ ID NO: 1 or 23,
CDR-H2 has the amino acid sequence shown as SEQ ID NO: 2 or 24,
CDR-H3 has the amino acid sequence shown as SEQ ID NO: 3,
CDR-L1 has the amino acid sequence shown as SEQ ID NO: 7,
CDR-L2 has the amino acid sequence shown as SEQ ID NO: 8, and
CDR-L3 has the amino acid sequence shown as SEQ ID NO: 9.

2. An anti-FRα antibody drug conjugate of formula (IV): 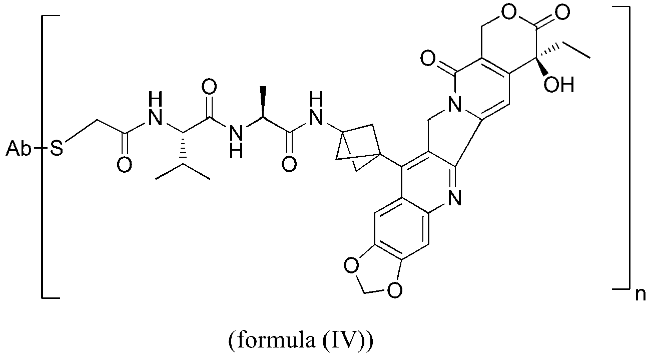 wherein n is an integer from 3 to 5, and wherein Ab is a bivalent biparatopic anti-FRα antibody comprising first and second heavy chain variable regions comprising a CDR-H1, a CDR-H2, and a CDR-H3; and first and second light chain variable regions comprising a CDR-L1, a CDR-L2, and a CDR-L3, wherein a first antigen binding moiety comprises:
a first CDR-H1 having the amino acid sequence shown as SEQ ID NO: 19 or 25,
a first CDR-H2 having the amino acid sequence shown as SEQ ID NO: 20 or 26,
a first CDR-H3 having the amino acid sequence shown as SEQ ID NO: 21,
a first CDR-L1 having the amino acid sequence shown as SEQ ID NO: 16,
a first CDR-L2 having the amino acid sequence shown as SEQ ID NO: 17, and
a first CDR-L3 having the amino acid sequence shown as SEQ ID NO: 18,
and wherein a second antigen binding moiety comprises:
a second CDR-H1 having the amino acid sequence shown as SEQ ID NO: 1 or 23,
a second CDR-H2 having the amino acid sequence shown as SEQ ID NO: 2 or 24,
a second CDR-H3 having the amino acid sequence shown as SEQ ID NO: 3,
a second CDR-L1 having the amino acid sequence shown as SEQ ID NO: 7,
a second CDR-L2 having the amino acid sequence shown as SEQ ID NO: 8, and
a second CDR-L3 having the amino acid sequence shown as SEQ ID NO: 9.

3. The anti-FRα antibody drug conjugate according to claim 1 or 2, wherein the antibody is an IgG1 antibody.

4. The anti-FRα antibody drug conjugate according to claim 1, wherein the antibody comprises (a) a heavy chain variable region having the amino acid sequence shown as SEQ ID NO: 4 and (b) a light chain variable region having the amino acid sequence shown as SEQ ID NO: 10 or 22.

5. The anti-FRα antibody drug conjugate according to claim 2, wherein (a) the first antigen binding moiety comprises a first heavy chain variable region having the amino acid sequence shown as SEQ ID NO: 14, and a first light chain variable region having the amino acid sequence shown as SEQ ID NO: 15; and (b) the second antigen binding moiety comprises a second heavy chain variable region having the amino acid sequence shown as SEQ ID NO: 4, and a second light chain variable region having the amino acid sequence shown as SEQ ID NO: 10 or 22.

6. The anti-FRα antibody drug conjugate according to claim 1, wherein the antibody comprises (a) a heavy chain having the amino acid shown as SEQ ID NO: 5 or 6 and (b) a light chain having the amino acid sequence shown as SEQ ID NO: 11.

7. The anti-FRα antibody drug conjugate according to claim 2, wherein the antibody comprises (a) a light chain having the amino acid shown as SEQ ID NO: 11, (b) a first heavy chain having the amino acid sequence shown as SEQ ID NO: 12, and (c) a second heavy chain having the amino acid sequence shown as SEQ ID NO: 13.

8. An anti-FRα antibody drug conjugate of formula (IV): 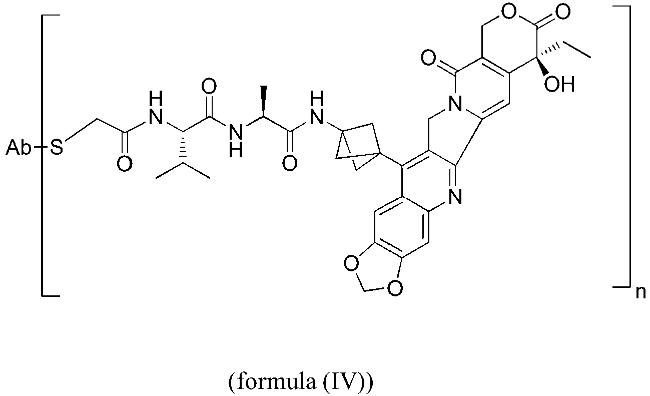 wherein n is an integer from 3 to 5, and wherein Ab is an anti-FRα antibody comprising: two heavy chains each having an amino acid sequence shown as SEQ ID NO: 5 or 6 and two light chains each having an amino acid sequence shown as SEQ ID NO: 11.

9. The anti-FRα antibody drug conjugate according to any one of claims 1 to 8, wherein n is 4 or about 4.

10. A composition comprising a plurality of the antibody drug conjugate molecules according to claim 9, wherein the predominant species of the antibody drug conjugate molecules in the composition has an n of 4.

11. A pharmaceutical composition comprising a plurality of the antibody drug conjugate molecules according to claim 9 and a pharmaceutically acceptable excipient, wherein the composition has a DAR of 4 or about 4.

12. A method of treating ovarian, peritoneal, primary peritoneal, fallopian tube, endometrial, or cervical cancer, the method comprising administering a therapeutically effective amount of the antibody drug conjugate according to claim 9 to a patient in need thereof.

13. A method of treating ovarian, peritoneal, primary peritoneal, fallopian tube, endometrial, or cervical cancer, the method comprising administering a therapeutically effective amount of the pharmaceutical composition of claim 11 to a patient in need thereof.
